# EUROPEAN PATENT APPLICATION

(11) **EP 3 889 156 A1**
(43) Date of publication of application: **06.10.2021**
(21) Application number: 19891494.7
(22) Date of filing: 02.12.2019
(51) Int. Cl.: C07D 487/04

(54) **NITROGEN-CONTAINING HETEROCYCLIC COMPOUND AND COMPOSITION THEREOF, PREPARATION METHOD THEREFOR, AND APPLICATION THEREOF**

(30) Priority: 30.11.2018 CN 201811455353
(71) Applicant: Shanghai Pharmaceuticals Holding Co., Ltd., Shanghai 201203 (CN)
(72) Inventor: LI, Di, Shanghai 201203 (CN); DUAN, Lingjun, Shanghai 201203 (CN); ZHENG, Tixu, Shanghai 201203 (CN); WANG, Yang, Shanghai 201203 (CN); JIN, Kaijun, Shanghai 201203 (CN); SU, Wei, Shanghai 201203 (CN); HONG, Yuan, Shanghai 201203 (CN); XU, Jia, Shanghai 201203 (CN); XIA, Guangxin, Shanghai 201203 (CN); KE, Ying, Shanghai 201203 (CN)
(74) Representative: Hoffmann Eitle
(86) International application number: PCT/CN2019/122459
(87) International publication number: WO 2020/108659

(57) **Abstract**

Disclosed by the present invention are a nitrogen-containing heterocyclic compound, and a composition thereof, a preparation method therefor and an application thereof. The structure of the nitrogen-containing heterocyclic compound according to the present invention is shown in Formula I. The nitrogen-containing heterocyclic compound according to the present invention or a pharmaceutically acceptable salt thereof has better inhibitory activity on p38 protein kinase and/or higher bioavailability. In addition, the preparation method is simple.

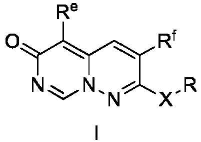

## Description

This present application claims the priority of the Chinese Patent Application CN2018114553535, filed on November 30, 2018, the contents of which are incorporated herein by reference in their entireties.

### Field of Invention

The present disclosure relates to a nitrogen-containing heterocyclic compound, and a composition thereof, a preparation method therefor and an application thereof.

### Background

MAPK (mitogen-activated protein kinases) is a kind of serine/threonine protein kinases, which is activated by the MKK-MKK-MAPK cascade, and conducts signals from the cell surface to the interior of the nucleus, expanding the cascade and causing a variety of cell biological responses (e.g. proliferation, differentiation, inflammation, stress and apoptosis, etc.).

p38-MAPK (abbreviated as p38) is widely expressed and distributed in human, its main function is to mediate cellular responses to extracellular stimuli, stress, shock, inflammatory factors, adhesion factors and other stimuli in various organ systems, including the nervous system.

The activation of p38 expression can induce the production of inflammatory factors (e.g. IL-1, TNF, TNF, IL-6), NO, prostaglandin/ prostacyclin, COX-2, matrix metalloproteinases, etc., which plays an important role in various cellular responses after acute and chronic inflammatory and stress (Arthritis Rheum. 2009 Feb;60(2) :317-20.). In addition, in neurons and glial cells, the activation of p38 leads to tau protein phosphorylation and synaptic damage. The hyperphosphorylation of tau is an important cause of neurofibrillary tangle formation and neuronal death in some neurodegenerative diseases, while the induction of COX-2 and others is closely related to the pain response.

Inhibition of p38 kinase will reduce or block the production of pro-inflammatory factors such as IL-1 and TNF, thereby decreasing the inflammatory response and reduces cellular damage. Inhibition of p38 kinase can also mediate the response typing of immune-related cells, such as the transformation of microglia from M1-type to M2-type; while specific inhibition of p38 can prevent the inflammatory responses and tau hyperphosphorylation and aggregation, improve synaptic function and cognition in AD model mice. Therefore, p38 has become an important target in drug development of many diseases, through its multifaceted effects centered on inflammatory pathways, which mainly relate to the overexpression of IL, TNF, COX-2 and tau hyperphosphorylation.

Over the past two decades, efforts have been made to try to develop drugs that use p38 as a target for inflammatory inhibition. For example, CN108349964A discloses a class of nitrogen-containing heterocyclic compounds containing S heteroatom, which can be used as inhibitors of p38 kinase. However, the p38 inhibitors currently under development have shortcomings, such as insufficient inhibitory specificity, low bioavailability, high toxic side effects, or unsuitable indication selection. Therefore it is urgent to develop and improve the current p38 inhibitors so as to improve efficacy and druggability.

There is an urgent need for a compound with better inhibitory specificity and higher bioavailability for p38.

### Invention content

The present disclosure provides a nitrogen-containing heterocyclic compound which is different from the prior art, and a composition thereof, a preparation method therefor and an application thereof. The nitrogen-containing heterocyclic compound of the present disclosure has better inhibitory activity and/or higher bioavailability for p38 protein kinase.

The present disclosure solves the above technical problems through the following technical solutions.

The present disclosure provides a nitrogen-containing heterocyclic compound represented by formula I, a pharmaceutically acceptable salt thereof, a hydrate thereof, a solvate thereof, a polymorph thereof, a metabolite thereof, a stereoisomer thereof, a tautomer thereof, or a prodrug thereof; 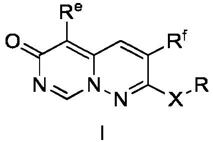
wherein, X is O, N or C₁₋₁₀ alkyleneoxy;
R is C₆₋₃₀ aryl, C₆₋₃₀ aryl substituted by R¹, C₁₋₃₀ heteroaryl, C₁₋₃₀ heteroaryl substituted by R², C₁₋₁₀ alkyl, C₁₋₁₀ alkyl substituted by R³, C₃₋₁₀ cycloalkyl, C₃₋₁₀ cycloalkyl substituted by R⁴, C₁₋₁₀ heterocycloalkyl, C₁₋₁₀ heterocycloalkyl substituted by R⁵, C₂₋₁₀ alkenyl, C₂₋₁₀ alkenyl substituted by R⁶, C₂₋₁₀ alkynyl, C₂₋₁₀ alkynyl substituted by R⁷, C₃₋₁₀ cycloalkenyl or C₃₋₁₀ cycloalkenyl substituted by R⁸;
R¹, R², R³, R⁴, R⁵, R⁶, R⁷ and R⁸ are independently one or more of halogen, C₁₋₁₀ alkyl, C₁₋₁₀ alkoxy, 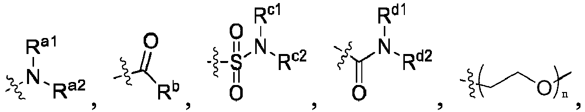 C₆₋₃₀ aryl, C₁₋₆ heterocycloalkyl, hydroxyl and cyano;
n is 1, 2or 3;
R^{a1}, R^{a2}, R^{b}, R^{c1}, R^{c2}, R^{d1} and R^{d2} are independently H, C₁₋₆ alkyl, C₁₋₆ alkoxy, C₂₋₆ alkenyl, C₂₋₆ alkynyl, C₆₋₃₀ aryl or C₆₋₃₀ aryl substituted by R^{a-1};
R^{a-1} is one or more of C₁₋₃ alkyl, C₁₋₃ alkoxy, halogen, cyano, nitro, amino and hydroxyl;
R^{e} is C₆₋₃₀ aryl, C₆₋₃₀ aryl substituted by R^{e-1} or C₃₋₁₀ cycloalkenyl;
R^{e-1} is independently one or more of halogen, C₁₋₁₀ alkyl, C₁₋₁₀ alkoxy, 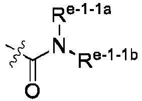 and 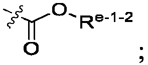
R^{e-1-1a}, R^{e-1-1b} and R^{e-1-2} are independently H or C₁₋₆ alkyl;
R^{f} is H, C₁₋₃₀ heteroaryl, C₁₋₃₀ heteroaryl substituted by R^{f-1}, C₁₋₁₀ heterocycloalkyl or C₁₋₁₀ heterocycloalkyl substituted by R^{f-2};
R^{f-2} is C₁₋₆ alkyl;
in the C₁₋₃₀ heteroaryl, the C₁₋₃₀ heteroaryl in the C₁₋₃₀ heteroaryl substituted by R², the C₁₋₁₀ heterocycloalkyl, the C₁₋₁₀ heterocycloalkyl in the C₁₋₁₀ heterocycloalkyl substituted by R⁴, the C₁₋₃₀ heteroaryl in the C₁₋₃₀ heteroaryl substituted by R^{f-1}, the C₁₋₁₀ heterocycloalkyl in the C₁₋₁₀ heterocycloalkyl substituted by R^{f-2}, and the C₁₋₆ heterocycloalkyl in "R¹, R², R³, R⁴, R⁵, R⁶, and R⁷", the number of heteroatom is independently 1-4, and the heteroatom is independently one or more of O, S and N.

The number of the R¹, the R², the R³, the R⁴, the R⁵, the R⁶, the R⁷, the R⁸ and the R^{a-1}, the R^{e-1}, the R^{f-1} and the R^{f-2} are independently be one or more than one; when the number of the R¹, the R², the R³, the R⁴, the R⁵, the R⁶, the R⁷, the R⁸ and the R^{a-1}, the R^{e-1} and the R^{e-2} are independently more than one (e.g., 1, 2, 3, 4), then the R¹, the R², the R³, the R⁴, the R⁵, the R⁶, the R⁷, the R⁸ and the R^{a-1}, the R^{e-1}, the R^{f-1} and the R^{f-2} are independently the same or different.

When X is C₁₋₁₀ alkyleneoxy, then the C₁₋₁₀ alkyleneoxy can be C₁₋₆ alkyleneoxy, and can also be a C₁₋₃ alkyleneoxy. The C₁₋₃ alkyleneoxy can be methyleneoxy, 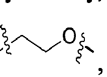 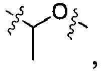 n-propyleneoxy, 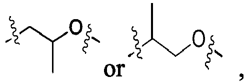 and can also be

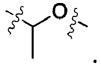

When R, R¹, R², R³, R⁴, R⁵, R⁶, R⁷, R⁸, R^{a1}, R^{a2}, R^{b}, R^{c1}, R^{c2}, R^{d1}, R^{d2} or R^{e} is C₆₋₃₀ aryl, then the C₆₋₃₀ aryl can be C₆₋₁₈ aryl, preferably C₆₋₁₄ aryl.

When R is C₆₋₃₀ aryl substituted by R¹, then the C₆₋₃₀ aryl can be C₆₋₁₈ aryl, and can also be C₆₋₁₄ aryl. The C₆₋₁₄ aryl can be phenyl, naphthyl or phenanthryl, and can also be phenyl.

When R is C₆₋₃₀ aryl substituted by R¹, then the number of R¹ can be 1, 2 or 3, preferably 2 or 3, more preferably 2. When the number of R¹ is more than one, then R¹ can be the same or different, preferably the same.

When R is C₆₋₃₀ aryl substituted by R¹, then the substitution site of R¹ can be located at one or more of the ortho position, meta position or para position C relative to the C connected to X, and can also be at least one located at the ortho position C relative to the C connected to X, further can be located at the ortho position C relative to the C connected to X "the ortho position C relative to the C connected to X and the ortho position C relative to the C connected to X", "the ortho position C relative to the C connected to X and the para position C relative to the C connected to X", or "the ortho position C relative to the C connected to X, the ortho position C relative to the C connected to X and the para position C relative to the C connected to X", and further can be located at "the ortho position C relative to the C connected to X and the para position C relative to the C connected to X".

When R is C₆₋₃₀ aryl substituted by R¹, then the C₆₋₃₀ aryl substituted by R¹ can be 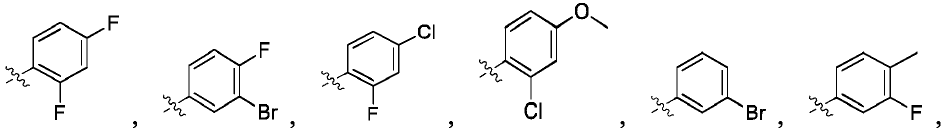 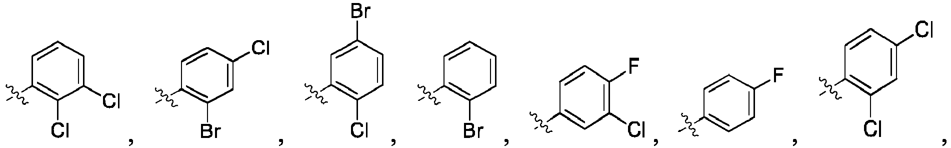 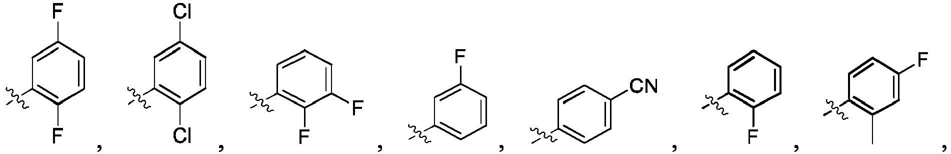 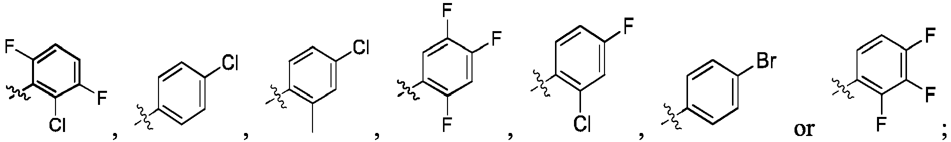 preferably 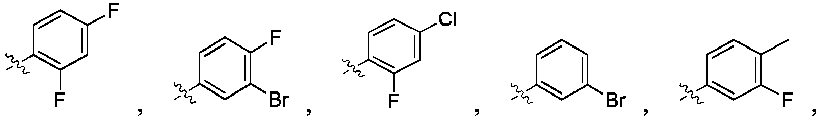 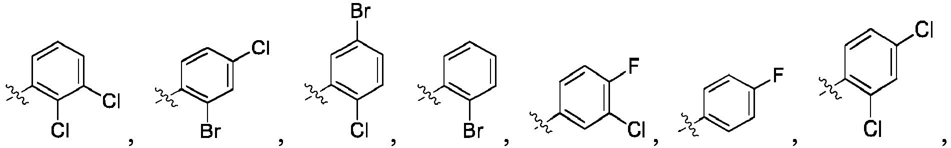 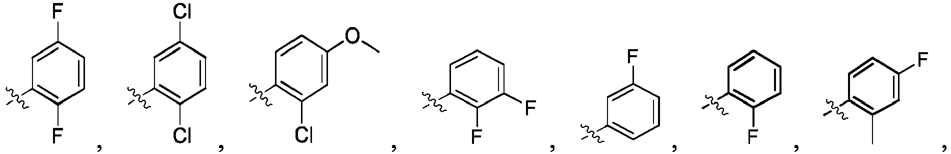 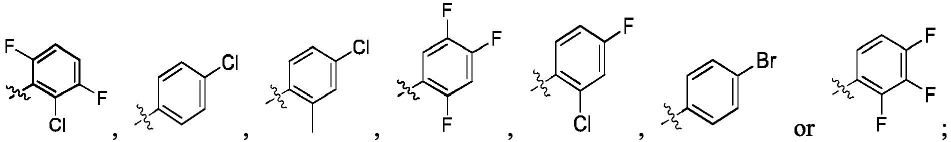 more preferably 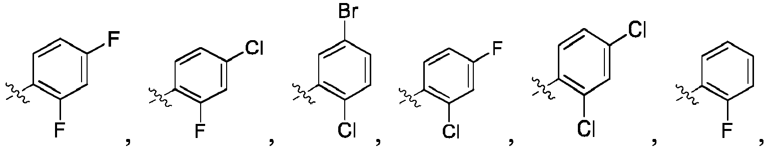 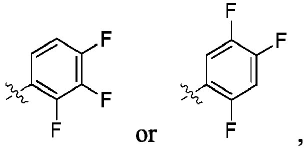 most preferably 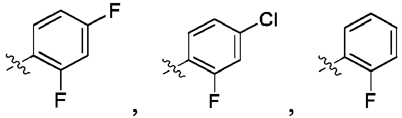 or

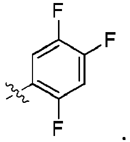

When R is C₁₋₃₀ heteroaryl or C₁₋₃₀ heteroaryl substituted by R², then the heteroatom in the C₁₋₃₀ heteroaryl can be N, and the number thereof is 1.

When R is C₁₋₃₀ heteroaryl or C₁₋₃₀ heteroaryl substituted by R², then the C₁₋₃₀ heteroaryl can be C₁₋₁₈ heteroaryl, and can also be C₁₋₁₄ heteroaryl, further can be pyridyl.

When R is C₁₋₃₀ heteroaryl substituted by R², then the number of the R² can be 1.

When R is C₁₋₃₀ heteroaryl substituted by R², then the substitution site of R² can be located at the meta position C relative to the C connected to X.

When R is C₁₋₃₀ heteroaryl substituted by R², then the C₁₋₃₀ heteroaryl substituted by R² can be

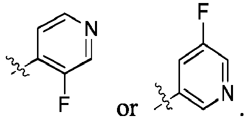

When R, R¹, R², R³, R⁴, R⁵, R⁶, R⁷, R⁸ or R^{e-1} is C₁₋₁₀ alkyl, then the C₁₋₁₀ alkyl can be C₁₋₆ alkyl, and can also be C₁₋₃ alkyl (e.g., methyl, ethyl, n-propyl, isopropyl), further can be methyl or ethyl.

When R is C₁₋₁₀ alkyl substituted by R³, then the number of the R³ can be 2 or 3. When the number of R³ is more than one, then the R³ can be the same or different, preferably the same.

When R is C₁₋₁₀ alkyl substituted by R³, then the C₁₋₁₀ alkyl substituted by R³ can be

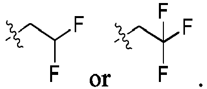

When R is C₃₋₁₀ cycloalkyl or C₃₋₁₀ cycloalkyl substituted by R⁴, then the C₃₋₁₀ cycloalkyl can be C₃₋₁₀ saturated monocycloalkyl or C₃₋₁₀ bridged ring saturated alkyl, and can also be C₃₋₆ monocyclo saturated alkyl or C₅₋₈ saturated bridged ring alkyl.

When R is C₃₋₁₀ cycloalkyl substituted by R⁴, then the number of the R⁴ can be 1 or 2. When the number of R⁴ is more than one, then the R⁴ can be the same or different, preferably the same.

When R is C₃₋₁₀ cycloalkyl substituted by R⁴, then the C₃₋₁₀ cycloalkyl substituted by R⁴ can be

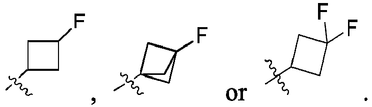

When R is C₁₋₁₀ heterocycloalkyl or C₁₋₁₀ heterocycloalkyl substituted by R⁵, then the heteroatom in the C₁₋₁₀ heterocycloalkyl can be O, and the number thereof is 1.

When R is C₁₋₁₀ heterocycloalkyl or C₁₋₁₀ heterocycloalkyl substituted by R⁵, then the C₁₋₁₀ heterocycloalkyl can be C₂₋₆ heterocycloalkyl, further can be heterocyclopentane or heterocyclohexane, and further can be

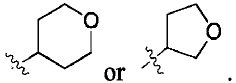

When R is C₂₋₁₀ alkenyl or C₂₋₁₀ alkenyl substituted by R⁶, then the C₂₋₁₀ alkenyl can be C₂₋₆ alkenyl, and can also be C₂₋₃ alkenyl. The C₂₋₃ alkenyl can be

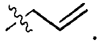

When R is C₃₋₁₀ cycloalkenyl or C₃₋₁₀ cycloalkenyl substituted by R⁸, then the C₃₋₁₀ cycloalkenyl can be C₃₋₆ cycloalkenyl, further can be cyclopentenyl or cyclohexenyl, for example

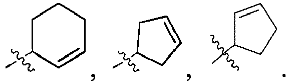

When R is C₃₋₁₀ cycloalkenyl substituted by R⁸, then the number of the R⁸ can be 1.

When R is C₃₋₁₀ cycloalkenyl substituted by R⁸, then the substitution site of the R⁸ can be located at the ortho position C relative to the C connected to X.

When R is the C₃₋₁₀ cycloalkenyl substituted by R⁸, then the C₃₋₁₀ cycloalkenyl substituted by R⁸ can be

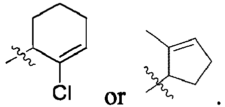

When R¹, R², R³, R⁴, R⁵, R⁶, R⁷, R⁸, R^{a-1} or R^{e-1} is halogen, then the halogen can be F, Cl, Br or I, and can also be F or Cl.

When R¹, R², R³, R⁴, R⁵, R⁶, R⁷, R⁸, or R^{e-1} is C₁₋₁₀ alkoxy, then the C₁₋₁₀ alkoxy can be C₁₋₃ alkoxy, further can be methoxy, ethoxy, n-propoxy or isopropoxy benzene.

When R^{e} is C₆₋₃₀ aryl substituted by R^{e-1}, then the C₆₋₃₀ aryl can be C₆₋₁₈ aryl, preferably C₆₋₁₄ aryl. The C₆₋₁₄ aryl is preferably phenyl, naphthyl or phenanthryl, and more preferably phenyl.

When R^{e} is C₆₋₃₀ aryl substituted by R^{e-1}, then the number of the R^{e-1} can be 1, 2 or 3.

When R^{e} is C₆₋₃₀ aryl substituted by R^{e-1}, then the substitution site of the R^{e-1} can be located at one or more of the ortho position C, meta position C or para position C relative to C connected to the connection site, and can at least one of them located at the ortho position C relative to C connected to the connection site, and further can "located at the ortho position C relative to C connected to the connection site and the ortho position C relative to C connected to the connection site ", " located at the ortho position C relative to C connected to the connection site and the meta position C relative to C connected to the connection site", or "located at the ortho position C relative to C connected to the connection site, the meta position C relative to C connected to the connection site and the para position C relative to C connected to the connection site", and further can " located at the ortho position C relative to C connected to the connection site and the ortho position C relative to C connected to the connection site ".

When R^{e} is C₆₋₃₀ aryl substituted by R^{e-1}, then the C₆₋₃₀ aryl substituted by R^{e-1} can be 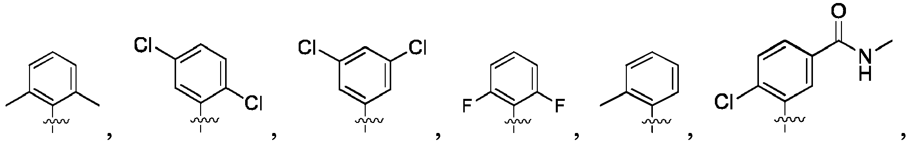 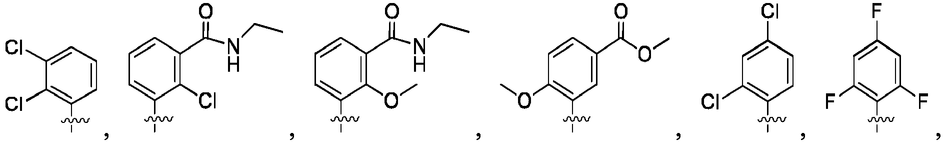 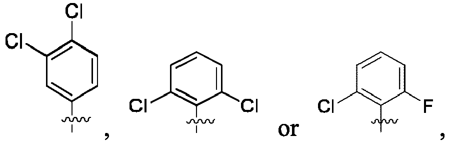 and can also be 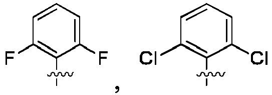 or

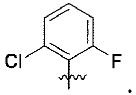

When R^{e} is C₃₋₁₀ cycloalkenyl, then the C₃₋₁₀ cycloalkenyl can be C₃₋₆ cycloalkenyl, further can be cyclohexenyl, for example

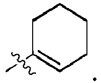

When R^{e-1-1a}, R^{e-1-1b} or R^{e-1-2} is C₁₋₆ alkyl, then the C₁₋₆ alkyl can be C₁₋₃ alkyl (methyl, ethyl, n-propyl or isopropyl), further can be methyl or ethyl.

When R^{f} is C₁₋₃₀ heteroaryl, then the heteroatom in the C₁₋₃₀ heteroaryl can be N and the number thereof can be 1.

When R^{f} is C₁₋₃₀ heteroaryl, then the C₁₋₃₀ heteroaryl can be C₁₋₁₄ heteroaryl, further can be pyridyl, for example

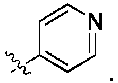

When R^{f} is C₁₋₁₀ heterocycloalkyl or C₁₋₁₀ heterocycloalkyl substituted by R^{f-2}, then the heteroatom in the C₁₋₁₀ heterocycloalkyl can be N and/or O, and the number thereof is 2. When the number of heteroatom is 2, then the heteroatom can be the same or different.

When R^{f} is C₁₋₁₀ heterocycloalkyl, then the C₁₋₁₀ heterocycloalkyl can be C₂₋₆ heterocycloalkyl, further can be heterocyclohexane, and further can be

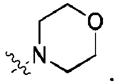

When R^{f} is C₁₋₁₀ heterocycloalkyl substituted by R^{f-2}, then the substitution position of R^{f-2} can be on the heteroatom, and the number thereof is 1.

When R^{f-2} is C₁₋₆ alkyl, then the C₁₋₆ alkyl can be C₁₋₃ alkyl (methyl, ethyl, n-propyl or isopropyl), and further can be isopropyl.

When R^{f} is C₁₋₁₀ heterocycloalkyl substituted by R^{f-2}, then the C₁₋₁₀ heterocycloalkyl substituted by R^{f-2} can be C₂₋₆ heterocycloalkyl, and further can be heterocyclohexane.

When R^{f} is C₁₋₁₀ heterocycloalkyl substituted by R^{f-2}, then the C₁₋₁₀ heterocycloalkyl substituted by R^{f-2} can be

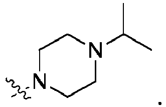

In the present disclosure, the cycloalkyl can be saturated monocyclic cycloalkyl or saturated bridged cycloalkyl.

In the present disclosure, the heterocycloalkyl can be saturated monocyclic heterocycloalkyl.

In a preferred embodiment, in the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof;
X is O, N or C₁₋₁₀ alkyleneoxy;
R is C₆₋₃₀ aryl, C₆₋₃₀ aryl substituted by R¹, C₁₋₃₀ heteroaryl, C₁₋₃₀ heteroaryl substituted by R², C₁₋₁₀ alkyl, C₁₋₁₀ alkyl substituted by R³, C₃₋₁₀ cycloalkyl, C₃₋₁₀ cycloalkyl substituted by R⁴, C₁₋₁₀ heterocycloalkyl, C₂₋₁₀ alkenyl, C₃₋₁₀ cycloalkenyl or C₃₋₁₀ cycloalkenyl substituted by R⁸;
R¹ is one or more of halogen, C₁₋₁₀ alkyl, C₁₋₁₀ alkoxy and cyano; and,
R², R³, R⁴, R⁵, R⁶, R⁷ and R⁸ are independently halogen.

In a preferred embodiment, X is O or N, preferably O.

In a preferred embodiment, R is a C₆₋₃₀ aryl, C₆₋₃₀ aryl substituted by R¹, C₃₋₁₀ cycloalkenyl or C₃₋₁₀ cycloalkenyl substituted by R⁸, preferably C₆₋₃₀ aryl substituted by R¹.

In a preferred embodiment, R¹ is one or more of halogen, C₁₋₁₀ alkyl and C₁₋₁₀ alkoxy, preferably halogen.

In a preferred embodiment, R^{e} is C₆₋₃₀ aryl substituted by R^{e-1} or C₃₋₁₀ cycloalkenyl, preferably C₆₋₃₀ aryl substituted by R^{e-1}.

In a preferred embodiment, R^{e-1} is independently one or more of halogen, C₁₋₁₀ alkoxy and 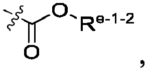 preferably halogen.

In a preferred embodiment, R^{f} is H, C₁₋₁₀ heterocycloalkyl or C₁₋₁₀ heterocycloalkyl substituted by R^{f-2}, preferably H or C₁₋₁₀ heterocycloalkyl.

In a preferred embodiment, X is N.

In a preferred embodiment, X is C₁₋₁₀ alkyleneoxy.

In a preferred embodiment, in the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof:
X is O;
R is C₆₋₃₀ aryl substituted by R¹, C₃₋₁₀ cycloalkenyl or C₃₋₁₀ cycloalkenyl substituted by R⁸;
R¹ is one or more of halogen, C₁₋₁₀ alkyl and C₁₋₁₀ alkoxy;
R^{e} is C₆₋₃₀ aryl substituted by R^{e-1} or C₃₋₁₀ cycloalkenyl;
R^{e-1} is one or more of halogen, C₁₋₁₀ alkoxy and 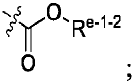 and,
R^{f} is H, C₁₋₁₀ heterocycloalkyl or C₁₋₁₀ heterocycloalkyl substituted by R^{f-2}.

In a preferred embodiment, in the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof:
X is O.
R is C₆₋₃₀ aryl substituted by R¹;
R¹ is one or more of halogen, C₁₋₁₀ alkyl and C₁₋₁₀ alkoxy;
R^{e} is C₆₋₃₀ aryl substituted by R^{e-1} or C₃₋₁₀ cycloalkenyl;
R^{e-1} is one or more of halogen, C₁₋₁₀ alkoxy and 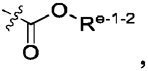 and at least one of the R^{e-1} is located at the ortho position carbon relative to carbon connected to the connection site; and,
R^{f} is H, C₁₋₁₀ heterocycloalkyl or C₁₋₁₀ heterocycloalkyl substituted by R^{f-2}.

In a preferred embodiment, in the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof:
X is O;
R is a C₆₋₃₀ aryl substituted by R¹;
R¹ is F, and at least one of the R¹ substitution sites is located at the ortho position C relative to the C connected to X;
R^{e} is C₆₋₃₀ aryl substituted by R^{e-1};
R^{e-1} is halogen, and at least one of the of R^{e-1} substitution sites is located at the ortho position C relative to C connected to the connection site; and,
R^{f} is H or C₁₋₁₀ heterocycloalkyl.

In a preferred embodiment, in the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof: -R is 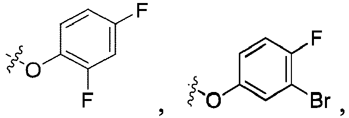 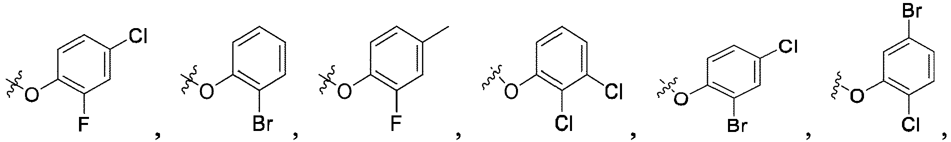 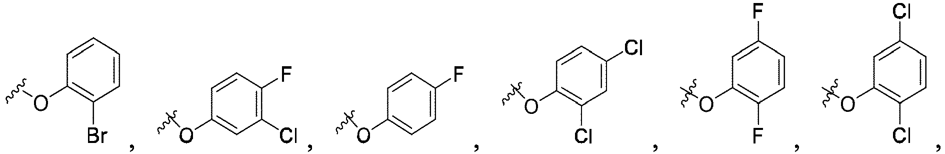 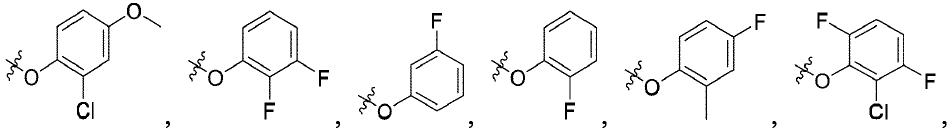 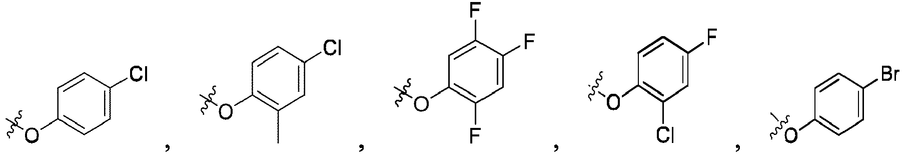 or

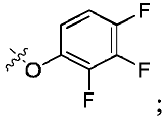

R^{e} is

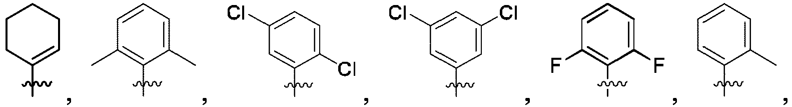

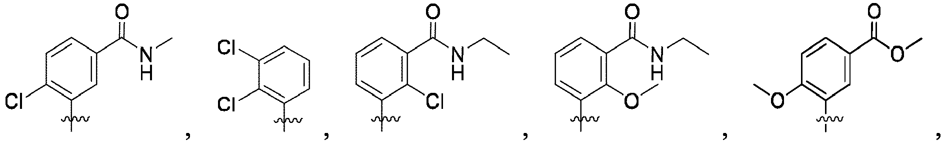

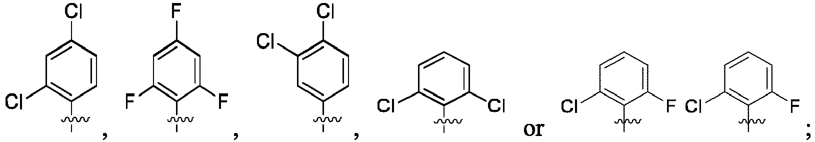

R^{f} is H,

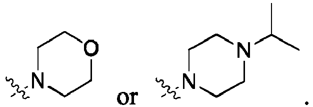

In a preferred embodiment, in the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof: X-R is

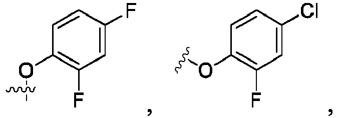

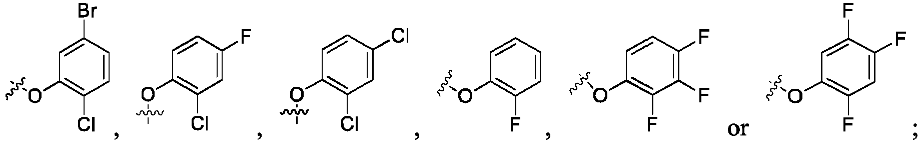

R^{e} is

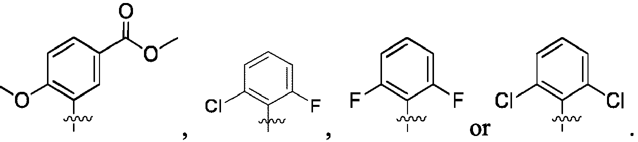

R^{f} is H,

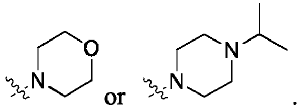

In a preferred embodiment, in the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof: X-R is

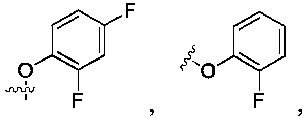

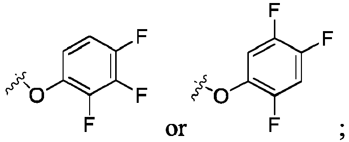

R^{e} is

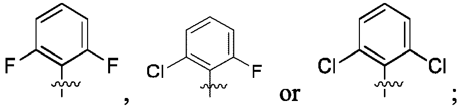

R^{f} is H or

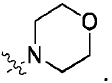

In a preferred embodiment, the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof is any of the following compounds: 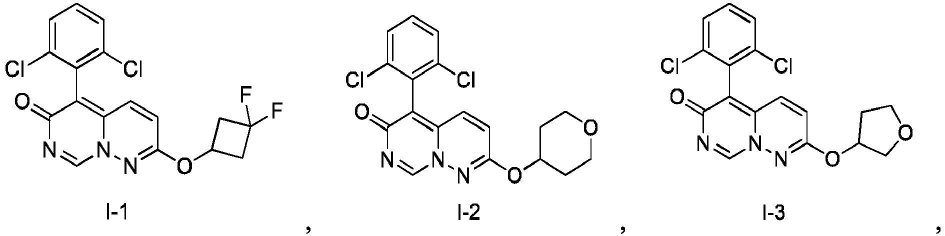 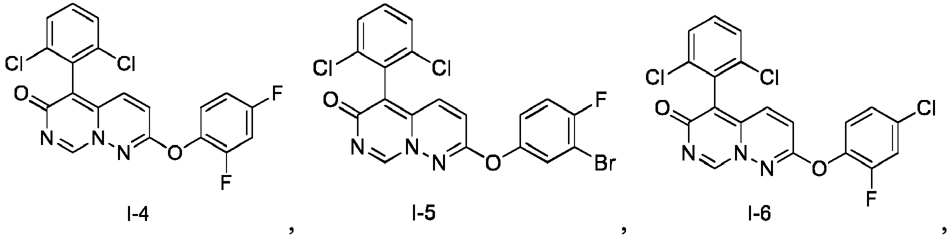 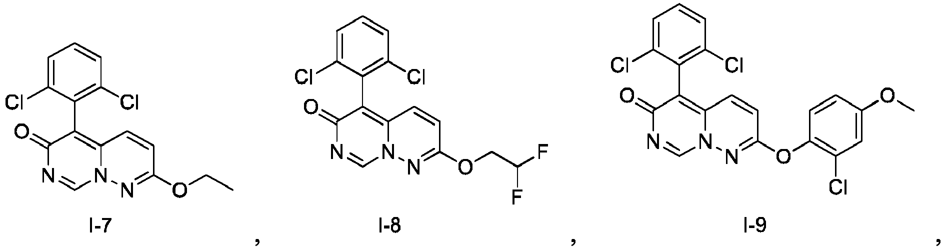 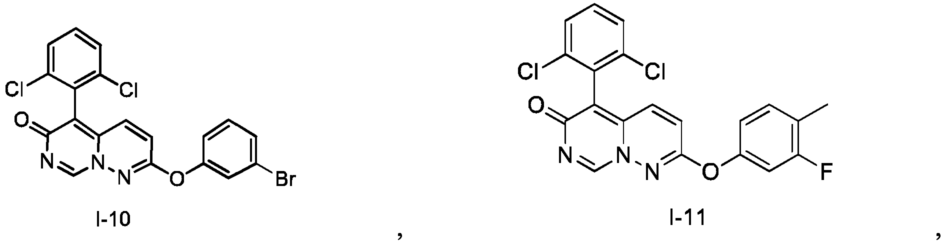 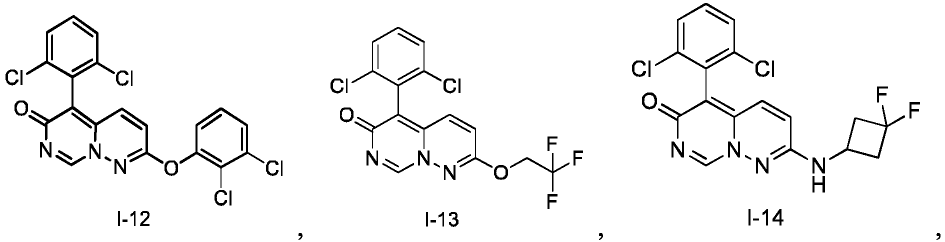 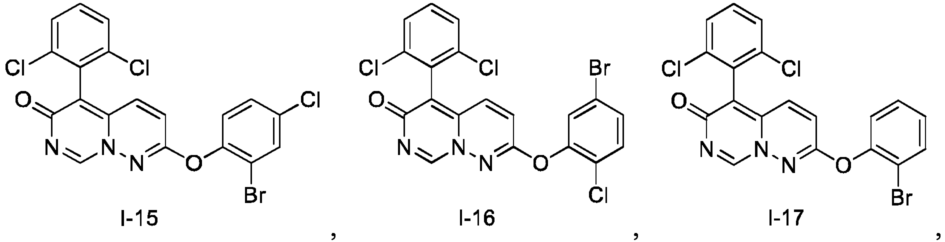 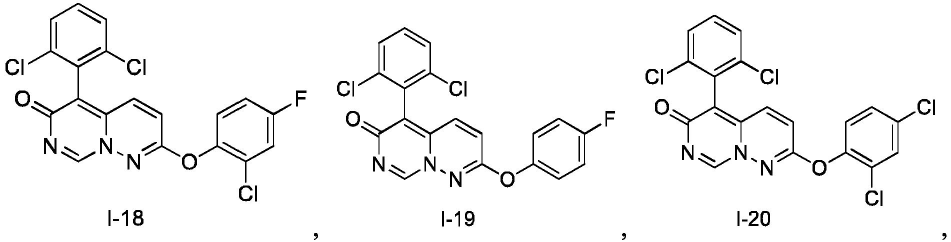 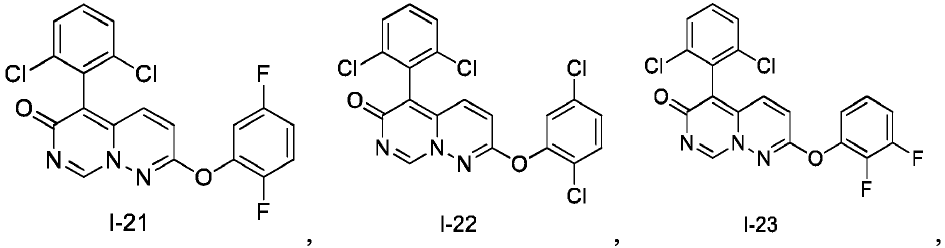 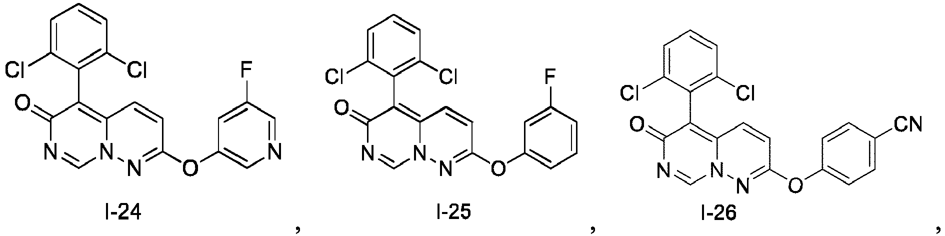 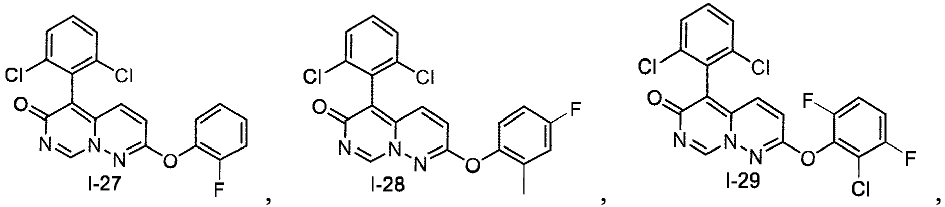 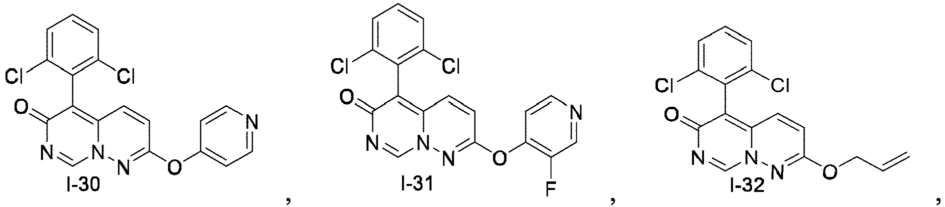 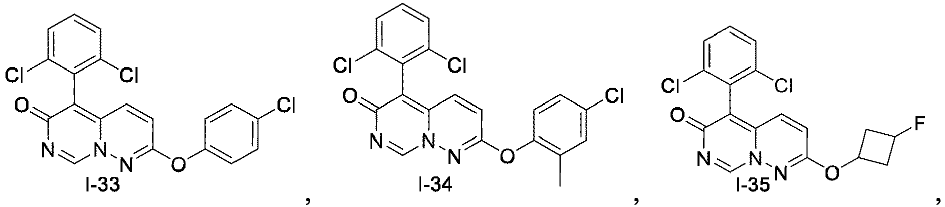 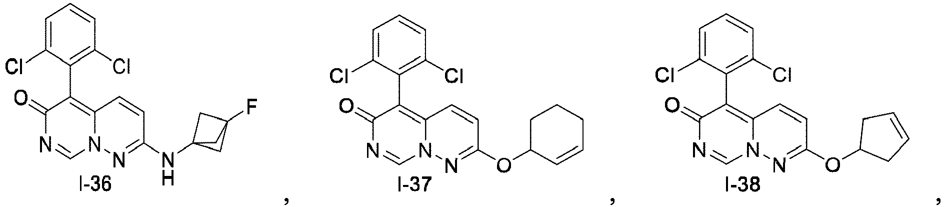 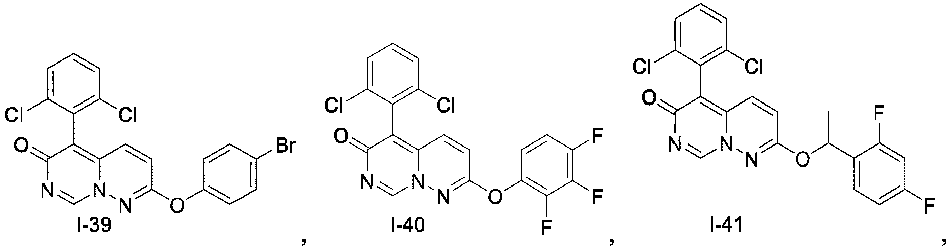 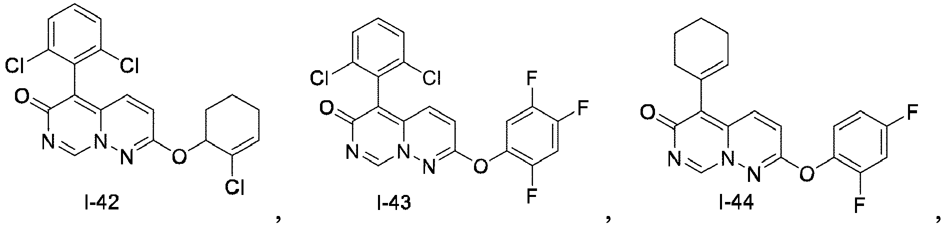 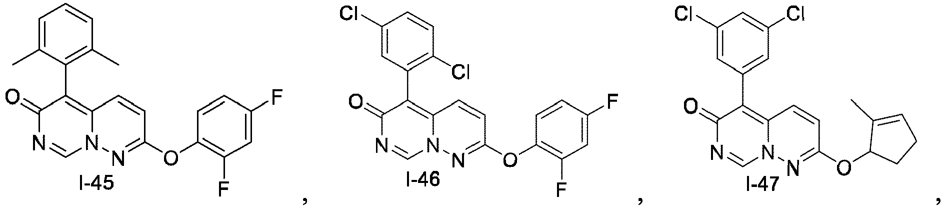 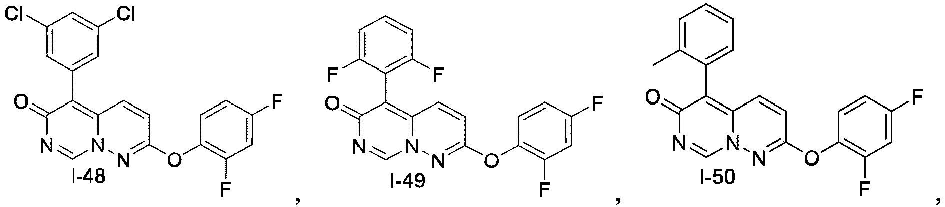 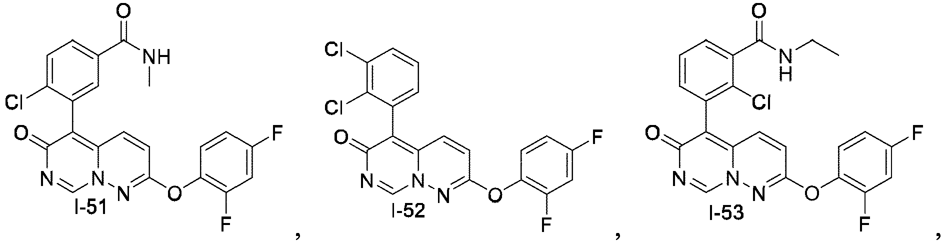 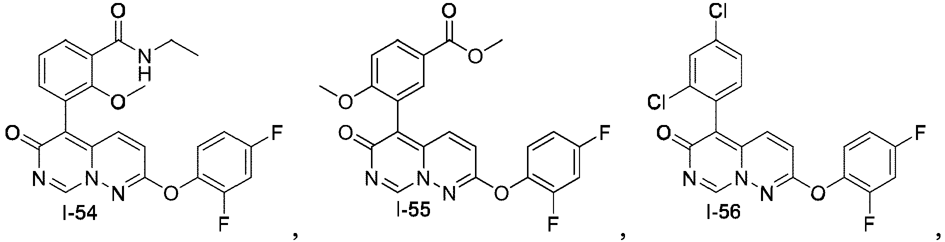 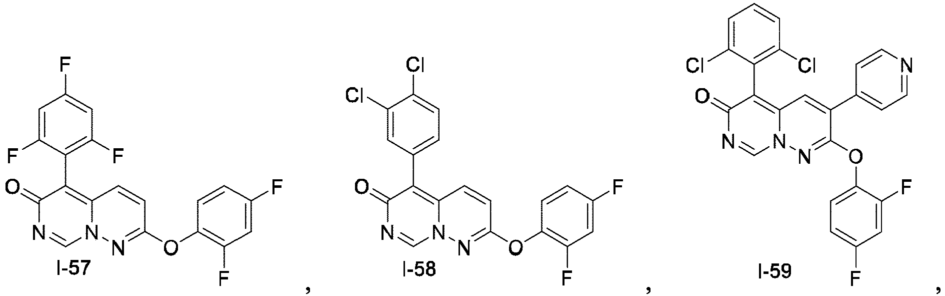 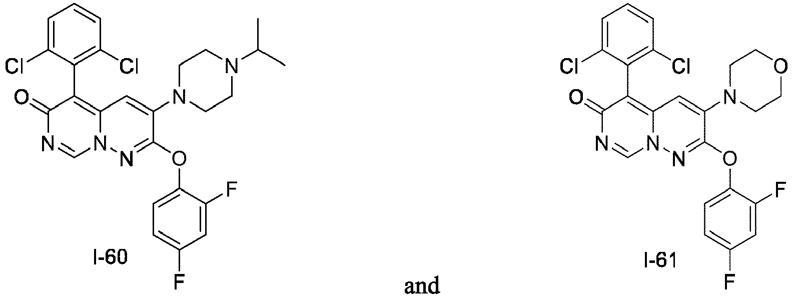 and

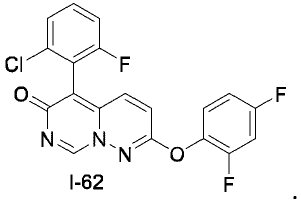

The present disclosure provides a method for preparing the nitrogen-containing heterocyclic compound represented by formula I, a pharmaceutically acceptable salt thereof, a hydrate thereof, a solvate thereof, a polymorph thereof, a metabolite thereof, a stereoisomer thereof, a tautomer thereof, or a prodrug thereof, comprising the following steps: carrying out a substitution reaction of compound II and compound III in the presence of a basic reagent in an organic solvent as shown below to obtain the nitrogen-containing heterocyclic compound represented by formula I, 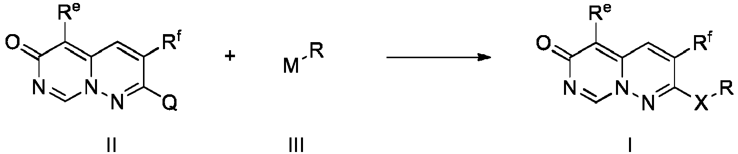 wherein, X, R, R^{e} and R^{f} are all defined as described above, Q is halogen and M is hydroxyl or amino.

The operation and conditions of the substitution reaction can be those conventional operation and conditions in the art for such reactions, and the following conditions are preferred for the present disclosure:
wherein, the halogen can be Cl, Br or I, preferably Cl.

In the substitution reaction, the basic reagent can be one or more of sodium hydrogen, alkali metal carbonate and organic weak base. The alkali metal carbonate can be one or more of potassium carbonate, sodium carbonate and cesium carbonate, preferably cesium carbonate. The organic weak base can be pyridine-like organic weak base and/or tertiary amine-like organic weak base. The pyridine-like organic weak base can be pyridine. The tertiary amine-like organic weak base can be triethylamine (TEA) or N,N-diisopropylethylamine (DIPEA).

In the substitution reaction, the molar ratio of the basic reagent to the compound II can be 1-10, preferably 1-4, for example 2, further for example 4.

In the substitution reaction, the molar ratio of the compound III to the compound II can be 1-10, preferably 1-3, for example from 1.5-2.

In the substitution reaction, the organic solvent can be one or more of halogenated hydrocarbon solvent, ether solvent, amide solvent, pyrrolidone solvent and nitrile solvent, preferably one or more of ether solvent, amide solvent and nitrile solvent, further preferably tetrahydrofuran, N,N-dimethylformamide or acetonitrile. The ether solvent can be one or more of tetrahydrofuran (THF), dioxane, ethyl ether and methyl tert-butyl ether (MTBE), preferably tetrahydrofuran. The amide solvents are preferably N,N-dimethylformamide (DMF) and/or N,N-dimethylacetamide (DMA). The pyrrolidone solvent is preferably N-methyl-pyrrolidone (NMP). The nitrile solvent can be acetonitrile.

In the substitution reaction, the temperature of the substitution reaction can be 20-120°C, preferably 25-100°C, for example 80°C, further for example 20°C.

The progress of the substitution reaction can be monitored by conventional monitoring methods in the art, (e.g. TLC or LCMS), the disappearance of the compound II is generally used as the end point of the reaction. The time of the reaction can be 2-16 hours, e.g. 3 hours, e.g. 16 hours.

After the completion of the substitution reaction, the following post-treatment steps can also be included: quenching the reaction solution after the reaction, extracting, drying, concentrating and purifying.

The operation and conditions of the quenching can be conventional operation and conditions in the art for such post-treatment of such reactions, preferably water quenching.

The operation and conditions of the extraction can be conventional operation and conditions in the art for such post-treatment of such reactions. The extraction solvent is preferably ester solvent, further preferably ethyl acetate.

The operation and conditions of the drying can be conventional operation and conditions in the art for such post-treatment of such reactions. The drying is preferably drying with a drying agent. The drying agent can be sodium sulfate and/or magnesium sulfate.

The method of purification can be a conventional purification method in such reactions in the art, preferably column chromatography, preparative thin-layer chromatography (TLC) or preparative high performance liquid chromatography (HPLC).

The present disclosure also provides a compound represented by formula II: 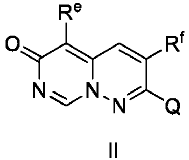 wherein, X, R, R^{e}, R^{f}, and Q are all defined as described above.

The compound II is preferably any of the following compounds:

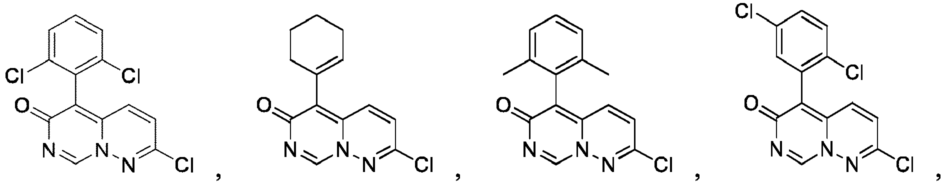

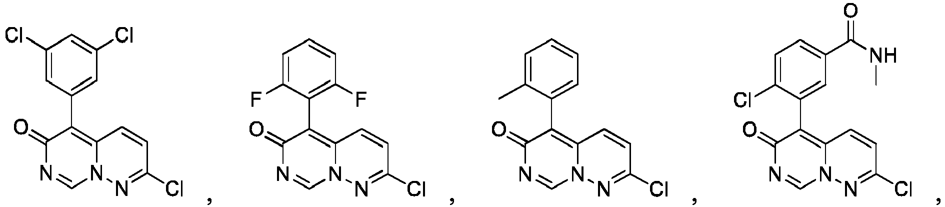

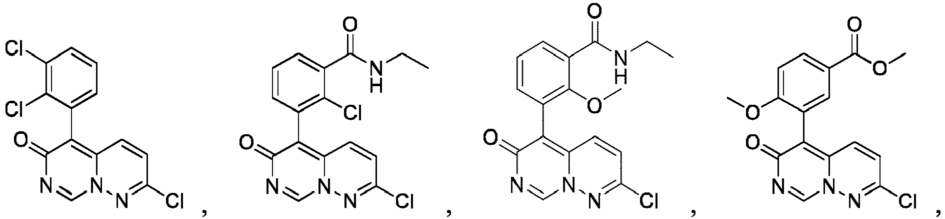

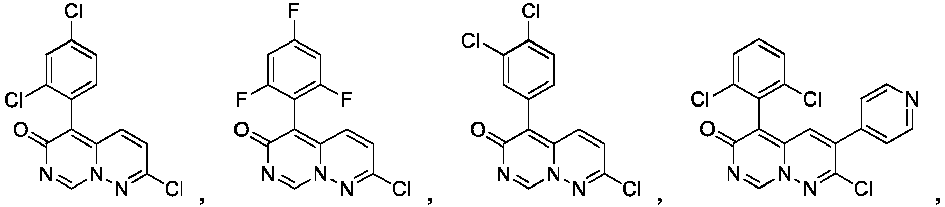

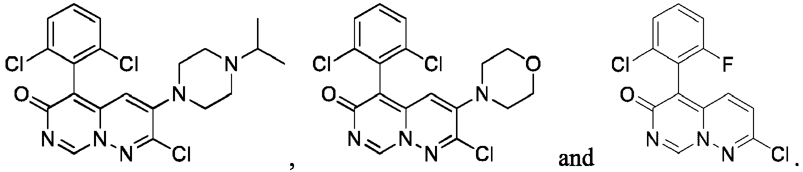

The present disclosure also provides a method for preparing the compound II, comprising the following step: carrying out a ring-closing reaction of compound IV and a ring-closing reagent in the presence of a basic reagent in an organic solvent as shown below to obtain the compound II, the ring-closing reagent is N,N-dimethylformamide dimethyl acetal and/or trimethyl orthoformate; 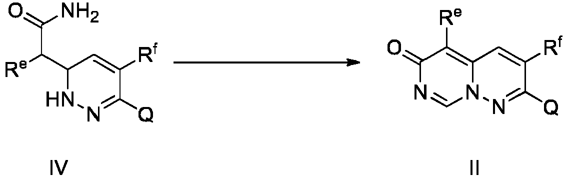 wherein, X, R, R^{e}, R^{f}, and Q are all defined as described above.

The operation and conditions of the ring-closing reaction can be conventional operation and conditions for such reactions in the art, and the following conditions are preferred for the present disclosure.
wherein, the halogen can be Cl, Br or I, preferably Cl.

The compound IV is preferably

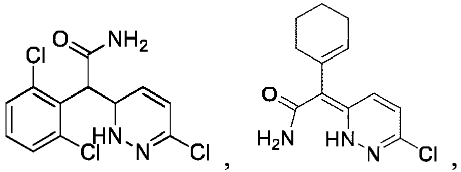

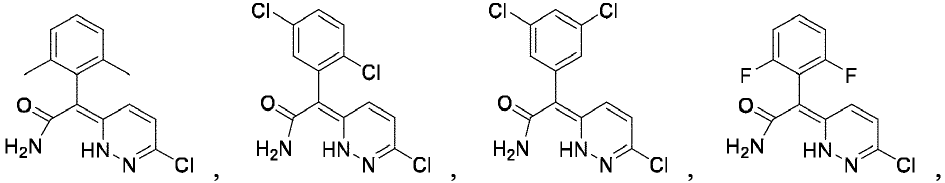

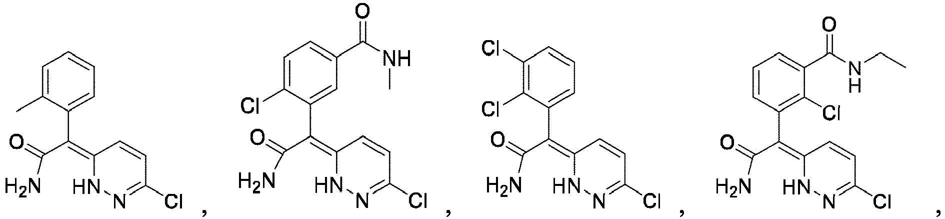

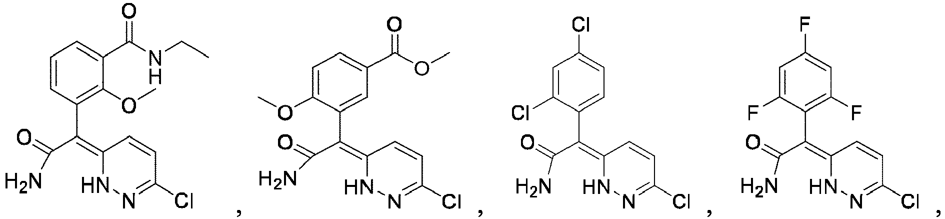

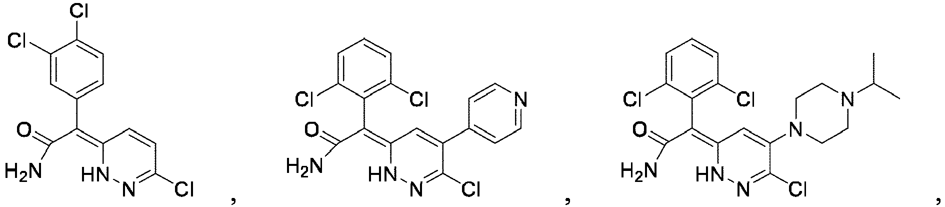

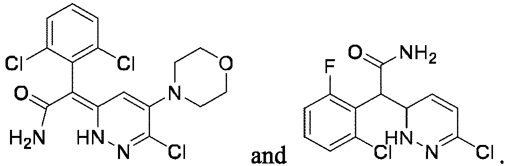

In the ring-closing reaction, the molar ratio of the compound IV to the ring-closing reagent can be 1-10, preferably 1-5, for example 3.

In the ring-closing reaction, the organic solvent can be aromatic hydrocarbon solvent. The aromatic hydrocarbon solvent is preferably toluene.

In the ring-closing reaction, the temperature of the substitution reaction can be 20-150°C, preferably 50-120°C, for example 50°C.

The progress of the ring-closing reaction can be monitored by conventional monitoring methods in the art, (e.g. TLC or LCMS), the disappearance of the compound IV is generally used as the end point of the reaction. The time of the reaction can be 2-6 hours, e.g. 3 hours.

After the completion of the ring-closing reaction, the following post-treatment step can also be included: after the reaction is completed, concentrating the reaction solution and purifying.

The operation and conditions of concentration can be those conventional operation and conditions in the art for such reactions, preferably reduced pressure concentration.

The purification method can be conventional purification method in such reactions in the art, preferably column chromatography, preparative thin-layer chromatography (TLC) or preparative high performance liquid chromatography (HPLC).

The present disclosure also provides a pharmaceutical composition, comprising the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof, and a pharmaceutically acceptable carrier.

In the present disclosure, the pharmaceutically acceptable carrier can be a conventional carrier in the art, comprising one or more of lubricant, binder, filler and disintegrant. The lubricant is selected from one or more of magnesium stearate, micronized silica, silica and talc; the disintegrant is selected from one or more of low-substituted hydroxypropyl cellulose, croscarmellose sodium, carboxymethyl starch sodium, starch and cross-linked povidone; the binder is selected from one or more of hydroxypropyl cellulose, polyvinylpyrrolidone and methyl cellulose; the filler is selected from one or more of lactose, pre-gelatinized starch and microcrystalline cellulose.

In the present disclosure, the amount of the pharmaceutically acceptable carrier is not particularly limited, as long as it meets the requirements of formulations conventional in the art.

The present disclosure also provides a use of the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof, in manufacturing a medicament.

The medicament can be used to treat or prevent related disease mediated by p38 protein kinase. The p38 protein kinase can be p38α protein kinase and/or a p38β protein kinase, and can also be p38α protein kinase.

The disease refers to any disease or other deleterious condition in which p38 protein kinase is known to play a direct or indirect role, and can be a disease caused by inhibition of overexpression of IL-1, TNF, IL-6 or IL-8, a disease caused by hyperphosphorylation of p38 substrates such as tau, or a disease caused by alteration of the p38-MAPK pathway, and can also be a disease caused by inhibition of overexpression of TNF, and further, can be one or more of inflammatory disease, autoimmune disease, destructive bone disease, neurodegenerative disease, stroke, prostaglandin endoperoxide synthase-2 (pain) related disease, cardiac disease, proliferative disease, allergy, and cancer.

The present disclosure also provides a use of the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof, or the pharmaceutical composition above, in manufacturing a medicament, and the medicament is used to treat or prevent disease, the disease is one or more of inflammatory disease, autoimmune disease, destructive bone disease, neurodegenerative disease, stroke, prostaglandin endoperoxide synthase-2 (pain) related disease, cardiac disease, proliferative disease, allergy, and cancer.

The present disclosure also provides a use of the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof, or the pharmaceutical composition above, in manufacturing p38 protein kinase inhibitor.

In the use described above, the p38 protein kinase inhibitor can be an in vivo or in vitro inhibitor.

In the use described above, the p38 protein kinase can be p38α protein kinase and/or p38β protein kinase, and can also be p38α protein kinase.

The present disclosure also provides a method for treating disease, comprising administering to a subject in need thereof an effective amount of the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof, or the pharmaceutical composition above; wherein the disease is related disease mediated by p38 protein kinase.

In the method, the p38 protein kinase and the disease are the same as those described previously.

The present disclosure also provides a method for treating disease, comprising administering to a subject in need thereof an effective amount of the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof, or the pharmaceutical composition above; wherein the disease is one or more of inflammatory disease, autoimmune disease, destructive bone disease, neurodegenerative disease, stroke, prostaglandin endoperoxide synthase-2 (pain) related disease, cardiac disease, proliferative disease, allergy, and cancer.

In a preferred embodiment of the present disclosure, he nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof is a compound represented by formula IA: 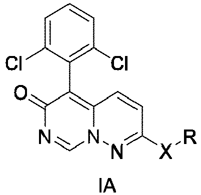
wherein, X is O or N;
R is C₆₋₃₀ aryl, C₆₋₃₀ aryl substituted by R¹, C₁₋₃₀ heteroaryl, C₁₋₃₀ heteroaryl substituted by R², C₁₋₁₀ alkyl, C₁₋₁₀ alkyl substituted by R³, C₃₋₁₀ cycloalkyl, C₃₋₁₀ cycloalkyl substituted by R⁴, C₁₋₁₀ heterocycloalkyl, C₁₋₁₀ heterocycloalkyl substituted by R⁵, C₂₋₁₀ alkenyl, C₂₋₁₀ alkenyl substituted by R⁶, C₂₋₁₀ alkynyl, C₂₋₁₀ alkynyl substituted by R⁷;
R¹, R², R³, R⁴, R⁵, R⁶ and R⁷ are independently selected from one or more of halogen, C₁₋₁₀ alkyl, C₁₋₁₀ alkoxy, 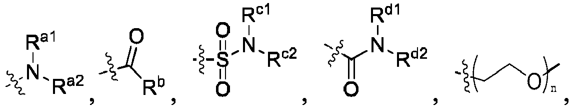 C6-30 aryl, C₁₋₆ heterocycloalkyl, hydroxyl and cyano;
n is 1, 2 or 3;
R^{a1}, R^{a2}, R^{b}, R^{c1}, R^{c2}, R^{d1} and R^{d2} are independently selected from H, C₁₋₆ alkyl, C₁₋₆ alkoxy, C₂₋₆ alkenyl, C₂₋₆ alkynyl, C₆₋₃₀ aryl or C₆₋₃₀ aryl substituted by R^{a-1};
R^{a-1} is selected from one or more of C₁₋₃ alkyl, C₁₋₃ alkoxy, halogen, cyano, nitro, amino and hydroxyl;
In the C₁₋₃₀ heteroaryl, the C₁₋₃₀ heteroaryl substituted by R², the C₁₋₁₀ heterocycloalkyl, the C₁₋₁₀ heterocycloalkyl in the C₁₋₁₀ heterocycloalkyl substituted by R⁴, and, in the C₁₋₆ heterocycloalkyl in "R¹, R², R³, R⁴, R⁵, R⁶, and R⁷ ", the number of heteroatom is independently 1-4, and the heteroatom is independently one or more of O, S and N.

In R, the C₆₋₃₀ aryl in the C₆₋₃₀ aryl substituted by R¹ can be C₆₋₁₈ aryl, preferably C₆₋₁₄ aryl. The C₆₋₁₄ aryl is preferably phenyl, naphthyl or phenanthryl, more preferably phenyl.

In R¹, the halogen can be F, Cl, Br or I, preferably F or Cl, more preferably F.

In R¹, the C₁₋₁₀ alkyl can be C₁₋₆ alkyl, preferably C₁₋₃ alkoxy, further preferably methyl.

In R¹, the C₁₋₁₀ alkoxy can be C₁₋₆ alkoxy, preferably C₁₋₃ alkoxy, further preferably methoxy.

In R, the C₁₋₃₀ heteroaryl in the C₁₋₃₀ heteroaryl and the C₁₋₃₀ heteroaryl in C₁₋₃₀ heteroaryl substituted by R² can be C₁₋₁₈ heteroaryl, preferably C₁₋₁₄ heteroaryl. The C₁₋₁₄ heteroaryl is preferably pyridyl.

In R², the halogen can be F, Cl, Br or I, preferably F, Cl or Br.

In R, the C₁₋₁₀ alkyl can be C₁₋₆ alkyl, preferably C₁₋₃ alkyl. The C₁₋₃ alkyl can be methyl, ethyl, propyl or isopropyl, preferably ethyl.

In R, the C₁₋₁₀ alkyl in the C₁₋₁₀ alkyl substituted by R³ can be C₁₋₆ alkyl, preferably C₁₋₃ alkyl. The C₁₋₃ alkyl can be methyl, ethyl, propyl or isopropyl, preferably methyl or ethyl.

In R³, the halogen can be F, Cl, Br or I, preferably F.

In R, the C₃₋₁₀ cycloalkyl in the C₃₋₁₀ cycloalkyl and the C₃₋₁₀ cycloalkyl substituted by R⁴ can independently be C₃₋₁₀ monocycloalkyl or C₅₋₁₀ bridged cycloalkyl, preferably C₃₋₆ monocycloalkyl or C₅₋₈ bridged cycloalkyl.

In R⁴, the halogen can be F, Cl, Br or I, preferably F, Cl or Br, more preferably F.

In R, the C₁₋₁₀ heterocycloalkyl can be C₂₋₆ heterocycloalkyl, further preferably heterocyclopentane or heterocyclohexane.

In R, the C₁₋₁₀ heterocycloalkyl in C₁₋₁₀ heterocycloalkyl substituted by R⁵ can be C₂₋₆ heterocycloalkyl.

In R, the C₂₋₁₀ alkenyl can be C₂₋₆ alkenyl, preferably C₂₋₃ alkenyl. The C₂₋₃ alkenyl can be

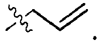

In R, the C₂₋₁₀ alkenyl in the C₂₋₁₀ alkenyl substituted by R⁶ can be C₂₋₆ alkenyl.

In R, the C₂₋₁₀ alkynyl in the C₂₋₁₀ alkynyl and the C₂₋₁₀ alkynyl substituted by R⁷ can independently be C₂₋₆ alkynyl.

The C₆₋₃₀ aryl in R, C₆₋₃₀ aryl in" R¹, R², R³, R⁴, R⁵, R⁶, and R⁷", C₆₋₃₀ aryl in "R^{a1}, R^{a2}, R^{b}, R^{c1}, R^{c2}, R^{d1} and R^{d2}", and, C₆₋₃₀ aryl in the C₆₋₃₀ aryl substituted by R^{a-1}, are independently preferably C₆₋₁₈ aryl.

The X is preferably O.

The R is preferably C₆₋₃₀ aryl, C₆₋₃₀ aryl substituted by R¹, C₁₋₃₀ heteroaryl, C₁₋₃₀ heteroaryl substituted by R², C₁₋₁₀ alkyl, C₁₋₁₀ alkyl substituted by R³, C₃₋₁₀ cycloalkyl, C₃₋₁₀ cycloalkyl substituted by R⁴, C₁₋₁₀ heterocycloalkyl, or, C₁₋₁₀ heterocycloalkyl substituted by R⁵;
more preferably C₆₋₃₀ aryl, C₆₋₃₀ aryl substituted by R¹, C₁₋₃₀ heteroaryl, C₁₋₃₀ heteroaryl substituted by R²;
most preferably, C₆₋₃₀ aryl substituted by R¹.

In the C₆₋₃₀ aryl substituted by R¹, the R¹ is preferably one or more of halogen, C₁₋₁₀ alkyl, C₁₋₁₀ alkoxy and cyano, more preferably halogen.

In the C₆₋₃₀ aryl substituted by R¹, the number of the R¹ can be 1, 2 or 3, preferably 2. When the number of R¹ is more than one, then R¹ can be the same or different, preferably the same.

In R, the C₆₋₃₀ aryl substituted by R¹ is preferably C₆₋₁₈ aryl substituted by one or more of halogen, C₁₋₁₀ alkyl, C₁₋₁₀ alkoxy and cyano, more preferably C₆₋₁₄ aryl substituted by one or more of halogen, C₁₋₁₀ alkyl, C₁₋₁₀ alkoxy and cyano, further preferably 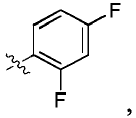 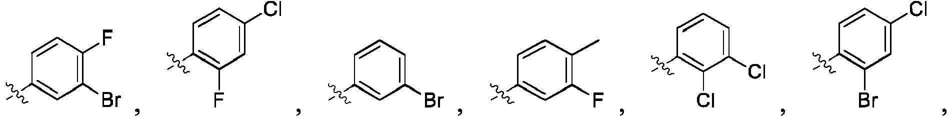 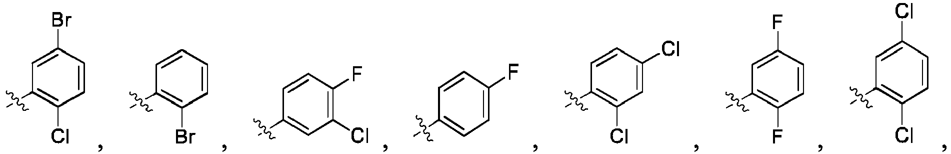 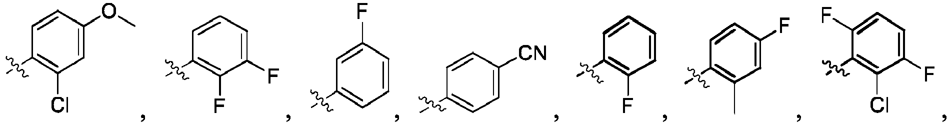 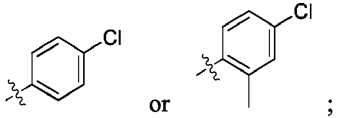 more further preferably 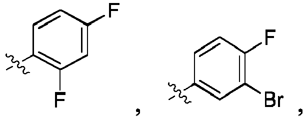 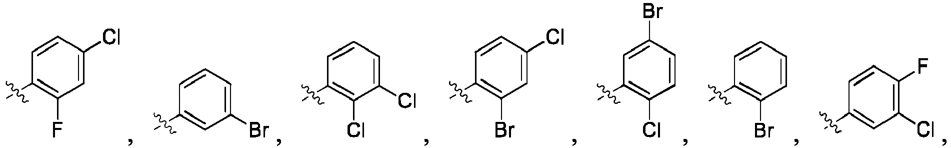 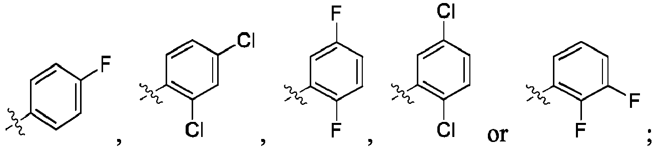 and more further preferably

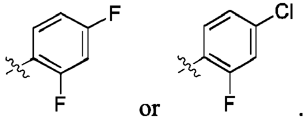

In the C₁₋₃₀ heteroaryl substituted by R², the R² is preferably halogen.

In the C₁₋₃₀ heteroaryl substituted by R², the number of the R² can be 1, 2 or 3, preferably 1 or 2. When the number of R² is more than one, then R² can be the same or different, preferably the same.

In the C₁₋₃₀ heteroaryl and the C₁₋₃₀ heteroaryl substituted by R², the number of heteroatom in the C₁₋₃₀ heteroaryl is independently preferably 1 or 2, more preferably 1.

In the C₁₋₃₀ heteroaryl and the C₁₋₃₀ heteroaryl substituted by R², the heteroatom in the C₁₋₃₀ heteroaryl is independently preferably N.

In R, the C₁₋₃₀ heteroaryl substituted by R² is preferably C₁₋₁₈ heteroaryl substituted by halogen, and the heteroatom is N, further preferably

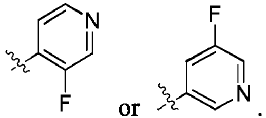

In the C₁₋₁₀ alkyl substituted by R³, the R³ is preferably halogen.

In the C₁₋₁₀ alkyl substituted by R³, the number of the R³ can be 1, 2 or 3. When the number of R³ is more than one, then R³ can be the same or different, preferably the same.

In R, the C₁₋₁₀ alkyl substituted by R³ is preferably C₁₋₆ alkyl substituted by halogen, further preferably

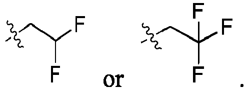

In the C₃₋₁₀ cycloalkyl substituted by R⁴, the R⁴ is preferably halogen.

In the C₃₋₁₀ cycloalkyl substituted by R⁴, the number of the R⁴ can be 1, 2, 3 or 4, preferably 2. When the number of R⁴ is more than one, R⁴ can be the same or different, preferably the same.

In R, the number of heteroatom in the C₁₋₁₀ heterocycloalkyl is preferably 1 or 2, more preferably 1.

In R, the heteroatom in the C₁₋₁₀ heterocycloalkyl is preferably O.

In R, the C₁₋₁₀ heterocycloalkyl is preferably "C₂₋₆ heterocycloalkyl and the heteroatom is O", further preferably

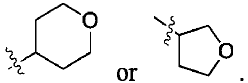

In R, the C₃₋₁₀ cycloalkyl substituted by R⁴ is preferably C₃₋₆ monocycloalkyl substituted by halogen, further preferably

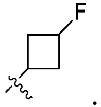

**In R,** C₃₋₁₀ cycloalkyl substituted by R⁴ is preferably C₅₋₁₀ bridged cycloalkyl substituted by halogen, further preferably

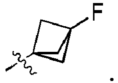

In a preferred embodiment, in the nitrogen-containing heterocyclic compound represented by formula I, when R is a C₆₋₃₀ aryl substituted by R¹, then X is O.

In a certain preferred embodiment, in the nitrogen-containing heterocyclic compound represented by formula I, the C₆₋₃₀ aryl in the C₆₋₃₀ aryl substituted by R¹ is phenyl, and the substitution site of R¹ is one or more of the 2-position, 3-position, 4-position and 5-position relative to the phenyl.

In a certain preferred embodiment, in the nitrogen-containing heterocyclic compound represented by formula I, the C₆₋₃₀ aryl in the C₆₋₃₀ aryl substituted by R¹ is phenyl, the number of R¹ is 2, and the R¹ substitution sites are the 2-position and 4-position of the phenyl.

In a certain preferred embodiment, in the nitrogen-containing heterocyclic compound represented by formula I, the C₆₋₃₀ aryl in the C₆₋₃₀ aryl substituted by R₁ is phenyl, and the R¹ substitution sites are the 2-position and 4-position of the phenyl, and the R¹ is halogen.

In a preferred embodiment, in the nitrogen-containing heterocyclic compound represented by formula I, the C₆₋₃₀ aryl in the C₆₋₃₀ aryl substituted by R¹ is phenyl, the X is O, and the R¹ substitution sites are the 2-position and 4-position of the phenyl, and the R¹ is halogen.

In a preferred embodiment, in the nitrogen-containing heterocyclic compound represented by formula I, X is O; R is C₆₋₃₀ aryl, C₆₋₃₀ aryl substituted by R¹, C₁₋₃₀ heteroaryl, C₁₋₃₀ heteroaryl substituted by R², C₁₋₁₀ alkyl, C₁₋₁₀ alkyl substituted by R³, C₃₋₁₀ cycloalkyl, C₃₋₁₀ cycloalkyl substituted by R⁴, C₁₋₁₀ heterocycloalkyl, C₁₋₁₀ heterocycloalkyl substituted by R⁵ or C₂₋₆ alkenyl.

In a preferred embodiment, in the nitrogen-containing heterocyclic compound represented by formula I, X is O; R is C₆₋₁₈ aryl substituted by one or more of halogen, C₁₋₁₀ alkyl, C₁₋₁₀ alkoxy and cyano, C₁₋₁₈ heteroaryl substituted by halogen and the heteroatom is N, C₁₋₆ alkyl, C₁₋₆ alkyl substituted by halogen, C₃₋₆ monocycloalkyl substituted by halogen, C₅₋₁₀ bridged cycloalkyl substituted by halogen, C₂₋₆ heterocycloalkyl with atom O or C₂₋₆ alkenyl.

The present disclosure also provides a use of the nitrogen-containing heterocyclic compound represented by formula I, a pharmaceutically acceptable salt thereof, a hydrate thereof, a solvate thereof, a polymorph thereof, a metabolite thereof, a stereoisomer thereof, a tautomer thereof, or a prodrug thereof in manufacturing p38 protein kinase inhibitor.

The present disclosure also provides a use of the nitrogen-containing heterocyclic compound represented by formula I, a pharmaceutically acceptable salt thereof, a hydrate thereof, a solvate thereof, a polymorph thereof, a metabolite thereof, a stereoisomer thereof, a tautomer thereof, or a prodrug thereof in manufacturing p38-mediated disease medicament.

The "p38-mediated disease" refers to any disease or other deleterious condition in which p38 is known to play a direct or indirect role. These include diseases caused by the inhibition of overexpression of IL-1, TNF, IL-6, IL-8, diseases caused by hyperphosphorylation of p38 substrates such as tau, or diseases caused by alterations in the p38-MAPK pathway. These diseases include, but are not limited to, inflammatory disease, autoimmune disease, destructive bone disease, neurodegenerative disease, stroke, prostaglandin endoperoxide synthase-2 (pain) related disease, cardiac disease, proliferative disease, allergy, cancer, etc.

The present disclosure also provides a pharmaceutical composition, comprising the nitrogen-containing heterocyclic compound represented by formula I, a pharmaceutically acceptable salt thereof, a hydrate thereof, a solvate thereof, a polymorph thereof, a metabolite thereof, a stereoisomer thereof, a tautomer thereof, or a prodrug thereof, and a pharmaceutically acceptable carrier.

In the present disclosure, the pharmaceutically acceptable carrier can be a **conventional** carrier in the art, comprising one or more of lubricant, binder, filler and disintegrant. The lubricant is selected from one or more of magnesium stearate, micronized silica, silica and talc; the disintegrant is selected from one or more of low-substituted hydroxypropyl cellulose, croscarmellose sodium, carboxymethyl starch sodium, starch and cross-linked povidone; the binder is selected from one or more of hydroxypropyl cellulose, polyvinylpyrrolidone and methyl cellulose; the filler is selected from one or more of lactose, pre-gelatinized starch and microcrystalline cellulose.

In the present disclosure, the term "aryl" refers to a group of 4n+2 aromatic ring system (e.g., with 6, 10, or 14 shared p-electrons in a circular array) with 6-14 ring atoms and zero heteroatom provided in an aromatic ring system in a monocyclic or polycyclic (e.g., bicyclic or tricyclic) ("C₆₋₁₄ aryl"), such as phenyl, naphthyl, phenanthryl, or anthryl.

In the present disclosure, the term "heteroaryl" refers to a 5-10 membered monocyclic or bicyclic 4n+2 aromatic ring system with a ring carbon atom and 1-4 ring heteroatom (each of which is independently selected from nitrogen, oxygen, and sulfur) provided in the aromatic ring system (e.g., with 6 or 10 shared p-electrons in a circular array) ("5-10 membered heteroaryl"). Heteroaryl within this definition include, but is not limited to: acridinyl, carbazolyl, cinnolinyl, quinoxalinyl, pyrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrole, tetrahydroquinolinyl. The term "heteroaryl" should also be understood to include any N-oxide derivative of any nitrogen-containing heteroaryl.

In the present disclosure, the term "cycloalkyl", when used herein alone or as part of another group, refers to a 3-12-membered monocyclic group or 5-12-membered polycyclic group (e.g., bridged, spiro), wherein each ring may contain one or more double bonds, but none of which has a fully conjugated π-electron system, such as saturated cycloalkyl, cycloalkenyl. Saturated cycloalkyl is a 3-12-membered monocyclic group or 5-12-membered polycyclic group (e.g., bridged, spiro), e.g, 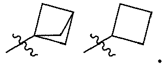 Cycloalkenyl is 3-12-membered monocyclic group with one or more double bonds, and the position of the double bond can be anywhere in the ring.

In the present disclosure, the term "heterocycloalkyl", when used herein alone or as part of another group, refers to a 4-12-membered monocyclic or polycyclic (e.g., bridged, spirocyclic) group containing 1-4 heteroatom, which is selected from O, N, and S, wherein each ring may contain one or more double bonds, but none of the rings has a fully conjugated π electron system, e.g., furanyl, morpholinyl, azetidinyl, pyrrolidinyl.

In the present disclosure, "C₁₋₁₀ alkyl" includes linear or branched C₁₋₁₀ alkyl.

In the present disclosure, "C₁₋₁₀ alkyl substituted by halogen" means C₁₋₁₀ alkyl substituted by halogen at any position.

In the present disclosure, the double bond in "C₂₋₁₀ alkenyl" and "C₂₋₁₀ alkenyl" in "C₂₋₁₀ alkenyl substituted by R⁶" can be in any position of C₂₋₁₀ alkenyl, including linear or branched C₂₋₁₀ alkenyl.

In the present disclosure, the triple bond in "C₂₋₁₀ alkynyl" and the "C₂₋₁₀ alkynyl" in "C₂₋₁₀ alkynyl substituted by R⁷" can be in any position of C₂₋₁₀ alkynyl, including linear or branched C₂₋₁₀ alkynyl.

In the present disclosure, the phrase of substituents are the same or different includes the substituents and the type of substituents are the same or different, for example 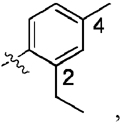 the substituent at the 2-position on the phenyl is ethyl and the substituent at the 4-position is methyl, in the present disclosure, the ethyl at the 2-position on the phenyl and the methyl at the 4-position are also called the substituents are the same; for example 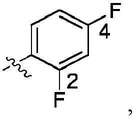 the substituent at the 2-position on the phenyl is F and the 4-position substituent is F, in the present disclosure, the 2-position and 4-position substituents on the phenyl group are the same.

In the present disclosure, the substitution position of a substituent is located at the ortho position C relative to a C connected a certain atom, which can also be referred to as the substitution position of a substituent is 2-position, and correspondingly, the substitution position of a substituent is located at the para position C relative to a C connected to a certain atom, which may also be referred to as the substitution position of a substituent is 4-position; for example 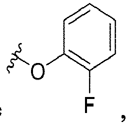 wherein the substitution position of F is located at the ortho position C relative to the C connected to O, or wherein the substitution position of F is the 2-position of phenyl; and also for example 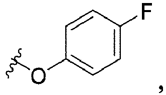 wherein the substitution position of F is located at the para position C relative to a the C connected to O, or the substitution site of F is at the 4-position of the phenyl.

In the present disclosure, "room temperature" refers to 10-30°C.

In the present disclosure, "overnight" means 12 to 16 hours.

In the present disclosure, "therapeutically effective amount" is an amount of a compound that is sufficient to effectively treat the disease or condition described herein, when administered to a patient. The "therapeutically effective amount" will vary depending on the compound, the disease and its severity, and the age of the patient to be treated, but can be adjusted by those skilled in the art as needed.

Each of the above preferred conditions can be combined in any way to obtain a better Embodiment of the invention without violating the common knowledge in the art.

The positive compound VX-745 in the present disclosure is homemade, and its preparation method is referred to Embodiment 5 in CN1244867A. Other reagents and raw materials are commercially available.

The positive progressive effect of the present disclosure is that the nitrogen-containing heterocyclic compounds of the present disclosure have better inhibitory activity and/or higher bioavailability for p38 protein kinase.

### Brief description of the drawings

Fig.1 is a histogram of the drug-time-concentration distribution in mice brain, wherein, 1 is compound I-4; 2 is the positive compound VX-745.
Fig. 2 is a histogram of the expression of TNF-α inflammatory factor in serum detected by ELISA kit, where "**" indicates p<0.01.

### Detailed description of the preferred embodiment

The following Embodiments further illustrate the present disclosure, but the present disclosure is not limited thereto. In the following Embodiments, the experimental methods without specific conditions are selected according to conventional methods and conditions, or according to the product specification.

### Embodiment 1 Synthesis of 5-(2,6-dichlorophenyl)-2-(3,3-difluorocyclobutoxy)-6H-pyrimido[1,6-blpyridazin-6-one (I-1)

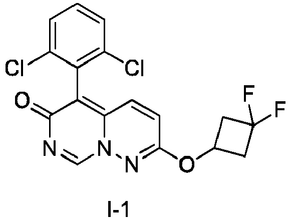

### Step A: Preparation of 2-(6-chloropyridazin-3-yl)-2-(2,6-dichlorophenyl) acetonitrile.

2-(2,6-Dichlorophenyl)acetonitrile (5 g, 26.88 mmol) and 3,6-dichloropyridazine (4 g, 26.85 mmol) were dissolved in tetrahydrofuran (75 mL), and potassium tert-butoxide (4.5 g, 40 mmol) was added at 0°C. The reaction solution was heated to 75°C and stirred overnight. The reaction solution was neutralized with IN hydrochloric acid, extracted with ethyl acetate (80 mL x 3). The organic phases were combined, dried over anhydrous sodium sulfate, filtered and concentrated under reduced pressure to obtain a residue, which was purified by silica gel column chromatography to obtain 6.5 g of red solid with a yield of 81%.

### Step B: Preparation of 2-(6-chloropyridazin-3-yl)-2-(2,6-dichlorophenyl) acetamide.

2-(6-Chloro-pyridazin-3-yl)-2-(2,6-dichlorophenyl)acetonitrile (1.72 g, 5.76 mmol) was added to concentrated sulfuric acid (15 mL) and stirred at 100°C for 2 hours. The reaction solution was poured into water and extracted with ethyl acetate (50 mL x 3). The organic phases were combined, dried over anhydrous sodium sulfate, filtered, and concentrated under reduced pressure to obtain 1.3 g of crude yellow solid, which was directly used in the next reaction.

### Step C: Preparation of 2-chloro-5-(2,6-dichlorophenyl)-6H-pyrimido[1,6-b] pyridazin-6-one.

2-(6-Chloro-pyridazin-3-yl)-2-(2,6-dichlorophenyl)acetamide (1.28 g, 4.04 mmol) and N,N-dimethylformamide dimethyl acetal (1.45 g, 12.2 mmol) were added to toluene (20 mL). The reaction solution was stirred at 50°C for 3 hours. The reaction solution was concentrated under reduced pressure to obtain a residue, which was purified by silica gel column chromatography to obtain 400 mg of yellow solid with a yield of 30%.

### Step D: Preparation of 5-(2,6-dichlorophenyl)-2-(3,3-difluorocyclobutoxy)-6H-pyrimido[1,6-b]pyridazin-6-one.

3,3-Difluorocyclobutanol (90 mg, 0.83 mmol) was dissolved in tetrahydrofuran (5 mL), and sodium hydrogen (88 mg, 2.2 mmol) was added at 0°C. After stirring for 15 minutes, 2-chloro-5-(2,6-dichlorophenyl)-6*H*-pyrimido[1,6-b]pyridazin-6-one (180 mg, 0.55 mmol) was added, and the final reaction solution was stirred at room temperature for 3 hours. The reaction was quenched with water and the obtained solution was extracted with ethyl acetate (50 mL x 3). The organic phases were combined, dried over anhydrous sodium sulfate, filtered and concentrated under reduced pressure to obtain a residue, which was purified by silica gel column chromatography to obtain 90 mg of yellow solid with a yield of 41%. LC-MS: 398.1 [M+H] detection value; ¹H NMR (400 MHz, CDCl₃) δ 8.79 (s, 1H), 7.47 (d, *J* = 8.4 Hz, 2H), 7.35 (dd, *J* = 8.4, 7.6 Hz, 1H), 7.00 (d, *J* = 10.0 Hz, 1H), 6.64 (d, *J* = 10.0 Hz, 1H), 5.20-5.08 (m, 1H), 3.26-3.17 (m , 2H), 2.94-2.78 (m, 2H).

### Embodiment 2 Synthesis of 5-(2,6-dichlorophenyl)-2-((tetrahydro-2H-pyran-4-yl)oxy)-6H-pyrimido [1,6-b]pyridazin-6-one(I-2)

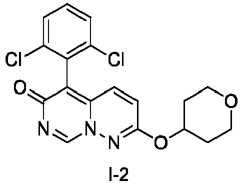

Tetrahydro-2H-pyran-4-ol (47 mg, 0.16 mmol) was dissolved in N,N-dimethylformamide (5 mL), and sodium hydride (36.8 mg, 0.92 mmol) was added at 0°C. After stirring for 15 minutes, 2-chloro-5-(2,6-dichlorophenyl)-6*H*-pyrimido[1,6-b] pyridazin-6-one (100 mg, 0.31 mmol) was added and the final reaction solution was stirred at room temperature for 16 hours. The reaction was quenched with saturated ammonium chloride solution (2 mL), and diluted with water (20 mL). The obtained mixture was extracted with ethyl acetate (50 mL x 3). The organic phases were combined, washed with water, dried over anhydrous sodium sulfate, filtered and concentrated under reduced pressure to obtain a residue, which was purified by silica gel column chromatography to obtain 40 mg of yellow solid with a yield of 33%. LC-MS: 392.1 [M+H] detection value; ¹H NMR (400 MHz, CDCl₃) δ 8.81 (s, 1H), 7.48-7.46 (m, 2H), 7.37-7.33 (m, 1H), 6.98 (d*, J* = 10.0 Hz, 1H), 6.63 (d, *J* = 10.0 Hz, 1H), 5.24-5.18 (m, 1H), 4.05- 4.00 (m, 2H), 3.69-3.63 (m, 2H), 2.19-2.15 (m, 2H), 1.93-1.85 (m, 2H).

### Embodiment 3 Synthesis of 5-(2,6-dichlorophenyl)-2-((tetrahydrofuran-3-yl) oxy)-6H-pyrimido[1,6-b]pyridazin-6-one (I-3)

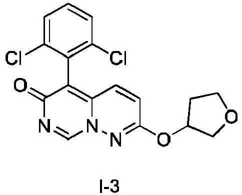

Preparation was performed according to the method of Embodiment 2, wherein tetrahydro-2*H*-pyran-4-ol was replaced with 3-hydroxytetrahydrofuran. LC-MS: 378.1 [M+H] detection value; ¹H NMR (400 MHz, CDCl₃) δ 8.81 (s, 1H), 7.48-7.46 (m, 2H), 7.37-7.32 (m, 1H), 6.99 (d, *J* = 9.6 Hz, 1H), 6.63 (d, *J* = 9.6 Hz, 1H), 5.54-5.51 (m, 1H), 4.09-4.01 (m, 3H), 3.98-3.92 (m, 1H), 2.42-2.33 (m, 1H), 2.28-2.22 (m, 1H).

### Embodiment 4 Synthesis of 5-(2,6-dichlorophenyl)-2-(2,4-difluorophenoxy)-6H-pyrimido[1,6-b]pyridazin-6-one (I-4)

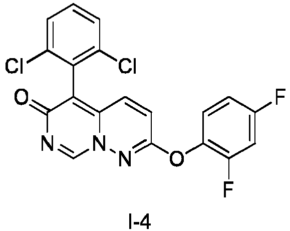

2-Chloro-5-(2,6-dichlorophenyl)-6*H*-pyrimido[1,6-b]pyridazin-6-one (100 mg, 0.31 mmol), 2,4-difluorophenol (48 mg, 0.37 mmol) and cesium carbonate (300 mg, 0.92 mmol) were added to N,N-dimethylformamide (5 mL). After stirring at 80°C for 3 hours, the reaction solution was filtered and concentrated under reduced pressure. The obtained residue was purified by silica gel column chromatography to obtain 55 mg of yellow solid with a yield of 43%. LC-MS: 420.0 [M+H] detection value; ¹H NMR (400 MHz, CDCl₃) δ 8.59 (s, 1H), 7.49 (s, 1H), 7.47 (s, 1H), 7.36 (dd, *J* = 8.8, 7.6 Hz, 1H), 7.31-7.25 (m, 2H), 7.11 (d*, J* = 10.0 Hz, 1H), 7.09-6.97 (m, 2H), 6.89 (d*, J* = 10.0 Hz, 1H).

### Embodiment 5 Synthesis of 2-(3-bromo-4-fluorophenoxy)-5-(2,6-dichlorophenyl)-6H-pyrimido[1,6-b]pyridazin-6-one (I-5)

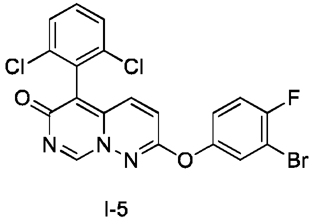

2-Chloro-5-(2,6-dichlorophenyl)-6*H*-pyrimido[1,6-b]pyridazin-6-one (60 mg, 0.18 mmol) was dissolved in N,N-dimethylformamide (5 mL), and cesium carbonate (120 mg, 0.37 mmol and 3-bromo-4-fluorophenol (52.7 mg, 0.28 mmol) were added. The reaction was stirred at room temperature for 16 hours. The reaction was quenched with water (20 mL) and extracted with ethyl acetate (20 mL X 3). The organic phases were combined, washed with water three times, dried over anhydrous sodium sulfate, filtered, and concentrated under reduced pressure. The obtained crude was purified by silica gel column chromatography to obtain 35 mg of yellow solid with a yield of 39.5%. LC-MS: 480.0 [M+H] detection value; ¹H NMR (400 MHz, CDCl₃) δ 8.63 (s, 1H), 7.52-7.47 (m, 3H), 7.38-7.34 (m, 1H), 7.28-7.19 (m, 2H), 7.10 (d, *J* = 10.0 Hz, 1H), 6.84 (d, *J* = 9.6 Hz, 1H).

### Embodiment 6 Synthesis of 2-(4-chloro-2-fluorophenoxy)-5-(2,6-dichlorophenyl)-6H-pyrimido[1,6-b]pyridazin-6-one (I-6)

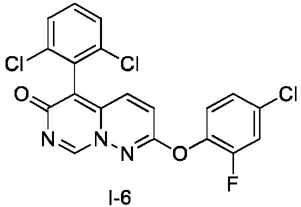

Preparation was performed according to the method of Embodiment 4, wherein 2,4-difluorophenol was replaced with 2-fluoro-4-chlorophenol. LC-MS: 436.0 [M+H] detection value; ¹H NMR (400 MHz, CDCl₃) δ 8.59 (s, 1H), 7.49 (d, *J* = 0.8 Hz, 1H), 7.47 (s, 1H), 7.38-7.31 (m, 2H), 7.26 (d, *J* = 1.2 Hz, 1H), 7.25 (d, *J* = 1.2 Hz, 1H), 7.11 (dd, *J* = 10.0, 0.4 Hz, 1H), 6.89 (d, *J* = 10.0 Hz, 1H).

### Embodiment 7 Synthesis of 5-(2,6-dichlorophenyl)-2-ethoxy-6H-pyrimido[1,6-b] pyridazin-6-one (I-7)

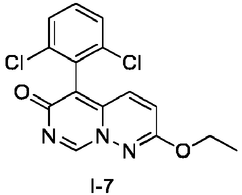

Preparation was performed according to the method of Embodiment 2, wherein tetrahydro-2*H*-pyran-4-ol was replaced with ethanol. LC-MS: 336.0 [M+H] detection value; ¹H NMR (400 MHz, CDCl₃) δ 8.82 (s, 1H), 7.47-7.45 (m, 2H), 7.36-7.31 (m, 1H), 6.95 (dd, *J* = 9.9, 0.6 Hz, 1H), 6.62 (d, *J* = 9.9 Hz, 1H), 4.43 (q, *J* = 7.2 Hz, 2H), 1.48 (t, J = 7.2 Hz, 3H).

### Embodiment 8 Synthesis of 5-(2,6-dichlorophenyl)-2-(2,2-difluoroethoxy)-6H-pyrimido[1,6-b]pyridazin-6-one (I-8)

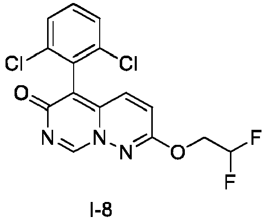

Preparation was performed according to the method of Embodiment 2, wherein tetrahydro-2*H*-pyran-4-ol was replaced with 2,2-difluoroethanol. LC-MS: 372.0 [M+H] detection value; ¹H NMR (400 MHz, CDCl₃) δ 8.82 (s, 1H), 7.48-7.46 (m, 2H), 7.37-7.33 (m, 1H), 7.03 (d, *J* = 9.6 Hz, 1H), 6.71 (d, *J* = 10.0 Hz, 1H), 6.32-6.03 (m, 1H), 4.63-4.55 (m, 2H).

### Embodiment 9 Synthesis of 2-(2-chloro-4-methoxyphenoxy)-5-(2,6-dichlorophenyl)-6H-pyrimido[1,6-b]pyridazin-6-one (I-9)

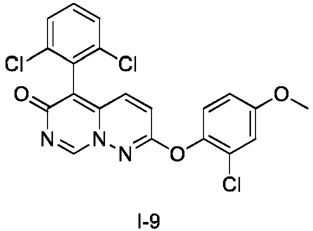

Preparation was performed according to the method of Embodiment 5, wherein 2,4-difluorophenol was replaced with 2-chloro-4-methoxyphenol. LC-MS: 448.0 [M+H] detection value; ¹H NMR (400 MHz, DMSO-d₆) δ 8.78 (s, 1H), 7.66-7.63 (m, 2H), 7.55-7.51 (m, 2H), 7.31-7.25 (m, 3H), 7.06 (dd, *J* = 8.8 Hz, 2.8 Hz, 1H), 3.83 (s, 3H).

### Embodiment 10 Synthesis of 2-(3-bromophenoxy)-5-(2,6-dichlorophenyl)-6H-pyrimido[1,6-blpyridazin-6-one (I-10)

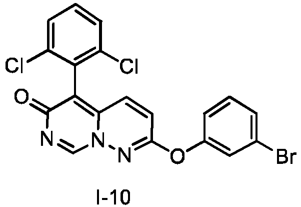

Preparation was performed according to the method of Embodiment 4, wherein 2,4-difluorophenol was replaced with 3-bromophenol. LC-MS: 464.0 [M+H] detection value; ¹H NMR (400 MHz, CDCl₃) δ 8.63 (s, 1H), 7.52-7.45 (m, 4H), 7.40-7.33 (m, 2H), 7.22 (dd, *J* = 8.0, 1.6 Hz, 1H), 7.10 (d, *J* = 10.0 Hz, 1H), 6.85 (d, *J* = 10.0 Hz, 1H).

### Embodiment 11 Synthesis of 5-(2,6-dichlorophenyl)-2-(3-fluoro-4-methylphenoxy)-6H-pyrimido[1,6-b]pyridazin-6-one (I-11)

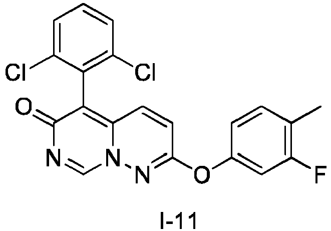

Preparation was performed according to the method of Embodiment 4, wherein 2,4-difluorophenol was replaced with 3-fluoro-4-methylphenol. LC-MS: 416.1 [M+H] detection value; ¹H NMR (400 MHz, CDCl₃) δ 8.63 (s, 1H), 7.49-7.47 (m, 2H), 7.38-7.36 (m, 1H), 7.31-7.27 (m, 1H), 7.08 (dd, *J* = 10.0 Hz, 0.8 Hz, 1H), 6.98-6.95 (m, 2H), 8.83 (d, *J* = 10.0 Hz, 1H), 2.35 (d, *J* = 2.0 Hz, 3H).

### Embodiment 12 Synthesis of 2-(2,3-dichlorophenoxy)-5-(2,6-dichlorophenyl)-6H-pyrimido[1,6-blpyridazin-6-one (I-12)

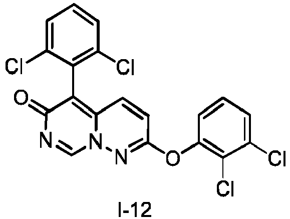

Preparation was performed according to the method of Embodiment 4, wherein 2,4-difluorophenol was replaced with 2,3-dichlorophenol. LC-MS: 454.0 [M+H] detection value; ¹H NMR (400 MHz, CDCl₃) δ 8.56 (s, 1H), 7.55-7.41 (m, 3H), 7.34 (dd, *J* = 8.7, 7.5 Hz, 2H), 7.24 (dd, J = 8.2, 1.5 Hz, 1H), 7.11 (d, J = 9.9 Hz, 1H), 6.90 (d, J = 9.9 Hz, 1H).

### Embodiment 13 Synthesis of 5-(2,6-dichlorophenyl)-2-(2,2,2-trifluoroethoxy)-6H-pyrimido[1,6-b]pyridazin-6-one (I-13)

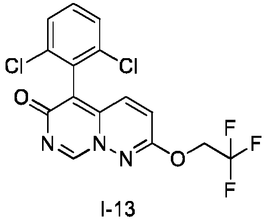

Preparation was performed according to the method of Embodiment 2, wherein tetrahydro-2*H*-pyran-4-ol was replaced with 2,2,2-trifluoroethanol. LC-MS: 390.0 [M+H] detection value; ¹H NMR (400 MHz, CDCl₃) δ 8.82 (s, 1H), 7.49-7.46 (m, 2H), 7.38-7.34 (m, 1H), 7.06 (dd, *J* = 9.6 Hz, 0.4 Hz, 1H), 6.74 (d, *J* = 10.0 Hz, 1H), 4.76 (q, *J* = 8.0 Hz, 2H).

### Embodiment 14 Synthesis of 5-(2,6-dichlorophenyl)-2-((3,3-difluorocyclobutyl) amino)-6H-pyrimido[1,6-b]pyridazin-6-one (I-14)

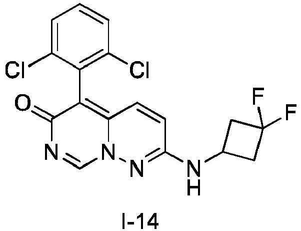

2-Chloro-5-(2,6-dichlorophenyl)-6*H-*pyrimido[1,6-b]pyridazin-6-one (100 mg, 0.31 mmol), 3,3-difluorocyclobutyl-1-amine hydrochloride (44 mg, 0.41 mmol) and N,N-diisopropylethylamine (220 mg, 1.71 mmol) were dissolved in N-methyl pyrrolidone (2.5 mL). The mixture was heated for 12 minutes at 110°C under microwave conditions, diluted with water, extracted with ethyl acetate (50 mL x 3). The organic phases were combined, washed with saturated saline, dried over anhydrous sodium sulfate, filtered and concentrated under reduced pressure. The obtained residue was purified by preparative TLC separation to obtain 10 mg of yellow solid with a yield of 8%. LC-MS: 397.1 [M+H] detection value; ¹H NMR (400 MHz, CD₃OD) δ 8.99 (s, 1H), 7.58 (d, *J=* 1.2 Hz, 1H), 7.56 (s, 1H), 7.48 (dd, *J* = 9.2 Hz, 7.2 Hz, 1H), 7.00 (d, *J* = 10.0 Hz, 1H), 6.83 (d, *J =* 10.0 Hz, 1H), 4.28-4.14 (m, 1H), 3.18- 3.03 (m, 2H), 2.68-2.56 (m, 2H).

### Embodiment 15 Synthesis of 2-(2-bromo-4-chlorophenoxy)-5-(2,6-dichlorophenyl)-6H-pyrimido[1,6-b]pyridazin-6-one (I-15)

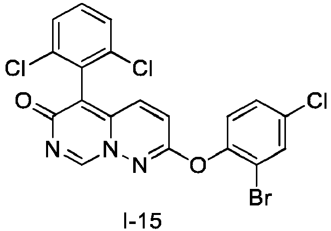

Preparation was performed according to the method of Embodiment 4, wherein 2,4-difluorophenol was replaced with 2-bromo-4-chlorophenol. LC-MS: 498.0 [M+H] detection value; ¹H NMR (400 MHz, CDCl₃) δ 8.56 (s, 1H), 7.71 (d, *J* = 2.4 Hz, 1H), 7.48-7.37 (m, 3H), 7.33 (dd, *J* = 8.8, 7.4 Hz, 1H), 7.24 (d, *J* = 8.6 Hz, 1H), 7.10 (d, *J* = 9.9 Hz, 1H), 6.89 (d, *J =* 9.9 Hz, 1H).

### Embodiment 16 Synthesis of 2-(5-bromo-2-chlorophenoxy)-5-(2,6-dichlorophenyl)-6H-pyrimido[1,6-b]pyridazin-6-one (I-16)

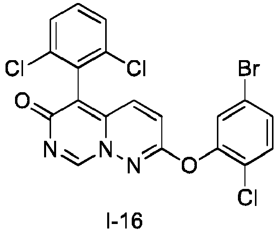

Preparation was performed according to the method of Embodiment 4, wherein 2,4-difluorophenol was replaced with 5-bromo-2-chlorophenol. LC-MS: 498.0 [M+H] detection value; ¹H NMR (400 MHz, CDCl₃) δ 8.58 (s, 1H), 7.50-7.38 (m, 5H), 7.34 (dd, *J=* 8.8, 7.4 Hz, 1H), 7.10 (d, *J=* 9.9 Hz, 1H), 6.88 (d, *J=* 9.9 Hz, 1H).

### Embodiment 17 Synthesis of 2-(2-bromophenoxy)-5-(2,6-dichlorophenyl)-6H-pyrimido[1,6-b]pyridazin-6-one (I-17)

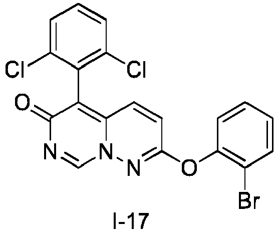

Preparation was performed according to the method of Embodiment 5, wherein 2,4-difluorophenol was replaced with 2-bromophenol. LC-MS: 462.0 [M+H] detection value; ¹H NMR (400 MHz, CDCl₃) δ 8.58 (s, 1H), 7.72 (dd, *J=* 8.0, 1.6 Hz, 1H), 7.49- 7.44 (m, 3H), 7.37-7.32 (m, 2H), 7.28-7.24 (m, 1H), 7.12 (d, *J* = 10.0 *Hz,* 1H), 6.93 (d, *J=* 10.0 Hz, 1H).

### Embodiment 18 Synthesis of 2-(2-chloro-4-fluorophenoxy)-5-(2,6-dichlorophenyl)-6H-pyrimido[1,6-b]pyridazin-6-one (I-18)

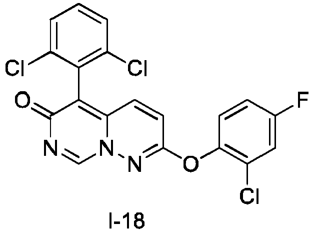

Preparation was performed according to the method of Embodiment 5, wherein 2,4-difluorophenol was replaced with 2-chloro-4-fluorophenol. LC-MS: 438.0 [M+H] detection value; ¹H NMR (400 MHz, CDCl₃) δ 8.55 (s, 1H), 7.46 (d, *J* = 8.3 Hz, 2H), 7.38-7.27 (m, 3H), 7.15-7.05 (m, 2H), 6.88 (d, *J=* 9.9 Hz, 1H).

### Embodiment 19 Synthesis of 5-(2,6-dichlorophenyl)-2-(4-fluorophenoxy)-6H-pyrimido[1,6-b]pyridazin-6-one (I-19)

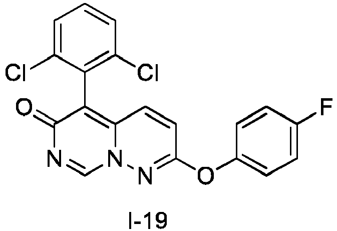

Preparation was performed according to the method of Embodiment 5, wherein 2,4-difluorophenol was replaced with 4-fluorophenol. LC-MS: 402.0 [M+H] detection value; ¹H NMR (400 MHz, CDCl₃) δ 8.58 (d, *J =* 0.6 Hz, 1H), 7.49-7.41 (m, 2H), 7.33 (dd, J = 8.7, 7.4 Hz, 1H), 7.25-7.10 (m, 4H), 7.06 (dd, J = 9.9, 0.7 Hz, 1H), 6.83 (d, J = 9.9 Hz, 1H).

### Embodiment 20 Synthesis of 2-(2,4-dichlorophenoxy)-5-(2,6-dichlorophenyl)-6H-pyrimido[1,6-b]pyridazin-6-one (I-20)

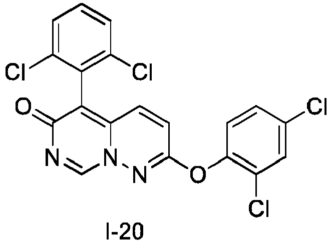

Preparation was performed according to the method of Embodiment 5, wherein 2,4-dichlorophenol was replaced with 2,4-difluorophenol. LC-MS: 454.0 [M+H] detection value; ¹H NMR (400 MHz, CDCl₃) δ 8.56 (s, 1H), 7.55 (d, *J =* 2.1 Hz, 1H), 7.45 (d, *J* = 8.0 Hz, 2H), 7.41-7.29 (m, 2H), 7.24 (d, *J =* 11.2 Hz, 1H), 7.09 (d, *J =* 9.8 Hz, 1H), 6.89 (d, *J =* 9.8 Hz, 1H).

### Embodiment 21 Synthesis of 5-(2,6-dichlorophenyl)-2-(2,5-difluorophenoxy)-6H-pyrimido[1,6-blpyridazin-6-one (I-21)

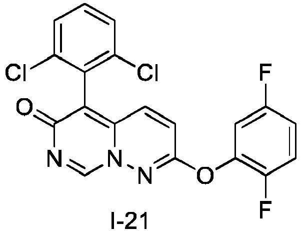

Preparation was performed according to the method of Embodiment 5, wherein 2,4-difluorophenol was replaced with 2,5-difluorophenol. LC-MS: 420.0 [M+H] detection value; ¹H NMR (400 MHz, CDCl₃) δ 8.58 (s, 1H), 7.49-7.39 (m, 2H), 7.33 (dd, *J* = 8.7, 7.4 Hz, 1H), 7.26-7.18 (m, 1H), 7.15-7.00 (m, 3H), 6.88 (d, J = 9.9 Hz, 1H).

### Embodiment 22 Synthesis of 2-(2,5-dichlorophenoxy)-5-(2,6-dichlorophenyl)-6H-pyrimido[1,6-b]pyridazin-6-one (I-22)

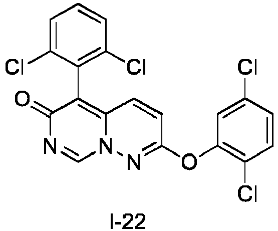

Preparation was performed according to the method of Embodiment 5, wherein 2,4-difluorophenol was replaced with 2,5-dichlorophenol. LC-MS: 454.0 [M+H] detection value; ¹H NMR (400 MHz, CDCl₃) δ 8.58 (s, 1H), 7.51-7.40 (m, 3H), 7.40-7.28 (m, 3H), 7.10 (d, J = 9.8 Hz, 1H), 6.89 (d, J = 9.8 Hz, 1H).

### Embodiment 23 Synthesis of 5-(2,6-dichlorophenyl)-2-(2,3-difluorophenoxy)-6H-pyrimido[1,6-b]pyridazin-6-one (I-23)

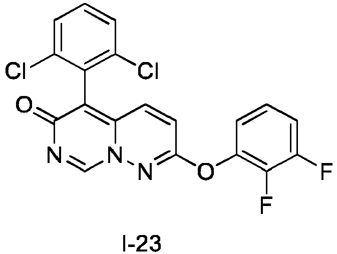

Preparation was performed according to the method of Embodiment 5, wherein 2,4-difluorophenol was replaced with 2,3-difluorophenol. LC-MS: 420.0 [M+H] detection value; ¹H NMR (400 MHz, CDCl₃) δ 8.59 (s, 1H), 7.46 (d, *J* = 8.0 Hz, 2H), 7.34 (dd, *J* = 8.7, 7.4 Hz, 1H), 7.21 (m, 2H), 7.10 (d, J = 9.7 Hz, 2H), 6.89 (d, J = 9.7 Hz, 1H).

### Embodiment 24 Synthesis of 5-(2,6-dichlorophenyl)-2-((5-fluoropyridin-3-yl) oxy)-6H-pyrimido[1,6-b]pyridazin-6-one (I-24)

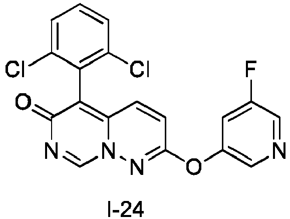

Preparation was performed according to the method of Embodiment 5, wherein 2,4-difluorophenol was replaced with 5-fluoropyridin-3-ol. LC-MS: 403.1 [M+H] detection value; ¹H NMR (400 MHz, CDCl₃) δ 8.65 (s, 1H), 8.55 (s, 1H), 8.51 (s, 1H), 7.49- 7.48 (m, 3H), 7.41-7.33 (m, 1H), 7.15 (d, *J* = 9.6 Hz, 1H), 6.89 (d, *J* = 9.6 Hz, 1H).

### Embodiment 25 Synthesis of 5-(2,6-dichlorophenyl)-2-(3-fluorophenoxy)-6H-pyrimido[1,6-b]pyridazin-6-one (I-25)

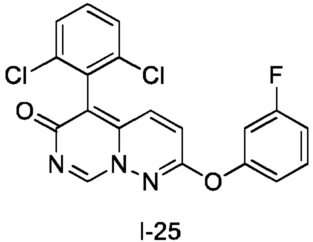

Preparation was performed according to the method of Embodiment 4, wherein 2,4-difluorophenol was replaced with 3-fluorophenol. LC-MS: 402.1 [M+H] detection value; ¹H NMR (400 MHz, CDCl₃) δ 8.64 (s, 1H), 7.48-7.46 (m, 3H), 7.36 (dd, *J =* 8.8, 7.6 Hz, 1H), 7.11-7.06 (m, 3H), 7.05-7.02 (m, 1H), 6.86 (d, *J=* 10.0 Hz, 1H).

### Embodiment 26 Synthesis of 4-((5-(2,6-dichlorophenyl)-6-oxo-6H-pyrimido [1,6-b]pyridazin-2-yl)oxy)benzonitrile (I-26)

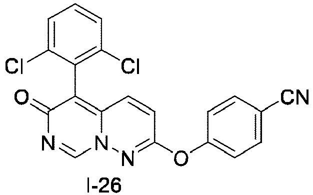

2-Chloro-5-(2,6-dichlorophenyl)-6*H*-pyrimido[1,6-b]pyridazin-6-one (50 mg, 0.15 mmol), 4-hydroxybenzonitrile (21.89 mg, 0.19 mmol) and potassium carbonate (150 mg, 0.46 mmol) were added to 5 mL of N,N-dimethylformamide. The reaction solution was reacted at room temperature overnight. The reaction solution was filtered and concentrated under reduced pressure. The obtained residue was purified by column chromatography (eluent: petroleum ether/ethyl acetate = 2/1) to obtain a yellow solid (12 mg, 0.026 mmol) with a yield of 18%. LC-MS: 409.0 [M+H] detection value; ¹H NMR (400 MHz, DMSO-d₆) δ 8.83 (d, J = 0.6 Hz, 1H), 8.04 - 8.00 (m, 2H), 7.68 (d, J = 2.1 Hz, 1H), 7.67 - 7.65 (m, 2H), 7.63 (s, 1H), 7.53 (m, 1H), 7.28 (m, 1H), 7.22 (d, *J =* 9.9 Hz, 1H).

### Embodiment 27 Synthesis of 5-(2,6-dichlorophenyl)-2-(2-fluorophenoxy)-6H-pyrimido[1,6-b]pyridazin-6-one (I-27)

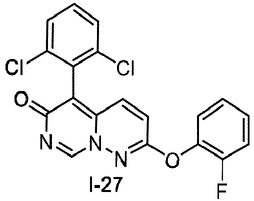

Preparation was performed according to the method of Embodiment 26, wherein 4-hydroxybenzonitrile was replaced with o-fluorophenol. LC-MS: 402.0 [M+H] detection value; ¹H NMR (400 MHz, CDCl₃) δ 8.59 (s, 1H), 7.51-7.46 (m, 2H), 7.35 (m, 2H), 7.31 (d, *J* = 2.0 Hz, 1H), 7.29-7.10 (m, 3H), 6.91 (d, *J =* 9.9 Hz, 1H).

### Embodiment 28 Synthesis of 5-(2,6-dichlorophenyl)-2-(4-fluoro-2-methylphenoxy)-6H-pyrimido[1,6-b]pyridazin-6-one (I-28)

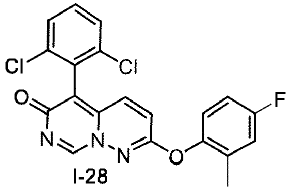

Preparation was performed according to the method of Embodiment 5, wherein 2,4-difluorophenol was replaced with 4-fluoro-2-methylphenol, and the solvent was replaced with acetonitrile. LC-MS: 415.9 [M+H] detection value; ¹H NMR (400 MHz, DMSO-d₆) δ 8.76 (d, *J* = 0.8 Hz, 1H), 7.66 (d, *J* = 0.8 Hz, 1H), 7.64 (s, 1H), 7.55-7.51 (m, 1H), 7.43-7.39 (m, 1H), 7.29-7.13 (m, 4H), 2.23 (s, 3H).

### Embodiment 29 Synthesis of 2-(2-chloro-3,6-difluorophenoxy)-5-(2, 6-dichlorophenyl)-6H-pyrimido[1,6-b]pyridazin-6-one (I-29)

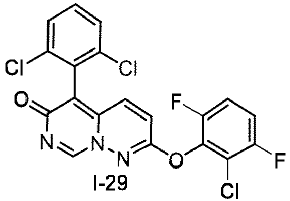

Preparation was performed according to the method of Embodiment 26, wherein 4-hydroxybenzonitrile was replaced with 2-chloro-3,6-difluorophenol. LC-MS: 456.0 [M+H] detection value; ¹H NMR (400 MHz, DMSO-d₆) δ 8.81 (s, 1H), 7.68-7.60 (m, 4H), 7.53 (m, 1H), 7.40 (d, *J* = 2.8 Hz, 2H).

### Embodiment 30 Synthesis of 5-(2,6-dichlorophenyl)-2-(3-fluorophenoxy)-6H-pyrimido[1,6-b]pyridazin-6-one (I-33)

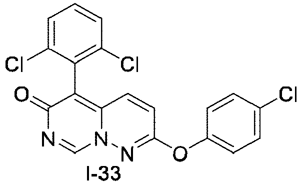

Preparation was performed according to the method of Embodiment 5, wherein 2,4-difluorophenol was replaced with 4-chlorophenol, and the solvent was replaced with acetonitrile. LC-MS: 417.9 [M+H] detection value; ¹H NMR (400 MHz, DMSO-d₆) δ 8.82 (s, 1H), 7.66-7.63 (m, 2H), 7.60-7.45 (m, 5H), 7.28-7.19 (m, 2H).

### Embodiment 31 Synthesis of 2-(4-chloro-2-methylphenoxy)-5-(2,6-dichlorophenyl)-6H-pyrimido[1,6-b]pyridazin-6-one (I-34)

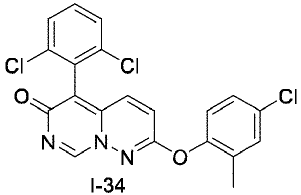

Preparation was performed according to the method of Embodiment 5, wherein 2,4-difluorophenol was replaced with 4-chloro-2-methylphenol, and the solvent was replaced with acetonitrile. LC-MS: 431.9 [M+H] detection value; ¹H NMR (400 MHz, DMSO-d₆) δ 8.78 (s, 1H), 7.66-7.63 (m, 2H), 7.55- 7.50 (m, 2H), 7.43-7.37 (m, 2H), 7.29-7.21 (m, 2H), 2.23 (s, 3H).

### Embodiment 32 Synthesis of 2-(cyclohex-2-en-1-yloxy)-5-(2,6-dichlorophenyl)-3 6H-pyrimido[1,6-b]pyridazin-6-one (I-37)

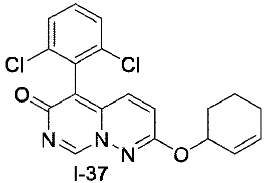

Preparation was performed according to the method of Embodiment 1, wherein 2-cyclohexen-1-ol was replaced with 3,3-difluorocyclobutanol. LC-MS:388.1 [M+H] detection value; ¹H NMR (400 MHz, CDCl₃) δ 8.81 (s, 1H), 7.46 (*J* = 8.0 Hz, 2H), 7.33 (dd, *J =* 8.8, 7.6 Hz, 1H), 6.95 (d, *J =* 10.0 Hz, 1H), 6.61 (d, *J =* 10.0 Hz, 1H), 6.16-6.08 (m, 1H), 6.00-5.91 (m, 1H), 5.49-5.48 (m, 1H), 2.25-2.09 (m, 2H), 2.05-1.94 (m, 2H), 1.91-1.80 (m, 1H), 1.79-1.71 (m, 1H).

### Embodiment 33 Synthesis of 2-(cyclopent-3-en-1-yloxy)-5-(2,6-dichlorophenyl)-6H-pyrimido[1,6-b]pyridazin-6-one (I-38)

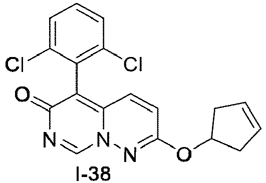

Preparation was performed according to the method of Embodiment 2, wherein tetrahydro-2*H*-pyran-4-ol was replaced with cyclopent-3-en-1-ol. LC-MS: 374.0 [M+H] detection value; ¹H NMR (400 MHz, *Chloroform-d)* δ 8.82 (s, 1H), 7.46 (s, 1H), 7.44 (s, 1H) 7.36 - 7.30 (m, 1H), 6.94 (d, *J* = 9.8 Hz, 1H), 6.59 (d, *J=* 9.9 Hz, 1H), 5.80 (s, 2H), 2.99 - 2.88 (m, 4H).

### Embodiment 34 Synthesis of 2-(4-bromophenoxy)-5-(2,6-dichlorophenyl)-6H-pyrimido[1,6-b]pyridazin-6-one (I-39)

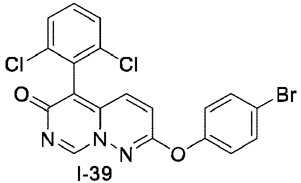

Preparation was performed according to the method of Embodiment 5, wherein 2,4-difluorophenol was replaced with 4-bromophenol. LC-MS: 462.0 [M+H] detection value; ¹H NMR (400 MHz, DMSO-d₆) δ 8.82 (d, *J =* 0.8 Hz, 1H), 7.72-7.68 (m, 2H), 7.65-7.63 (m, 2H), 7.55-7.51 (m, 1H), 7.43-7.39 (m, 2H), 7.27-7.18 (m, 2H).

### Embodiment 35 Synthesis of 2-(cyclopent-3-en-1-yloxy)-5-(2,6-dichlorophenyl)-6H-pyrimido[1,6-b]pyridazin-6-one (I-40)

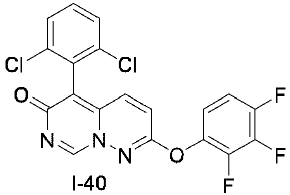

Preparation was performed according to the method of Embodiment 5, wherein 2,4-difluorophenol was replaced with 2,3,4-trifluorophenol. LC-MS: 462.0 [M+H] detection value; LC-MS: 438.0 [M+H] detection value; ¹H NMR (400 MHz, CDCl₃) δ 8.57 (s, 1H), 7.46 (d, *J* = 8.0 Hz, 2H), 7.34 (t, *J* = 8.1 Hz, 1H), 7.07-7.11 (m, 3H), 6.88 (d, *J* = 9.8 Hz, 1H).

### Embodiment 36 Synthesis of 5-(2,6-dichlorophenyl)-2-(1-(2,4-difluorophenyl) ethoxy)-6H-pyrimido [1,6-b] pyridazin-6-one (I-41)

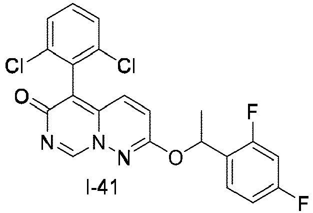

### Step A: Synthesis of 1-(2,4-difluorophenyl)ethanol

2,4-Difluoroacetophenone (5 g, 32.02 mmol) was dissolved in 60 mL of methanol, and sodium borohydride (1.34 g, 35.3 mmol) was added at 0°C. The reaction solution was returned to room temperature and stirred for one hour, then concentrated under reduced pressure to obtain a residue. The residue was dissolved in ethyl acetate, washed with water. The organic phases were dried over anhydrous sodium sulfate, and concentrated under reduced pressure to obtain a colorless oil (5.3 g, 34 mmol) with a yield of 100%. LC-MS: 141.1 [M-OH] detection value; ¹H NMR (400 MHz, CDCl₃) δ 7.51-7.45 (m, 1H), 6.96 - 6.85 (m, 1H), 6.85 - 6.71 (m, 1H), 5.18 (q, *J* = 6.4 Hz, 1H), 1.91 (s, 2H), 1.52 (d, *J* = 6.4 Hz, 3H).

### Step B: Synthesis of 5-(2,6-dichlorophenyl)-2-(1-(2,4-difluorophenyl)ethoxy)-6H-pyrimido [1,6-b]pyridazin-6-one

2-Chloro-5-(2,6-dichlorophenyl)-6*H*-pyrimido[1,6-b]pyridazin-6-one (200 mg, 0.61 mmol), 1-(2,4-difluorophenyl)ethanol (146 mg, 0.92 mmol) and cesium carbonate (400 mg, 1.21 mmol) were added to 5 mL of N-methylpyrrolidone. The reaction solution was heated to 90°C for 2 hours. The reaction solution was diluted with water, extracted with ethyl acetate (50 mL x 3). The organic phases were combined, dried over anhydrous sodium sulfate, concentrated under reduced pressure to obtain a residue, which was purified by column chromatography to obtain a crude product, then purified by preparative chromatography to obtain a colorless oil (10 mg, 0.02 mmol) with a yield of 3.6%. LC-MS: 448.1 [M+H] detection value; ¹H NMR (400 MHz, CDCl₃) δ 8.73 (s, 1H), 7.49-7.41 (m, 3H), 7.36-7.30 (m, 1H), 6.99-6.85 (m, 3H), 6.64 (d, *J* = 10.0 Hz, 1H), 6.28 (q, *J* = 6.4 Hz, 1H), 1.74 (d, *J* = 6.4 Hz, 3H).

### Embodiment 37 Synthesis of 5-(2,6-dichlorophenyl)-2-(1-(2,4-difluorophenyl) ethoxy)-6H-pyrimido [1,6-b] pyridazin-6-one (I-42)

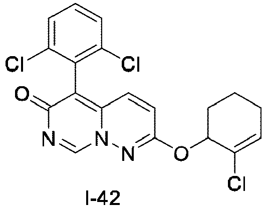

### Step A: Synthesis of 2-chloro-2-cyclohexen-1-one

2-Cyclohexen-1-one (1 g, 10.40 mmol) was dissolved in 1 M hydrochloric acid-DMF (16 mL), and m-chloroperoxybenzoic acid (2.7 g, 16 mmol) was added in one time at room temperature. The reaction solution was reacted at room temperature for 30 minutes. The reaction was quenched with 1 M aqueous sodium bicarbonate, extracted with ethyl acetate (50 mL x 3). The organic phases were combined, dried over anhydrous sodium sulfate and concentrated under reduced pressure to obtain a crude product, which was purified by column chromatography to obtain a white solid (690 mg, 5.28 mmol) with a yield of 51%. ¹H NMR (400 MHz, CDCl₃) δ 7.16 (t, *J* = 4.5 Hz, 1H), 2.65- 2.58 (m, 2H), 2.54-2.47 (m, 2H), 2.11-2.04 (m, 2H).

### Step B: Synthesis of 2-chloro-2-cyclohexen-1-ol

Cerium trichloride heptahydrate (569 mg, 1.52 mmol) and 2-chloro-2-cyclohexen-1-one (200 mg, 1.53 mmol) were dissolved in 10 mL of methanol, and the reaction solution was stirred for 15 minutes at 0°C. Sodium borohydride (61 mg, 1.60 mmol) was added and the reaction solution was returned to room temperature and continued to react for 3 hours. The reaction was quenched with 1 M hydrochloric acid. The reaction was concentrated under reduced pressure, and the obtained aqueous solution was extracted with ethyl acetate, washed with brine, dried over anhydrous sodium sulfate and then concentrated under reduced pressure to obtain a colorless oil (180 mg, 1.35 mmol) with a yield of 89%. ¹H NMR (400 MHz, CDCl₃) δ 6.06- 5.96 (t, *J =* 4.0 Hz, 1H), 4.19 (s, 1H), 2.26-2.08 (m, 2H), 2.00-1.84 (m, 2H), 1.81-1.69 (m, 1H), 1.69-1.56 (m, 1H).

### Step C: Synthesis of 2-((2-c+hlorocyclohex-2-en-1-yl)oxy)-5-(2,6-dichlorophenyl)-6H-pyrimido[1,6-b]pyridazin-6-one

Preparation was performed according to the method of step D in Embodiment 1, wherein 3,3-difluorocyclobutanol was replaced with 2-chloro-2-cyclohexen-1-ol. LC-MS: 422.0 [M+H] detection value; ¹H NMR (400 MHz, CDCl₃) δ 8.81 (s, 1H), 7.47-7.46 (m, 1H), 7.46- 7.44 (m, 1H), 7.34 (d, *J* = 8.0 Hz, 1H), 6.99 (d, *J* = 10.0 Hz, 1H), 6.67 (d, *J =* 10.0 Hz, 1H), 6.27 (dd, *J =* 5.2, 3.2 Hz, 1H), 5.57- 5.56 (m, 1H), 2.38-2.19 (m, 3H), 2.04-1.94 (m, 1H), 1.81 -1.72 (m, 2H).

### Embodiment 38 Synthesis of 2-(2-chloro-4-fluorophenoxy)-5-(2,6-dichlorophenyl)-6H-pyrimido[1,6-b]pyridazin-6-one (I-43)

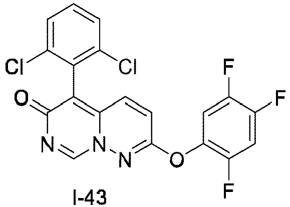

Preparation was performed according to the method of Embodiment 5, wherein 2,4-difluorophenol was replaced with 2,4,5-trifluorophenol. LC-MS: 438.0 [M+H] detection value; ¹H NMR (400 MHz, DMSO-d₆) δ 8.82 (s, 1H), 7.66-7.63 (m, 2H), 7.60-7.45 (m, 5H), 7.28-7.19 (m, 2H).

### Embodiment 39 Synthesis of 5-(cyclohex-1-en-1-yl)-2-(2,4-difluorophenoxy)-6H-pyrimido[1,6-b]pyridazin-6-one (I-44)

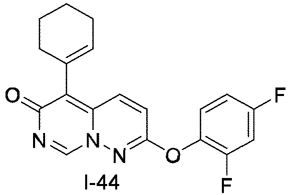

### Step A: Synthesis of 2-(6-chloropyridazin-3-yl)-2-(cyclohex-1-en-1-yl) acetonitrile

To a 100 mL round bottom flask, 3,6-dichloropyridazine (1.0 g, 8.25 mmol), 2-(cyclohex-1-en-1-yl)acetonitrile (1.23 g, 8.25 mmol) were added, and anhydrous tetrahydrofuran (20 mL) as solvent was added, and then potassium tert-butoxide (1.39 g,12.38 mmol) was added. The reaction solution was heated to reflux and stirred for 16 hours. The reaction solution was cooled to room temperature, and was added dropwise to ice-saturated ammonium chloride solution with stirring; extracted with ethyl acetate (30 mL x 3). The extracts was combined, washed with water (30 mL x 2), washed with saturated brine (30 mL x 1), dried over anhydrous sodium sulfate, filtered and concentrated. The obtained crude product was purified by column chromatography to obtain a brownish red solid (0.45 g, 1.93 mmol) with a yield of 23%. LCMS: 234 [M+H] detection value.

### Step B: Synthesis of 2-(6-chloropyridazin-3-yl)-2-(cyclohex-1-en-1-yl)acetamide

To a 100 mL round bottom flask, 2-(6-chloropyridazin-3-yl)-2-(cyclohex-1-en-1-yl) acetonitrile (0.45 g,1.93 mmol) was added, and concentrated sulfuric acid (2 mL) as solvent was added. The reaction solution was heated to 100°C and stirred for 2 hours, and then cooled to room temperature. The reaction solution was added dropwise to ice water, to which a saturated sodium bicarbonate solution was added dropwise to adjust the pH to 6. The solution was extracted with ethyl acetate (30 mL x 3). The extracts were combined, washed with water (30 mL x 1), and washed with saturated brine (30 mL x 1), dried over anhydrous sodium sulfate, filtered and concentrated to obtain a brown crude product (330 mg, 1.31 mmol) with a yield of 67%. The obtained crude product was used directly in the next step. LCMS: 252[M+H] detection value.

### Step C: Synthesis of 2-chloro-5-(cyclohex-1-en-1-yl)-6H-pyrimido[1,6-b] pyridazin-6-one

To a 100 mL round bottom flask, 2-(6-chloropyridazin-3-yl)-2-(cyclohex-1-en-1-yl) acetamide (330 mg, 1.31 mmol), trimethyl orthoformate (0.71 mg, 6.67 mmol), p-toluenesulfonic acid monohydrate (10 mg) were added, and toluene (10 mL) as solvent was added finally. The reaction solution was heated to reflux and stirred for two hours, and then cooled to room temperature. The reaction solution was concentrated to obtain a crude product, which was purified by column chromatography to obtain a brown solid (90 mg, 0.34 mmol) with a yield of 26%. LCMS: 262 [M+H] detection value.

### Step D: Synthesis of 5-(cyclohex-1-en-1-yl)-2-(2,4-difluorophenoxy)-6H-pyrimido[1,6-b]pyridazin-6-one

To a 100 mL round bottom flask, 2-chloro-5-(cyclohex-1-en-1-yl)-6*H*-pyrimido [1,6-b]pyridazin-6-one (90 mg, 0.34 mmol), 2,4-difluorophenol (66 mg, 0.51 mmol), N,N-diisopropylethylamine (70 mg, 0.68 mmol) were added, and acetonitrile (15 mL) as solvent was added finally. The reaction solution was heated to 50°C and stirred overnight. The reaction solution was concentrated under reduced pressure, and the obtained solid was dissolved with ethyl acetate (50 mL), washed with water (30 mL x 2), saturated brine (15 mL x 1) sequentially, then dried over anhydrous sodium sulfate and filtered and concentrated to obtain a brown crude product. The obtained crude product was purified by column chromatography to obtain a brown solid (70 mg, 0.19 mmol) with a yield of 55%. LCMS: 356 [M+H] detection value. LCMS: 356 [M+H] detection value. ¹H NMR (400 MHz, CDCl₃) δ 8.46 (s 1H), 7.84 (d, *J=* 10.0 Hz, 1H), 7.26-7.22 (m, 1H), 7.06-6.96 (m, 2H), 6.82 (d, *J* = 10.0 Hz, 1H), 5.74 (br, 1H), 2.30-2.25 (br, 4H), 1.82-1.74 (m, 4H).

### Embodiment 40 Synthesis of 2-(2,4-difluorophenoxy)-5-(2,6-dimethylphenyl)-6H-pyrimido[1,6-b]pyridazin-6-one (I-45)

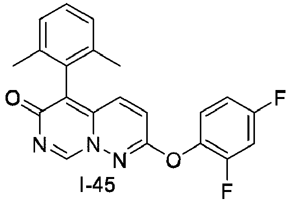

### Step A: Synthesis of 2-(6-chloropyridazin-3-yl)-2-(2,6-dimethylphenyl) acetonitrile

2,6-Dimethylphenylacetonitrile (500 mg, 3.44 mmol) and 3,6-dichloropyridazine (513 mg, 3.44 mmol) were dissolved in 12 mL of tetrahydrofuran, and potassium tert-butoxide (579 mg, 5.17 mmol) was added at room temperature. The reaction solution was heated to 75°C for 16 hours. The reaction was quenched with IN hydrochloric acid, extracted with ethyl acetate (50 mL x 3). The organic phases were combined, dried over anhydrous sodium sulfate, and concentrated under reduced pressure to obtain a residue, which was purified by column chromatography to obtain a brown oil (700 mg, 2.71 mmol) with a yield of 79%. LC-MS: 258.1 [M+H] detection value.

### Step B: Synthesis of 2-(6-chloropyridazin-3-yl)-2-(2,6-dimethylphenyl) acetamide

2-(6-chloropyridazin-3-yl)-2-(2,6-dimethylphenyl)acetonitrile (670 mg, 2.60 mmol) was dissolved in 6 mL of dimethyl sulfoxide, and 3 mL of 30% aqueous hydrogen peroxide and potassium carbonate (72 mg, 0.52 mmol) were added at 0°C. The reaction was stirred for 5 minutes and then returned to room temperature for 2 hours. The reaction solution was then diluted with water, extracted with ethyl acetate (60 mL x 3). The organic phases were combined, washed with brine, dried over anhydrous sodium sulfate and concentrated under reduced pressure to obtain a brown oil (640 mg, 2.32 mmol) with a yield of 89%. LC-MS: 276.1 [M+H] detection value.

### Step C: Synthesis of 2-chloro-5-(2,6-dimethylphenyl)-6H-pyrimido[1,6-b] pyridazin-6-one

2-(6-Chloro-pyridazin-3-yl)-2-(2,6-dimethylphenyl)acetamide (610 mg, 2.21 mmol), trimethyl orthoformate (1.4 g, 13 mmol) and p-toluenesulfonic acid monohydrate (21 mg, 0.11 mmol) were added to 10 mL of toluene. The reaction solution was heated to 60°C for 2 hours. The reaction solution was concentrated under reduced pressure to obtain a crude product, which was purified by column chromatography to obtain a yellow solid (140 mg, 0.49 mmol) with a yield of 22%. LC-MS: 286.1 [M+H] detection value.

### Step D: Synthesis of 2-(2,4-difluorophenoxy)-5-(2,6-dimethylphenyl)-6H-pyrimido [1,6-b] pyridazin-6-one

2-Chloro-5-(2,6-dimethylphenyl)-6*H*-pyrimido[1,6-b]pyridazin-6-one (138 mg, 0.48 mmol), 2,4-difluorophenol (75 mg, 0.57 mmol) and cesium carbonate (313 mg, 0.96 mmol) were added to 4 mL of N,N-dimethylformamide. The reaction solution was reacted at room temperature for 16 hours. The reaction solution was diluted with water and extracted with ethyl acetate (50 mL x 3). The organic phases were combined, washed with brine, dried over anhydrous sodium sulfate, concentrated under reduced pressure. The crude product was purified by column chromatography to obtain a yellow solid (66 mg, 0.17 mmol) with a yield of 36%. LC-MS: 380.2 [M+H] detection value; ¹H NMR (400 MHz, CDCl₃) δ 8.57 (s, 1H), 7.30 -7.22 (m, 2H), 7.16 (d, *J* = 7.6 Hz, 2H), 7.09-6.96 (m, 3H), 6.77 (d, *J* = 10.0 Hz, 1H), 2.11 (s, 6H).

### Embodiment 41 Synthesis of 5-(2,5-dichlorophenyl)-2-(2,4-difluorophenoxy)-6H-pyrimido[1,6-b]pyridazin-6-one (I-46)

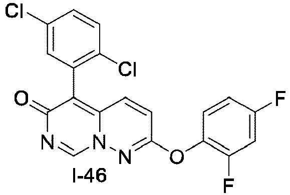

### Step A: Synthesis of 2-(6-chloropyridazin-3-yl)-2-(2,5-dichlorophenyl) acetonitrile

2-(2,5-Dichlorophenyl)acetonitrile (1 g, 5.4 mmol) and 3,6-dichloropyridazine (0.72 g, 4.8 mmol) were dissolved in 20 mL of tetrahydrofuran, and potassium tert-butoxide (0.90 g, 40 mmol) was added at 0°C. The reaction solution was returned to room temperature and heated to reflux overnight. The reaction solution was neutralized with 1 N hydrochloric acid, extracted with ethyl acetate (80 mL x 3). The organic phases were combined, dried over anhydrous sodium sulfate and concentrated under reduced pressure to obtain a residue, which was purified by column chromatography (eluent: petroleum ether/ethyl acetate = 4/1) to obtain a red solid (0.80 g, 2.7 mmol) with a yield of 50%. LC-MS: 298.0 [M+H] detection value.

### Step B: Synthesis of 2-(6-chloropyridazin-3-yl)-2-(2,5-dichlorophenyl)acetamide

2-(6-Chloro-pyridazin-3-yl)-2-(2,5-dichlorophenyl)acetonitrile (0.80 g, 2.7 mmol) was added to 10 mL of concentrated sulfuric acid, and the reaction solution was reacted at 100°C for 2 hours. The reaction solution was poured into water and extracted with ethyl acetate (50 mL x 3). The organic phases were combined, dried over anhydrous sodium sulfate and concentrated by reduced pressure to obtain a yellow solid (0.5 g, 2mmol) with a yield of 60%. LC-MS: 318.0 [M+H] detection value.

### Step C: Synthesis of 2-chloro-5-(2,5-dichlorophenyl)-6H-pyrimido[1,6-b] pyridazin-6-one

2-(6-Chloro-pyridazin-3-yl)-2-(2,5-dichlorophenyl)acetamide (0.5 g, 2 mmol) and trimethyl orthoformate (0.5 g, 4.73 mmol), p-methylbenzenesulfonic acid (0.07 g, 0.4 mmol) were added to 10 mL of toluene. The reaction solution was reacted at 100°C for 3 hours. The reaction solution was concentrated under reduced pressure to obtain a residue, which was purified by column chromatography (eluent: petroleum ether/ethyl acetate=1/2) to obtain a yellow solid (0.35 mg, 1.1 mmol) with a yield of 70%. LC-MS: 328.0 [M+H] detection value.

### Step D: Synthesis of 5-(2,5-dichlorophenyl)-2-(2,4-difluorophenoxy)-6H-pyrimido [1,6-b] pyridazin-6-one

2-Chloro-5-(2,5-dichlorophenyl)-6*H*-pyrimido[1,6-b]pyridazin-6-one (0.2 g, 0.6 mmol), 2,4-difluorophenol (80 mg, 0.6 mmol) and potassium carbonate (0.25 g, 1.8 mmol) were added to 8 mL of N,N-dimethylformamide. The reaction solution was stirred at room temperature overnight. The end of reaction was monitored by TLC plate. The reaction solution was quenched with 20 mL of water, extracted with ethyl acetate (20 mL x 3). The obtained extracts were combined, washed with water three times and partitioned. The obtained ethyl acetate solution was dried over anhydrous sodium sulfate, filtered, and concentrated under reduced pressure to obtain a crude product. The crude product was purified by column chromatography to obtain a yellow solid, and the obtained residue was purified by column chromatography (eluent: petroleum ether/ethyl acetate = 2/1) to obtain a yellow solid (0.15 g, 0.36 mmol) with a yield of 60%. LC-MS: 420.0 [M+1] detection value; ¹H NMR (400 MHz, Chloroform- *d*) δ 8.57 (s, 1H), 7.48 (d, *J* = 9.3 Hz, 1H), 7.40-7.36 (m, 2H), 7.27-7.20 (m, 2H), 7.08-6.96 (m, 2H), 6.91 (d, *J* = 9.9 Hz, 1H).

### Embodiment 42 Synthesis of 5-(2,6-dichlorophenyl)-2-((2-methylcyclopent-1-en-1-yl)oxy)-6H-pyrimido[1,6-b]pyridazin-6-one(I-47)

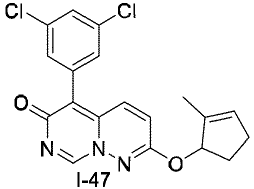

2-Methylcyclopent-2-en-1-ol (30 mg, 0.30 mmol) was dissolved in N,N-dimethylformamide (2 mL), and sodium hydrogen (12 mg, 0.30 mmol) was added, the reaction solution was stirred at room temperature for 30 minutes. 2-Chloro-5-(2,6-dichlorophenyl)-6*H*-pyrimido[1,6-b]pyridazin-6-one (100 mg, 0.30 mmol) was added, and the reaction solution was stirred at room temperature for 16 hours. The reaction solution was evaporated to dryness under reduced pressure, added with water (10 mL), extracted with ethyl acetate (10 mLx3). The organic phases were combined, washed with saturated brine. The organic phases were evaporated to dryness. The residue was separated by silica gel column chromatography (MeOH/DCM=0-5%) to obtain 5-(2,6-dichlorophenyl)-2-((2-methylcyclopent-1-en-1-yl)oxy)-6*H*-pyrimido[1,6-b]pyridazin-6 -one (10 mg, 0.026 mmol) as a white solid with a yield of 8.4%. LC-MS: 388 [M+H] detection value; ¹H NMR (400 MHz, *Chloroform-d)* δ 8.80 (s, 1H), 7.44 (d, *J=* 8.1 Hz, 2H), 7.31 (dd, *J =* 8.7, 7.5 Hz, 1H), 6.94 (d, *J* = 9.9 Hz, 1H), 6.60 (d, *J* = 9.8 Hz, 1H), 5.83-5.77 (m, 1H), 5.77-5.69 (m, 1H), 2.61-2.43 (m, 2H), 2.43-2.28 (m, 1H), 2.04-1.92 (m, 1H), 1.82 (s, 3H).

### Embodiment 43 Synthesis of 5-(3,5-dichlorophenyl)-2-(2,4-difluorophenoxy)-6H-pyrimido[1,6-b]pyridazin-6-one (I-48)

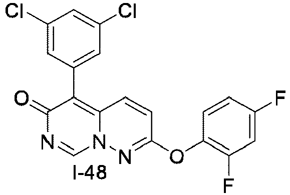

### Step A: Synthesis of 2-(6-chloropyridazin-3-yl)-2-(3,5-dichlorophenyl) acetonitrile

2-(3,5-Dichlorophenyl)acetonitrile (1 g, 5.4 mmol) and 3,6-dichloropyridazine (0.72 g, 4.8 mmol) were dissolved in 20 mL of tetrahydrofuran, and potassium tert-butoxide (0.90 g, 40 mmol) was added at 0°C. The reaction solution was returned to room temperature and heated to reflux overnight. The reaction solution was neutralized with 1 N hydrochloric acid, extracted with ethyl acetate (80 mL x 3). The organic phases were combined, dried over anhydrous sodium sulfate and concentrated under reduced pressure to obtain a residue, which was purified by column chromatography (eluent: petroleum ether/ethyl acetate = 4/1) to obtain a red solid (0.82 g, 2.7 mmol) with a yield of 51%. LC-MS: 298.0 [M+H] detection value.

### Step B: Synthesis of 2-(6-chloropyridazin-3-yl)-2-(3,5-dichlorophenyl)acetamide

2-(6-Chloro-pyridazin-3-yl)-2-(3,5-dichlorophenyl)acetonitrile (0.82 g, 2.7 mmol) was added to 10 mL of concentrated sulfuric acid, and the reaction solution was reacted at 100°C for 2 hours. The reaction solution was poured into water and extracted with ethyl acetate (50 mL x 3). The organic phases were combined, dried over anhydrous sodium sulfate and concentrated under reduced pressure to obtain a yellow solid (0.82g, 2.6 mmol) with a yield of 94%. LC-MS: 318.0 [M+H] detection value.

### Step C: Synthesis of 2-chloro-5-(3,5-dichlorophenyl)-6H-pyrimido[1,6-b] pyridazin-6-one

2-(6-Chloro-pyridazin-3-yl)-2-(3,5-dichlorophenyl)acetamide (0.82 g, 2.6 mmol) and trimethyl orthoformate (0.86 g, 12.2 mmol), p-toluenesulfonic acid (0.02 g, 0.1 mmol) were added to 10 mL of toluene. The reaction solution was reacted at 100°C for 3 hours. The residue was concentrated under reduced pressure to obtain a residue, which was purified by column chromatography (eluent: petroleum ether/ethyl acetate=1/2) to obtain a yellow solid (0.48 mg, 1.5 mmol) with a yield of 57%. LC-MS: 328.0 [M+H] detection value.

### Step D: Synthesis of 5-(3,5-dichlorophenyl)-2-(2,4-difluorophenoxy)-6H-pyrimido [1,6-b] pyridazin-6-one

2-Chloro-5-(3,5-dichlorophenyl)-6*H*-pyrimido[1,6-b]pyridazin-6-one (0.2 g, 0.6 mmol), 2,4-difluorophenol (80 mg, 0.6 mmol) and potassium carbonate (0.25 g, 1.8 mmol) were added to 8 mL of N,N-dimethylformamide. The reaction solution was stirred at room temperature overnight. The end of the reaction was monitored by TLC. The reaction solution was quenched with 20 mL of water, extracted with ethyl acetate (20 mL x 3). The obtained extracts were combined, washed with water three times and partitioned. The obtained ethyl acetate solution was dried over anhydrous sodium sulfate, filtered and concentrated under reduced pressure to obtain a crude product. The crude product was purified by column chromatography to obtain a yellow solid, and the obtained residue was purified by column chromatography (eluent: petroleum ether/ethyl acetate = 2/1) to obtain a yellow solid (86 mg, 0.2 mmol) with a yield of 30%. LC-MS: 420.0 [M+H] detection value; ¹H NMR (400 MHz, *Chloroform-d)* δ 8.53 (d, J = 0.7 Hz, 1H), 7.52 (dd, J = 9.9, 0.7 Hz, 1H), 7.45 (t, J = 1.9 Hz, 1H), 7.32 (d, J = 1.9 Hz, 2H), 7.26 (m, 1H), 7.09-6.97 (m, 2H), 6.90 (d, *J* = 10.0 Hz, 1H).

### Embodiment 44 Synthesis of 2-(2,4-difluorophenoxy)-5-(2,6-difluorophenyl)-6H-pyrimido[1,6-b]pyridazin-6-one (I-49)

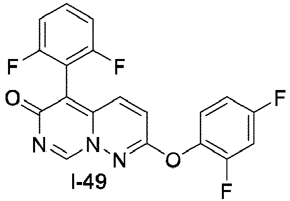

### Step A: Synthesis of 2-(6-chloropyridazin-3-yl)-2-(2,6-difluorophenyl) acetonitrile

To a 100 mL reaction round bottom flask, 1.026g of 2,6-difluorophenylacetonitrile, 1.0 g of 3,6-dichloropyridazine, 20 mL of tetrahydrofuran were added sequentially, and 0.90 g of anhydrous potassium tert-butoxide was added to the reaction finally. The reaction solution was heated to reflux overnight, then cooled to 0 °C. The reaction solution was quenched with saturated ammonium chloride solution, added with 1 N dilute hydrochloric acid to adjusted pH to 5, and extracted with ethyl acetate three times (30 mL) finally. The obtained extracts were combined, washed with brine three times, dried over anhydrous sodium sulfate, filtered and concentrated under reduced pressure to obtain a crude product. The obtained crude product was purified by column chromatography to obtain a brown solid (1.0g, with a yield of 56.1%). LC-MS: 266.0 [M+H] detection value.

### Step B: Synthesis of 2-(6-chloropyridazin-3-yl)-2-(2,6-difluorophenyl) acetamide.

To a 100 mL round bottom flask, 1.0 g of 2-(6-chloropyridazin-3-yl)-2-(2,6-difluorophenyl)acetonitrile was added, and then 3 mL of concentrated sulfuric acid was added. The reaction solution was heated to 100°C and stirred for two hours. TLC (PE:EA = 2:1) monitored that the chromogenic material had reacted completely. The reaction solution was cooled to 0 °C, and ice water was added. Saturated sodium bicarbonate solution was added to pH of the solution to 7. The solution was extracted with ethyl acetate three times (30 mL). The obtained extracts were combined, washed with water and brine sequentially, then dried over anhydrous sodium sulfate, filtered and concentrated under reduced pressure to obtain a crude product (0.98g, with a yield of 98%). The obtained crude product was used directly in the next step. LC-MS: 284.0 [M+H] detection value.

### Step C: Synthesis of 2-chloro-5-(2,6-difluorophenyl)-6H-pyrimido [1,6-b] pyridazin-6-one.

To a 100 mL round bottom flask, 0.98 g of 2-(6-chloropyridazin-3-yl)-2-(2,6-difluorophenyl)acetamide, 2.2 g of trimethyl orthoformate, 50 mL of toluene as solvent were added sequentially, and 50 mg of p-toluenesulfonic acid as catalyst was added finally. The reaction solution was heated to reflux for 2 hours and then cooled to room temperature. The reaction solution was concentrated under pressure, and the obtained product was dissolved with ethyl acetate (30 mL), washed with water twice and with saturated brine once, dried over anhydrous sodium sulfate, filtered and concentrated under reduced pressure. The obtained crude product was purified by column chromatography to obtain a brown solid (0.4g, with a yield of 39.4%). LC-MS: 294.0 [M+H] detection value.

### Step D: Synthesis of 2-(2,4-difluorophenoxy)-5-(2,6-difluorophenyl)-6H-pyrimido[1,6-b]pyridazin-6-one

50 mg of 2-chloro-5-(2,6-difluorophenyl)-6*H*-pyrimido[1,6-b]pyridazin-6-one was dissolved in 5 mL of N,N-dimethylformamide, and anhydrous cesium carbonate (83 mg, 0.26 mmol), 2,4-difluorophenol (27 mg, 0.20 mmol) was added. The reaction solution was stirred at room temperature for 16 hours. The end of the reaction was monitored by TLC (DCM: MeOH = 20: 1). The reaction solution was quenched with water, extracted with ethyl acetate (30 mL X 3). The obtained extracts were combined, washed with water three times, washed with brine twice, dried over anhydrous sodium sulfate, filtered and concentrated under reduced pressure to obtain a crude product. The obtained crude product was purified by column chromatography to obtain a yellow solid (30 mg, with a yield of 45.5%). LC-MS: 388.1 [M+H] detection value. ¹H NMR (400 MHz, DMSO-d₆) δ 8.75 (d, *J=* 0.4 Hz, 1H), 7.67-7.55 (m, 3H), 7.49 (d, J = 9.6 Hz, 1H), 7.31-7.22 (m, 4H).

### Embodiment 45 Synthesis of 2-(2,4-difluorophenoxy)-5-(o-methylphenyl)-6H-pyrimido[1,6-b]pyridazin-6-one (I-50)

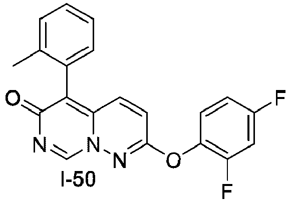

### Step A: Synthesis of 2-(6-chloropyridazin-3-yl)-2-(o-methylphenyl)acetonitrile

2-Methylphenylacetonitrile (1.93 g, 14.7 mmol) and 3,6-dichloropyridazine (2 g, 13.42 mmol) were dissolved in 20 mL of tetrahydrofuran, and potassium tert-butoxide (2.25 g, 20.1 mmol) was added at room temperature. The reaction solution was heated to 75°C for 16 hours. The reaction solution was quenched with IN hydrochloric acid, extracted with ethyl acetate (80 mL x 3). The organic phases were combined, dried over anhydrous sodium sulfate, concentrated under reduced pressure to obtain a residue, which was purified by column chromatography to obtain brown oil (2.05 g, 8.41 mmol) with a yield of 57%. LC-MS: 244.1 [M+H] detection value.

### Step B: Synthesis of 2-(6-chloropyridazin-3-yl)-2-(o-methylphenyl)acetamide

2-(6-Chloro-pyridazin-3-yl)-2-(o-methylphenyl)acetonitrile (1.77 g, 7.26 mmol) was dissolved in 20 mL of dimethyl sulfoxide, and 10 mL of 30% aqueous hydrogen peroxide and potassium carbonate (201 mg, 1.45 mmol) were added at 0°C. The reaction was reacted for 5 minutes, after that the reaction was continued to react with stirring for 4 hours at room temperature. The reaction solution was diluted with water, extracted with ethyl acetate (100 mL x 3). The organic phases were combined, washed with brine, dried over anhydrous sodium sulfate, concentrated under reduced pressure to obtain a crude product, which was slurried with ethyl acetate for purification to obtain a white solid (1.18 g, 4.51 mmol) with a yield of 62%. LC-MS: 262.1 [M+H] detection value.

### Step C: Synthesis of 2-chloro-5-(o-methylphenyl)-6H-pyrimido[1,6-b] pyridazin-6-one

2-(6-Chloro-pyridazin-3-yl)-2-(o-methylphenyl)acetamide (1.18 g, 4.51 mmol), trimethyl orthoformate (2.63 g, 24.8 mmol) and p-toluenesulfonic acid monohydrate (40 mg, 0.21 mmol) were added to 20 mL of toluene. The reaction solution was heated to 60°C for 16 hours. The reaction solution was concentrated under reduced pressure, dissolved in dichloromethane, washed with aqueous sodium bicarbonate, dried over anhydrous sodium sulfate, and concentrated under reduced pressure to obtain a crude product, which was slurried with ethyl acetate for purification to obtain a yellow solid (900 mg, 3.31 mmol) with a yield of 74%. LC-MS: 272.1 [M+H] detection value.

### Step D: Synthesis of 2-(2,4-difluorophenoxy)-5-(o-methylphenyl)-6H-pyrimido [1,6-b]pyridazin-6-one

2-Chloro-5-(o-methylphenyl)-6*H*-pyrimido[1,6-b]pyridazin-6-one (100 mg, 0.37 mmol), 2,4-difluorophenol (57 mg, 0.44 mmol) and cesium carbonate (239 mg, 0.73 mmol) were added to 3 mL of N,N-dimethylformamide. The reaction solution was reacted at room temperature for 16 hours. The reaction solution was diluted with water, extracted with ethyl acetate (50 mL x 3). The organic phases were combined, dried over anhydrous sodium sulfate, concentrated under reduced pressure to obtain a crude product, which was purified by column chromatography to obtain a yellow solid (60 mg, 0.16 mmol) with a yield of 45%. LC-MS: 366.1 [M+H] detection value; ¹H NMR (400 MHz, CDCl₃) δ 8.55 (s, 1H), 7.36-7.34 (m, 2H), 7.31-7.27 (m, 2H), 7.26-7.23 (m, 1H), 7.15 (d, *J=* 7.2 Hz, 1H), 7.07-6.97 (m, 2H), 6.77 (d, *J* = 10.0 Hz, 1H), 2.21 (s, 3H).

### Embodiment 46 Synthesis of 4-chloro-3-(2-(2,4-difluorophenoxy)-6-oxo-6H-pyrimido[1,6-b]pyridazin-5-yl)-N-methylbenzamide (I-51)

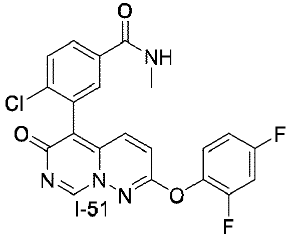

### Step A: Synthesis of methyl 4-chloro-3-((6-chloropyridazin-3-yl)(cyano)methyl) benzoate

Methyl 4-chloro-3-(cyanomethyl)benzoate (2 g, 9.54 mmol) was added to 40 mL of tetrahydrofuran, and sodium hydrogen (761 mg, 19.02 mmol) was added at 0°C. After stirring for 30 minutes, 3,6-dichloropyridazine (1.4 g, 9.4 mmol) was added, and the reaction solution was returned to room temperature and continued to react for 2 hours. The reaction was quenched with water, extracted with ethyl acetate (60 mL x 3). The organic phases were combined, dried over anhydrous sodium sulfate, and then concentrated under reduced pressure. The crude product was slurried with ethyl acetate for purification to obtain a gray solid (1.8 g, 5.6 mmol) with a yield of 59%. LC-MS: 322.1 [M+H] detection value.

### Step B: Synthesis of 3-(2-amino-1-(6-chloropyridazin-3-yl)-2-oxoethyl)-4-chlorobenzoic acid

Methyl 4-chloro-3-((6-chloropyridazin-3-yl)(cyano)methyl)benzoate (1 g, 3.10 mmol) was added to 4 mL of concentrated sulfuric acid, and the reaction solution was heated to 100°C for 2 hours. The reaction solution was then cooled to room temperature and poured into ice water, with a solid precipitated, filtered and dried to obtain a gray solid (700 mg, 2.14 mmol) with a yield of 69%. LC-MS: 326.1 [M+H] detection value.

### Step C: Synthesis of 3-(2-amino-1-(6-chloropyridazin-3-yl)-2-oxoethyl)-4-chloro-N-methylbenzamide

3-(2-Amino-1-(6-chloropyridazin-3-yl)-2-oxoethyl)-4-chlorobenzoic acid (424 mg, 1.300 mmol), HATU (741 mg, 1.95 mmol) and N,N-diisopropylethylamine (840 mg, 6.5116 mmol) were added to 6 mL of N,N-dimethylformamide. The reaction solution was stirred at room temperature for 10 minutes, and then methylamine (1.95 mL, 3.90 mmol) was added. The reaction solution was continued to be stirred for 16 hours. The reaction solution was concentrated under reduced pressure to obtain a residue, diluted with water, extracted with ethyl acetate (50 mL x 3). The organic phases were combined, dried over anhydrous sodium sulfate, concentrated under reduced pressure. The crude product was slurried with ethyl acetate for purification to obtain a white solid (300 mg, 0.8844 mmol) with a yield of 68%. LC-MS: 326.1 [M+H] detection value.

### Step D: Synthesis of 4-chloro-3-(2-chloro-6-oxo-6H-pyrimido[1,6-b]pyridazin-5-yl)-N-methylbenzamide

3-(2-Amino-1-(6-chloropyridazin-3-yl)-2-oxoethyl)-4-chloro-N-methylbenzamide (300 mg, 0.88 mmol) and N,N-dimethylformamide dimethyl acetal (635 mg, 5.32 mmol) were added to 10 mL of tetrahydrofuran, and the reaction solution was heated to 60 °C for 2 hours. The reaction solution was concentrated under reduced pressure to obtain a residue, which was purified by column chromatography to obtain a yellow solid (78 mg, 0.22 mmol) with a yield of 25%. LC-MS: 349.1 [M+H] detection value.

### Step E: Synthesis of 4-chloro-3-(2-(2,4-difluorophenoxy)-6-oxo-6H-pyrimido [1,6-b]pyridazin-5-yl)-N-methylbenzamide

4-Chloro-3 -(2-chloro-6-oxo-6*H*-pyrimido[1,6-b]pyridazin-5-yl)-N-methylbenzamide (78 mg, 0.22 mmol), 2,4-difluorophenol (35 mg, 0.26904 mmol) and cesium carbonate (147 mg, 0.45 mmol) were added to 3 mL of N,N-dimethylformamide. The reaction solution was reacted at room temperature for 16 hours. The reaction solution was diluted with water and extracted with ethyl acetate (50 mL x 3). The organic phases were combined, washed with brine, dried over anhydrous sodium sulfate and concentrated under reduced pressure to obtain a residue, which was purified by preparative TLC to obtain yellow solid (20 mg, 0.04 mmol) with a yield of 20%. LC-MS: 443.1[M+H] detection value; ¹H NMR (400 MHz, CDCl₃) δ 8.56 (s, 1H), 7.80 (dd, *J* = 8.4, 2.4 Hz, 1H), 7.72-7.71 (m, 2H), 7.46 (d, *J =* 8.4 Hz, 1H), 7.32 - 7.25 (m, 1H), 7.17 (d, *J* = 10.0 Hz, 1H), 7.10 - 6.96 (m, 2H), 6.90 (d, J = 10.0 Hz, 1H), 2.84 (d, *J =* 4.4 Hz, 3H).

### Embodiment 47 Synthesis of 5-(2,3-dichlorophenyl)-2-(2,4-difluorophenoxy)-6H-pyrimido[1,6-b]pyridazin-6-one (I-52)

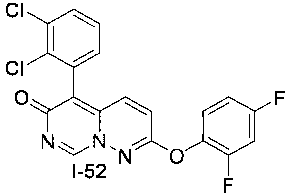

### Step A: Synthesis of 2-(6-chloropyridazin-3-yl)-2-(2,3-dichlorophenyl) acetonitrile.

To a 100 mL reaction round-bottom flask, 1.0 g of 2-(2,3-dichlorophenyl) acetonitrile, 1.24 g of 3,6-dichloropyridazine and 20 mL of tetrahydrofuran were added sequentially, and 0.9 g of anhydrous potassium tert-butoxide was added to the reaction mixture finally. The reaction solution was heated to reflux overnight, then cooled to 0 °C. The reaction solution was quenched with saturated ammonium chloride solution, added with IN diluted hydrochloric acid to adjust the pH to 5, and extracted with ethyl acetate (30 mL x 3) finally. The obtained extracts were combined, washed with brine three times, dried over anhydrous sodium sulfate, filtered, and concentrated under reduced pressure to obtain a crude product. The obtained crude product was purified by column chromatography to obtain a brown solid (0.8 g, with a yield of 39.9%). LC-MS: 298.1 [M+H] detection value.

### Step B: Synthesis of 2-(6-chloropyridazin-3-yl)-2-(2,3-dichlorophenyl)acetamide

To a 100 mL round bottom flask, 0.8 g of 2-(6-chloropyridazin-3-yl)-2-(2,3-dichlorophenyl)acetonitrile was added, and then 3 mL of concentrated sulfuric acid was added. The solution was heated to 100°C and stirred for two hours. The reaction solution was cooled to room temperature, quenched with ice water, and then added with saturated sodium bicarbonate solution to adjust the pH to 6, and then extracted with ethyl acetate three times. The extracts were combined, washed with water once, washed with saturated brine once, partitioned, dried over anhydrous sodium sulfate, filtered, and concentrated to obtain a crude product (0.6 g, with a yield of 71.0%), which was used directly in the next step. LC-MS: 316.1 [M+H] detection value.

### Step C: Synthesis of 2-chloro-5-(2,3-dichlorophenyl)-6H-pyrimido[1,6-b] pyridazin-6-one

To a 100 mL round bottom flask, 20 mL of toluene, 0.6 g of 2-(2-chloro-6-fluorophenyl)-2-(6-chloropyridazin-3 -yl)acetamide, trimethyl orthoformate (1.21 g, 11.4 mmol) were added, and 20 mg of p-toluenesulfonic acid as a catalyst was added finally. The reaction solution was heated to reflux for 2 hours and cooled to room temperature. The end of the reaction was monitored by TLC. The reaction was quenched with 30 mL of water, extracted with ethyl acetate (25 mL X 3). The extracts were combined, washed with saturated brine three times, and partitioned. The obtained ethyl acetate phase was dried over anhydrous sodium sulfate, filtered, and concentrated under reduced pressure to obtain a crude product. The obtained crude product was purified by column chromatography to obtain a yellow solid (0.33 g, 1.01 mmol) with a yield of 53.2%. LC-MS: 326.1 [M+H] detection value.

### Step D: Synthesis of 5-(2,3-dichlorophenyl)-2-(2,4-difluorophenoxy)-6H-pyrimido[1,6-b]pyridazin-6-one.

100 mg of 2-chloro-5-(2,3-dichlorophenyl)-6*H*-pyrimido[1,6-b]pyridazin-6-one was dissolved in 5 mL of N,N-dimethylformamide, and anhydrous cesium carbonate (150 mg, 0.46 mmol) was added at room temperature, and then 2,4-difluorophenol (48 mg, 0.37 mmol) was added. The reaction was stirred at room temperature for 16 hours. The end of the reaction was monitored by TLC. The reaction was quenched with 30 mL of water, extracted with ethyl acetate (30 mL X 3). The obtained extracts were combined, washed with water three times and partitioned. The obtained ethyl acetate solution was dried over anhydrous sodium sulfate, filtered, and concentrated under reduced pressure to obtain a crude product. The crude product was purified by column chromatography to obtain a yellow solid (25 mg, 0.06 mmol) with a yield of 19.4%. LC-MS: 326.1 [M+H] detection value; ¹H NMR (400 MHz, DMSO-d₆) δ 8.75 (d, J = 0.4 Hz, 1H), 7.75 (dd, J1 = 8.4 Hz, J2 = 1.6 Hz, 1H), 7.66-7.56 (m, 2H), 7.51-7.47 (m, 1H), 7.38-7.34 (m, 2H), 7.28-7.20 (m, 2H).

### Embodiment 48 Synthesis of 2-chloro-3-(2-(2,4-difluorophenoxy)-6-oxo-6H-pyrimidinyl[1,6-b]pyridazin-5-yl)-N-ethylbenzamide (I-53)

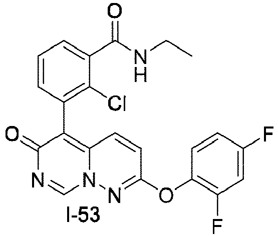

### Step A: Synthesis of methyl 2-chloro-3-methylbenzoate

To a 100 mL round bottom flask, 2-chloro-3-methylbenzoic acid (1.0 g, 5.86 mmol) was added, then methanol (15 mL) as solvent was added, and 5 drops of concentrated sulfuric acid was added as catalyst finally. The reaction solution was heated to reflux and stirred for 16 hours. The reaction solution was cooled to room temperature, concentrated under reduced pressure, and then dissolved with ethyl acetate (40 mL). The ethyl acetate solution was washed with saturated sodium bicarbonate solution (15 mL x 2), water (15 mL x 2), saturated brine (15 mL x 1) sequentially, dried over anhydrous sodium sulfate, filtered. The filtrate was concentrated to obtain a colorless oil (1.05 g, 5.68 mmol) with a yield of 97%. ¹H NMR (400 MHz, CDCl₃) δ 7.60-7.58 (m, 1H), 7.40-7.37 (m, 1H), 7.24-7.21 (m, 1H), 3.95 (s, 3H), 2.45 (s, 2H).

### Step B: Synthesis of methyl 3-(bromomethyl)-2-chlorobenzoate

To a 100 mL round bottom flask, methyl 2-chloro-3-methylbenzoate (0.63 g, 3.41 mmol), N-bromosuccinimide (0.55 g, 3.07 mmol), azo bisisobutyronitrile (20 mg) were added sequentially, and carbon tetrachloride (40 mL) as solvent was added finally. The reaction solution was heated to reflux and stirred for 16 hours. The reaction solution was cooled to room temperature and filtered. The filtrate was concentrated under reduced pressure. The obtained crude product was purified by column chromatography to obtain a colorless oily product (0.6 g, 2.28 mmol) with a yield of 66%.

### Step C: Synthesis of methyl 2-chloro-3-(cyanomethyl)benzoate

To a 100 mL round bottom flask, tetrahydrofuran (20 mL) as solvent was added, and then methyl 3-(bromomethyl)-2-chlorobenzoate (0.56 g,2.14 mmol), trimethylsilyl cyanide (0.32 g,3.21 mmol), and tetrabutylammonium fluoride tetrahydrofuran solution (1M, 3.21 mL, 3.21 mmol) were added. The reaction solution was stirred at room temperature overnight. The reaction solution was concentrated under reduced pressure to obtain a residue. The obtained residue was dissolved in ethyl acetate (40 mL), washed with water (20 mL x 2) and saturated brine (20 mL x 2) sequentially, dried over anhydrous sodium sulfate, filtered, and the filtrate was concentrated. The crude product was purified by column chromatography to obtain a white solid (0.31 g, 1.47 mmol) with a yield of 68%. ¹HNMR (400 MHz, CDCl₃) δ 7.82-7.80 (m, 1H), 7.73-7.71 (m, 1H), 7.42 (t, *J=* 8.0 Hz, 1H), 3.98 (s, 3H), 3.94 (s, 2H).

### Step D: Synthesis of 2-chloro-3-(cyanomethyl)benzoic acid

To a 100 mL round bottom flask, methyl 2-chloro-3-(cyanomethyl)benzoate (0.31 g, 1.47 mmol), lithium hydroxide monohydrate (90 mg, 2.2 mmol) were added, and tetrahydrofuran (5 mL), water (2 mL) as solvent were added finally. The reaction solution was stirred at room temperature overnight. Dilute hydrochloric acid (2M) was added dropwise to the reaction solution until the pH of the solution was 4. Then the reaction solution was added with ethyl acetate (30 mL) and water (20 mL), and partitioned. The aqueous phase was extracted with ethyl acetate (20 mL x 2), and the obtained ethyl acetate solution was combined. The combined extracted phases were washed with saturated brine (20 mL x 2), dried over anhydrous sodium sulfate, filtered, and concentrated to obtain a crude product as white solid (0.28 g, 1.45 mmol) with a yield of 98%. ¹H NMR (400 MHz, DMSO) δ 13.58 (br, 1H), 7.75-7.69 (m, 2H), 7.49 (t, *J=* 7.6 Hz, 1H), 4.17 (s, 2H).

### Step E: Synthesis of 2-chloro-3-(cyanomethyl)-N-ethylbenzamide

To a 100 mL round bottom flask, 2-chloro-3-(cyanomethyl)benzoic acid (0.28 g, 1.45 mmol), ethylamine tetrahydrofuran solution (2 M, 0.87 mL, 1.74 mmol), 2-(7-azobenzotriazole)-N,N,N',N'-tetramethyluronium hexafluorophosphate (0.83 g, 1.74 mmol), triethylamine (0.3 g, 2.9 mmol), 4-dimethylaminopyridine (5 mL) were added, and then dichloromethane (10 mL) as solvent was added finally. The reaction solution was stirred at room temperature overnight. Then the reaction solution was diluted with dichloromethane (30 mL). The reaction solution was washed with water (20 mL x 2) and saturated brine (20 mL x 2) sequentially, dried over anhydrous sodium sulfate, filtered and concentrated to obtain a crude product. The crude product was purified by column chromatography to obtain a white solid (215 mg, 0.97 mmol) with a yield of 66%. LCMS: 223[M+H] detection value; ¹H NMR (400 MHz, DMSO) δ 8.49 (t, *J* = 5.6 Hz, 1H), 7.59 (dd, *J1* = 1.6 Hz, *J2* = 7.2 Hz, 1H), 7.46-7.38 (m, 2H), 4.14 (s, 2H), 3.29-3.22 (m, 2H), 1.12 (t, *J=* 7.2 Hz, 3H).

### Step F: Synthesis of 2-chloro-3-((6-chloropyridazin-3-yl)(cyano)methyl)-N-ethylbenzamide

To a 100 mL round bottom flask, 2-chloro-3-(cyanomethyl)-N-ethylbenzamide (175 mg, 0.79 mmol), 3,6-dichloropyridazine (117 mg, 0.79 mmol) were added, and then anhydrous tetrahydrofuran (10 mL) as solvent was added. Sodium hydride (60%, 63.2 mg, 1.58 mmol) was added to the reaction solution in batches, and the reaction solution was stirred at room temperature for 2 hours. The reaction solution was added dropwise to ice saturated ammonium chloride solution, and extracted with ethyl acetate (30 mL x 2). The extracts were combined, washed with water (30 mL x 1) and saturated brine (30 mL x 1), dried over anhydrous sodium sulfate, filtered and concentrated. The obtained crude product was purified by column chromatography under reduced pressure to obtain a brown solid (200 mg, 0.6 mmol) with a yield of 75%. LCMS: 335[M+H]⁺.

### Step G: Synthesis of 3-(2-amino-1-(6-chloropyridazin-3-yl)-2-oxoethyl)-2-chloro-N-ethylbenzamide

To a 100 mL round bottom flask, 2-chloro-3-((6-chloropyridazin-3-yl)(cyano)methyl)-N-ethylbenzamide (200 mg, 0.6 mmol) was added, and then concentrated sulfuric acid (2 mL) as solvent was added. The reaction solution was heated to 50°C and then cooled to room temperature. The reaction solution was quenched with ice water (10 mL), and then added with saturated sodium bicarbonate solution dropwise to adjust the pH of the solution to 5. The reaction solution was extracted with ethyl acetate (30 mL x 3). The extracts were combined, washed with water (30 mL x 1) and saturated sodium chloride solution (30 mL x 1), dried over anhydrous sodium sulfate, filtered and concentrated to obtain a crude product as a brown solid (140 mg, 0.4 mmol) with a yield of 66%. LCMS: 353 [M+H] detection value.

### Step H: Synthesis of 2-chloro-3-(2-chloro-6-oxo-6H-pyrimidinyl[1,6-b] pyridazin-5-yl)- N-ethylbenzamide

To a 100 mL round bottom flask, 3-(2-amino-1-(6-chloropyridazin-3-yl)-2-oxoethyl)-2-chloro-N-ethylbenzamide (0.14 g,0.4 mmol), trimethyl orthoformate (0.25 g,2.4 mmol) were added, and p-toluenesulfonic acid monohydrate (10 mg) was added as catalyst, and then toluene (10 mL) as solvent was added finally. The reaction solution was heated to 100°C and stirred for 2 hours, and then cooled to room temperature. The reaction solution was diluted with ethyl acetate (30 mL), washed with water (20 mL x 2) and with saturated brine (30 mL x 1), partitioned. The ethyl acetate phases were dried over anhydrous sodium sulfate and filtered. The filtrate was concentrated under pressure to obtain a crude product. The obtained crude product was purified by column chromatography to obtain a brown solid (80 mg, 0.22 mmol) with a yield of 55%. LCMS: 363[M+H] detection value.

### Step I: Synthesis of 2-chloro-3-(2-(2,4-difluorophenoxy)-6-oxo-6H-pyrimidinyl [1,6-b] pyridazin-5-yl)-N-ethylbenzamide

To a 100 mL round bottom flask, 3-(2-chloro-6-oxo-6*H*-pyrimidinyl[1,6-b] pyridazin-5-yl)-N-ethyl-2-methoxybenzamide (120 mg, 0.33 mmol), 2,4-difluorophenol (65 mg, 0.5 mmol), N,N-diisopropylethylamine (128 mg, 0.99 mmol) were added, and acetonitrile (10 mL) as solvent was added finally. The reaction solution was heated to 50°C and stirred for 3 hours. The reaction solution was concentrated under reduced pressure to obtain a residue. The residue was dissolved in ethyl acetate (50 mL), washed with water (15 mL x 3) and saturated brine (15 mL x 1) sequentially, filtered and concentrated under reduced pressure to obtain a crude product. The crude product was purified by column chromatography to obtain yellow solid (75 mg, 0.165 mmol) with a yield of 50%. LCMS: 453[M+H] detection value; ¹H NMR (400 MHz, CDCl₃) δ 8.60 (s, 1H), 7.61 (d, J = 7.2 Hz, 1H), 7.48-7.38 (m, 2H), 7.29-7.24 (m, 2H), 7.08-6.98 (m, 2H), 6.89 (d, *J* = 9.6 Hz, 1H), 6.17 (br, 1H), 3.62-3.46 (m, 2H), 1.28 (t, *J* = 7.2 Hz, 3H).

### Embodiment 49 Synthesis of 3-(2-(2,4-difluorophenoxy)-6-oxo-6H-pyrimidinyl [1,6-b]pyridazin-5-yl)-N-ethyl-2-methoxybenzamide (I-54)

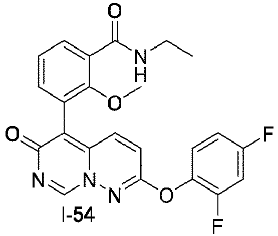

### Step A: Synthesis of methyl 2-methoxy-3-methylbenzoate

To a 100 mL round bottom flask, 3-methylsalicylic acid (5.0 g, 32.86 mmol), potassium carbonate (13.6 g, 98.6 mmol), iodomethane (10.26 g, 72.3 mmol) were added, and anhydrous N,N-dimethylformamide (50 mL) as solvent was added finally. The reaction solution was heated to 50 °C and stirred overnight. The reaction solution was filtered and the filter cake was washed with ethyl acetate (100 mL x 2). The filtrate was washed with water (200 mL x 2), then washed with saturated brine (100 mL x 2), dried over anhydrous sodium sulfate, filtered and concentrated under reduced pressure to obtain a light brown oil (5.1 g,28.3 mmol) with a yield of 86%. The obtained crude product was used directly in the next step. ¹H NMR (400 MHz, CDCl₃) δ 7.67-7.64 (m, 1H), 7.38-7.35 (m, 1H), 7.07 (t, *J* = 7.6 Hz, 1H), 3.93 (s, 3H), 3.85 (s, 3H), 2.34 (s, 3H).

### Step B: Synthesis of methyl 3-(bromomethyl)-2-methoxybenzoate

To a 100 mL round bottom flask, methyl 2-methoxy-3-methylbenzoate (2.6 g, 13.45 mmol), N-bromosuccinimide (2.27 g, 12.78 mmol) were added, and then benzoyl peroxide (20 mg) as catalyst, carbon tetrachloride (50 mL) as solvent were added. The reaction solution was heated to reflux and stirred for 16 hours. The reaction solution was cooled to room temperature, filtered to remove the insoluble substances, and the filtrate was concentrated under reduced pressure to obtain a crude product. The obtained crude product was purified by column chromatography to obtain a product as colorless oil (3.75 g, 14.47 mmol) with a yield of 100%.

### Step C: Synthesis of methyl 3-(cyanomethyl)-2-methoxybenzoate

To a 100 mL round bottom flask, methyl 3-(bromomethyl)-2-methoxybenzoate (3.75 g, 14.47 mmol), tetrabutylammonium fluoride tetrahydrofuran solution (1 M, 21.7 mL, 21.7 mmol), trimethylsilyl cyanide (2.15 g, 21.7 mmol) were added, and then anhydrous tetrahydrofuran (20 mL) as solvent was added. The reaction solution was stirred at room temperature for 16 hours. The reaction solution was concentrated under reduced pressure. The residue was dissolved in ethyl acetate (60 mL), washed with water (30 mL x 2) and saturated brine (30 mL x 1), dried over anhydrous sodium sulfate, filtered and concentrated under reduced pressure. The crude product was purified by column chromatography to obtain a colorless oil (2.65 g, 12.9 mmol) with a yield of 89%. ¹H NMR (400 MHz, CDCl₃) δ 7.85 (dd, *J1* = 1.6 Hz, *J2* = 8.0 Hz, 1H), 7.62-7.60 (m, 1H), 7.21 (t, *J=* 7.6 Hz, 1H), 3.97 (s, 3H), 3.93 (s, 3H), 3.81 (s, 2H).

### Step D: Synthesis of 3-(cyanomethyl)-2-methoxybenzoic acid

To a 100 mL round bottom flask, methyl 3-(cyanomethyl)-2-methoxybenzoate (0.3 g, 1.66 mmol), lithium hydroxide monohydrate (84 mg, 2.0 mmol) were added, and then tetrahydrofuran (5 mL) and water (1 mL) as solvent were added. The reaction solution was stirred at room temperature for 16 hours. Dilute hydrochloric acid (2M) was added dropwise to the reaction solution to adjust the pH of the solution to 4. The solution was extracted with ethyl acetate (20 mL x 2), partitioned. The extracts were combined, washed with water (30 mL x 2) and saturated brine (30 mL x 1), dried over anhydrous sodium sulfate, filtered and concentrated under reduced pressure to obtain a white solid (288 mg,1.5 mmol) with a yield of 90% yield.

### Step E: Synthesis of 3-(cyanomethyl)-N-ethyl-2-methoxybenzamide

To a 100 mL round bottom flask, 3-(cyanomethyl)-2-methoxybenzoic acid (0.22 g,1.33 mmol), O-(7-azabenzotriazol-1-yl)-N,N,N',N'-tetramethyluronium hexafluorophosphate (760 mg, 2.0 mmol), N,N-diisopropylethylamine (0.27 g, 2.66 mmol) were added, and dichloromethane (10 mL) as solvent was added, and then ethylamine tetrahydrofuran solution (2M, 0.8 mL,1.6 mmol) was added finally. The reaction solution was stirred at room temperature for 16 hours. The reaction solution was added with dichloromethane (30 mL), then washed with water (15 mL x 2) and saturated brine (30 mL x 1), dried over anhydrous sodium sulfate, filtered and concentrated. The crude product was purified by column chromatography to obtain a white solid (220 mg, 1.0 mmol) with a yield of 75%. LCMS: 219 [M+H] detection value.

### Step F: Synthesis of 3-((6-chloropyridazin-3-yl)(cyano)methyl)-N-ethyl-2-methoxybenzamide

To a 100 mL round bottom flask, 3-(cyanomethyl)-N-ethyl-2-methoxybenzamide (220 mg, 1.0 mmol), 3,6-dichloropyridazine (149 mg, 1.0 mmol) were added, and then anhydrous tetrahydrofuran (10 mL) as solvent was added. Sodium hydride (60%, 80 mg, 2.0 mmol) was added to the reaction solution in batches. The reaction solution was stirred for 2 hours at room temperature. The reaction solution was added dropwise to ice saturated ammonium chloride solution, extracted with ethyl acetate (30 mL x 2). The extracts were combined, washed with water (30 mL x 1) and saturated brine (30 mL x 1), dried over anhydrous sodium sulfate, filtered and concentrated. The crude product was purified by column chromatography under reduced pressure to obtain a brown solid (230 mg, 0.69 mmol) with a yield of 69%. LCMS: 331 [M+H] detection value.

### Step G: Synthesis of 3-(2-amino-1-(6-chloropyridazin-3-yl)-2-oxoethyl)-N-ethyl-2-methoxybenzamide

To a 100 mL round bottom flask, 3-((6-chloropyridazin-3-yl)(cyano)methyl)-N-ethyl-2-methoxybenzamide (230 mg, 0.69 mmol) was added, and then concentrated sulfuric acid (2 mL) as solvent was added. The reaction solution was heated to 50°C and then cooled to room temperature. The reaction solution was quenched with ice water (10 mL), and then added with saturated sodium bicarbonate solution dropwise to adjust the pH of the solution to 5, and extracted with ethyl acetate (30 mL x 3). The extracts were combined, washed with water (30 mL x 1) and saturated sodium chloride solution (30 mL x 1), dried over anhydrous sodium sulfate, filtered and concentrated to obtain a crude product as brown solid (230 mg, 0.69 mmol) with a yield of 100%. LCMS: 349 [M+H] detection value.

### Step H: Synthesis of 3-(2-chloro-6-oxo-6H-pyrimidinyl[1,6-blpyridazin-5-yl)-N-ethyl-2-methoxybenzamide

To a 100 mL round bottom flask, 3-(2-amino-1-(6-chloropyridazin-3-yl)-2-oxoethyl)-N-ethyl-2-methoxybenzamide (0.23 g, 0.66 mmol), trimethyl orthoformate (0.56 g, 5.28 mmol) were added, and p-toluenesulfonic acid monohydrate (10 mg) was added as catalyst, and then toluene (10 mL) was added as the solvent finally. The reaction solution was heated to 100 °C and stirred for 2 hours, and then cooled to room temperature. The reaction solution was diluted with ethyl acetate (30 mL), washed with water (20 mL x 2), washed with saturated brine (30 mL x 1), partitioned. The ethyl acetate phase was dried over anhydrous sodium sulfate and filtered. The filtrate was concentrated under pressure to obtain a crude product. The obtained crude product was purified by column chromatography to obtain a brown solid (120 mg, 0.33 mmol) with a yield of 50%. LCMS: 359 [M+H] detection value.

### Step I: Synthesis of 3-(2-(2,4-difluorophenoxy)-6-oxo-6H-pyrimidinyl[1,6-b] pyridazin-5-yl)-N-ethyl-2-methoxybenzamide

To a 100 mL round bottom flask, 3-(2-chloro-6-oxo-6*H*-pyrimidinyl[1,6-b] pyridazin-5-yl)-N-ethyl-2-methoxybenzamide (120 mg, 0.33 mmol), 2,4-difluorophenol (65 mg, 0.5 mmol), N,N-diisopropylethylamine (128 mg, 0.99 mmol) were added, and acetonitrile (10 mL) as solvent was added finally. The reaction solution was heated to 50 °C and stirred for 3 hours. The reaction solution was concentrated under reduced pressure to obtain residue. The residue was dissolved in ethyl acetate (50 mL), washed with water (15 mL x 3) and saturated brine (15 mL x 1) sequentially, filtered and concentrated under reduced pressure. The crude product was purified by column chromatography to obtain a yellow solid (75 mg, 0.165 mmol) with a yield of 50%. LCMS: 453[M+H] detection value; ¹H NMR (400 MHz, CDCl₃) δ 8.61 (br, 1H), 8.18 (dd, *J1* = 1.6 Hz, *J2* = 7.6 Hz, 1H), 7.68 (br, 1H), 7.46 (dd, *J1* = 1.6 Hz, *J2* = 7.6 Hz, 1H), 7.39-7.30 (m, 2H), 7.27-7.24 (m, 1H), 7.09-6.99 (m, 2H), 6.88 (d, *J* = 9.6 Hz, 1H), 3.64 (s, 3H), 3.63-3.45 (m, 2H), 1.28 (t, *J=* 7.2 Hz, 3H).

### Embodiment 50 Synthesis of methyl 3-[2-(2,4-difluorophenoxy)-6-oxo-pyrimido [1,6-b]pyridazin-5-yl]-4-methoxybenzoate (I-55)

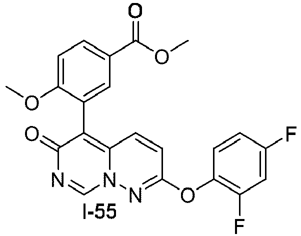

### Step A: Synthesis of methyl 4-methoxy-3-methyl-benzoate

The reaction solution of 4-hydroxy-3-methyl-benzoic acid (15.22 g, 100.0 mmol), iodomethane (19.0 mL, 305 mmol), and potassium carbonate (41.5 g, 300 mmol) in acetonitrile (200 mL) was stirred at reflux for 16 hours. The reaction solution was cooled to room temperature, filtered, washed with dichloromethane. The filtrate was evaporated to dryness, added with water (50 mL), extracted with ethyl acetate (50 mLx3). The organic phases were combined, washed with saturated saline, evaporated to dryness to obtain methyl 4-methoxy-3-methyl-benzoate as a light red solid (16.8 g, 93.2 mmol with a yield of 93.2%).

### Step B: Synthesis of methyl 3-(bromomethyl)-4-methoxy-benzoate

Methyl 4-methoxy-3-methyl-benzoate (16.8 g, 93.2 mmol), N-bromosuccinimide (17.9 g, 101 mmol), azo bisisobutyronitrile (1.53 g, 9.32 mmol) were in carbon tetrachloride (230 mL). The reaction solution was stirred at reflux for 16 hours under argon protection. The reaction solution was cooled to room temperature, filtered. The filtrate was evaporated to dryness and purified by column chromatography (PE/EA=0->10%->20%) to obtain methyl 3-(bromomethyl)-4-methoxy-benzoate as a light red solid (22.5 g, 86.8 mmol with a yield of 93.1%).

### Step C: Synthesis of methyl 3-(cyanomethyl)-4-methoxy-benzoate

Methyl 3-(bromomethyl)-4-methoxy-benzoate (10.36 g, 39.98 mmol) was dissolved in acetonitrile (200 mL), and trimethylsilyl cyanide (7.5 mL, 60 mmol), tetrabutylammonium fluoride (1M in THF) (60 mL, 60 mmol) were added under ice bath. The reaction solution was stirred at room temperature for 2 hours, evaporated under reduced pressure to dryness, added with water (100 mL), extracted (60 mLx3), evaporate under reduced pressure to dryness, purified by column chromatography (EA/PE=0->20% -> MeOH/DCM=0->2%), to obtain methyl 3-(cyanomethyl)-4-methoxy-benzoate (6.6 g, 32 mmol, with a yield of 80%) as a pale yellow solid. LC-MS: 206 [M+H] detection value.

### Step D: Synthesis of methyl 3-[(6-chloropyridazin-3-yl)(cyano)methyl]-4-methoxybenzoate

Methyl 3-(cyanomethyl)-4-methoxy-benzoate (4.5 g, 22 mmol) was in N,N-dimethylformamide (44 mL), and sodium hydrogen (2.4 g, 60 mmol) was added under ice bath and stirred for 15 minutes, and then the solution of 3,6-dichloropyridazine (6 g, 40.274 mmol) in N,N-dimethylformamide (10 mL) was added. The reaction solution was stirred at room temperature for 2 hours. The reaction solution was quenched with water (500 mL) under ice bath, extracted with ethyl acetate (100 mLx3), evaporated to dryness under reduced pressure, slurried with ethyl acetate and filtered to obtain earthy yellow solid. The filtrate was evaporated to dryness and purified by column chromatography (EA/PE=0->50% -> MeOH/DCM=0->2%) to obtain yellow-brown solid, which was combined to obtain methyl 3-[(6-chloropyridazin-3-yl)(cyano)methyl]-4-methoxybenzoate (4.8 g, 15 mmol, with a yield of 69%). LC-MS: 318[M+H] detection value.

### Step E: Synthesis of 3-[2-amino-1-(6-chloropyridazin-3-yl)-2-oxo-ethyl]-4-methoxy-benzoic acid

Methyl 3-[(6-chloropyridazin-3-yl)(cyano)methyl]-4-methoxybenzoate (4.8 g, 15 mmol) was in sulfuric acid (30 mL), and the reaction solution was stirred at 100 °C for 2 hours. The reaction solution was poured into ice water mixture (500 mL), with a solid was precipitated, filtered, washed with water, and dried to obtain 3-[2-amino-1-(6-chloropyridazin-3-yl)-2-oxo-ethyl]-4-methoxy-benzoic acid as a brown solid (4.2 g, 13 mmol, with a yield of 86%). LC-MS: 322[M+H] detection value.

### Step F: Synthesis of methyl 3-[2-amino-1-(6-chloropyridazin-3-yl)-2-oxo-ethyl] -4-methoxybenzoate

3-[2-Amino-1-(6-chloropyridazin-3-yl)-2-oxo-ethyl]-4-methoxy-benzoic acid (3.1 g, 9.6 mmol) was in methanol (5 mL) and dichloromethane (50 mL), and trimethylsilyl diazomethane (2M in Hexane) (10 mL, 20 mmol) was added in batches. The reaction solution was stirred at room temperature for 2 hours. The reaction was quenched with trifluoroacetic acid, evaporated under reduced pressure to dryness to obtain methyl 3-[2-amino-1-(6-chloropyridazin-3-yl)-2-oxo-ethyl]-4-methoxybenzoate as a brown solid (3.3 g, 9.8 mmol, with a yield of 100%).
LC-MS: 336[M+1]⁺

### Step G: Synthesis of methyl 3-(2-chloro-6-oxo-pyrimido[1,6-b]pyridazin-5-yl)-4-methoxybenzoate

Methyl 3-[2-amino-1-(6-chloropyridazin-3 -yl)-2-oxo-ethyl]-4-methoxybenzoate (300 mg, 0.8937 mmol) was in tetrahydrofuran (8 mL), and N,N-dimethylformamide dimethyl acetal (0.5 mL) was added. The reaction solution was reacted at 100°C for 2 hours in sealed tube under argon protection. The reaction solution was evaporated under reduced pressure to dryness, purified by column chromatography (MeOH/DCM=0->5%) to obtain methyl 3-(2-chloro-6-oxo-pyrimido[1,6-b]pyridazin-5-yl)-4-methoxybenzoate as a yellow solid (190 mg, 0.5496 mmol, with a yield of 61.50%). LC-MS: 346[M+H] detection value.

### Step H: Synthesis of methyl 3-[2-(2,4-difluorophenoxy)-6-oxo-pyrimido[1,6-b] pyridazin-5-yl]-4-methoxybenzoate

Methyl 3-(2-chloro-6-oxo-pyrimido[1,6-b]pyridazin-5-yl)-4-methoxybenzoate (190 mg, 0.5496 mmol), 2,4-difluorophenol (100 mg, 0.76870 mmol), cesium carbonate (325 mg, 0.99748 mmol) were dissolved in N,N-dimethylformamide (5 mL). The reaction solution was stirred at room temperature for 16 hours. The reaction solution was evaporated under reduced pressure to dryness, added with water (20 mL), extracted with ethyl acetate (10 mLx3), evaporated to dryness, purified by column chromatography (MeOH/DCM=0->5%) to obtain methyl 3-[2-(2,4-difluorophenoxy)-6-oxo-pyrimido[1,6-b]pyridazin-5-yl]-4-methoxybenzoate as a yellow solid (210 mg, 0.4779 mmol, with a yield of 86.96%). LC-MS: 440 [M+H] detection value; ¹H NMR (400 MHz, *Chloroform-d)* δ 8.51 (d, *J* = 0.6 Hz, 1H), 8.12 (dd, *J =* 8.7, 2.2 Hz, 1H), 7.97 (d, *J =* 2.2 Hz, 1H), 7.27 - 7.19 (m, 3H), 7.09 - 6.91 (m, 3H), 6.78 (d, *J=* 9.9 Hz, 1H), 3.88 (s, 3H), 3.84 (s, 3H).

### Embodiment 51 Synthesis of 5-(2,4-dichlorophenyl)-2-(2,4-difluorophenoxy)-6H-pyrimido[1,6-b]pyridazin-6-one (I-56)

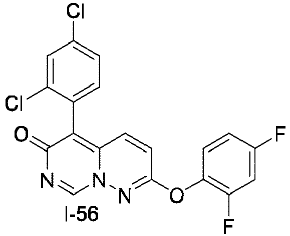

### Step A: Synthesis of 2-(6-chloropyridazin-3-yl)-2-(2,4-dichlorophenyl) acetonitrile.

To a 100 mL reaction round bottom flask, 0.5 g of 2-(2,4-dichlorophenyl) acetonitrile, 0.62 g of 3,6-dichloropyridazine, and 20 mL of tetrahydrofuran were added sequentially, and 0.45 g of anhydrous potassium tert-butoxide was added to the reaction mixture finally. The reaction solution was heated to reflux overnight, then cooled to 0 °C. The reaction solution was quenched with saturated ammonium chloride solution, added with IN diluted hydrochloric acid to adjust the pH to 5, and extracted with ethyl acetate three times finally. The obtained extracts were combined, washed with brine three times, dried over anhydrous sodium sulfate, filtered, and concentrated under reduced pressure to obtain a crude product. The obtained crude product was purified by column chromatography to obtain a brown solid (0.8 g, with a yield of 79%). LCMS: 298.1 [M+H] detection value.

### Step B: Synthesis of 2-(6-chloropyridazin-3-yl)-2-(2,4-dichlorophenyl) acetamide.

To a 100 mL round bottom flask, 0.8 g of 2-(6-chloropyridazin-3-yl)-2-(2, 4-dichlorophenyl)acetonitrile was added, and then 2 mL of concentrated sulfuric acid was added to the reaction solution. The reaction solution was heated to 100°C and stirred for two hours. The reaction solution was cooled to room temperature, quenched with ice water, and then added with saturated sodium bicarbonate solution to adjust the pH of the solution to 6, and then extracted with ethyl acetate three times. The extracts were combined, washed with water once and with saturated brine once, partitioned, dried over anhydrous sodium sulfate, filtered, and concentrated to obtain a crude product (0.6 g, with a yield of 70.7%). The crude product was used directly in the next step. LCMS: 316.1 [M+H] detection value.

### Step C: Synthesis of 2-chloro-5-(2,4-dichlorophenyl)-6H-pyrimido[1,6-b] pyridazin-6-one.

To a 100 mL round bottom flask, 20 mL of toluene, 0.6 g of 2-(6-chloropyridazin-3-yl)-2-(2,4-dichlorophenyl)acetamide, 1.21 g of trimethyl orthoformate were added, and 20 mg of p-toluenesulfonic acid as catalyst was added finally. The reaction solution was heated to reflux for 2 hours and cooled to room temperature. The end of the reaction was monitored by TLC. The reaction solution was quenched with 30 mL of water, extracted with ethyl acetate three times. The extracts were combined, washed with saturated brine three times, partitioned. The obtained ethyl acetate phase was dried over anhydrous sodium sulfate, filtered and concentrated under reduced pressure to obtain a crude product. The crude product was purified by column chromatography to obtain a yellow solid (0.32 g, 1.0 mmol) with a yield of 39.8%. LCMS: 326.1 [M+H] detection value.

### Step D: Synthesis of 5-(2,4-dichlorophenyl)-2-(2,4-difluorophenoxy)-6H-pyrimido[1,6-b]pyridazin-6-one.

100 mg of 2-chloro-5-(2,4-dichlorophenyl)-6*H*-pyrimido[1,6-b]pyridazin-6-one was dissolved in 10 mL of N,N-dimethylformamide, and anhydrous cesium carbonate (150 mg, 0.46 mmol) was added at room temperature, and then 2,4-difluorophenol (38 mg, 0.29 mmol) was added finally. The reaction was stirred at room temperature for 16 hours. The end of the reaction was monitored by TLC. The reaction solution was quenched with 20 mL of water, extracted with ethyl acetate (30 mL X 3). The obtained extracts were combined, washed with water three times, partitioned. The obtained ethyl acetate solution was dried over anhydrous sodium sulfate, filtered and concentrated under reduced pressure to obtain a crude product. The obtained crude product was purified by column chromatography to obtain a yellow solid (30 mg, 0.071 mmol) with a yield of 23.3%. LCMS: 420.0 [M+H] detection value; ¹H NMR (400 MHz, DMSO-d₆) δ 8.74 (s, 1H), 7.79 (d, *J =* 2.0 Hz, 1H), 7.66-7.55 (m, 3H), 7.41-7.36 (m, 2H), 7.27-7.21 (m, 2H).

### Embodiment 52 Synthesis of 5-(2,4,6-trifluorophenyl)-2-(2,4-difluorophenoxy)-6H-pyrimido [1,6-b]pyridazin-6-one (I-57)

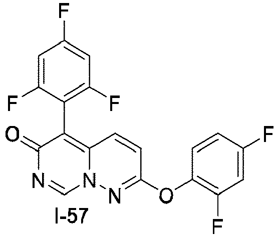

### Step A: Synthesis of 2-(6-chloropyridazin-3-yl)-2-(2,4,6-trifluorophenyl) acetonitrile

2-(2,4,6-Trifluorophenyl)acetonitrile (1 g, 5.8 mmol) and 3,6-dichloropyridazine (0.78 g, 5.3 mmol) were dissolved in 20 mL tetrahydrofuran, and potassium tert-butoxide (0.98 g, 8 mmol) was added at 0°C. The reaction solution was returned to room temperature and heated to reflux overnight. The reaction solution was neutralized with 1 N hydrochloric acid, extracted with ethyl acetate (80 mL x 3). The organic phases were combined, dried over anhydrous sodium sulfate, and concentrated under reduced pressure to obtain a residue. The residue was purified by column chromatography (eluent: petroleum ether/ethyl acetate = 4/1) to obtain a red solid (0.78 g, 2.7 mmol) with a yield of 47%. LC-MS: 284.0 [M+H] detection value.

### Step B: Synthesis of 2-(6-chloropyridazin-3-yl)-2-(2,4,6-trifluorophenyl) acetamide

2-(6-Chloro-pyridazin-3-yl)-2-(2,4,6-trifluorophenyl)acetonitrile (0.78 g, 2.7 mmol) was added to 10 mL of concentrated sulfuric acid, and the reaction solution was reacted at 100°C for 2 hours. The reaction solution was poured into water and extracted with ethyl acetate (50 mL x 3). The organic phases were combined, dried over anhydrous sodium sulfate and concentrated by reduced pressure to obtain a yellow solid (0.6 g, 2 mmol) with a yield of 70%. LC-MS: 302.0 [M+H] detection value.

### Step C: Synthesis of 2-chloro-5-(2,4,6-trifluorophenyl)-6H-pyrimido[1,6-b] pyridazin-6-one

2-Chloro-5-(2,4,6-trifluorophenyl)-6*H*-pyrimido[1,6-b]pyridazin-6-one (0.6 g, 2 mmol) and trimethyl orthoformate (0.63 g, 5.96 mmol), p-toluenesulfonic acid (0.017 g, 0.01 mmol) were added to 10 mL of toluene. The reaction solution was reacted at 100°C for 3 hours. The reaction solution was concentrated under reduced pressure to obtain a residue, and the obtained residue was purified by column chromatography (eluent: petroleum ether/ethyl acetate=1/2) to obtain a yellow solid (0.34 mg, 1.1 mmol) with a yield of 50%. LC-MS: 312.0 [M+H] detection value.

### Step D: Synthesis of 5-(2,4,6-trifluorophenyl)-2-(2,4-difluorophenoxy)-6H-pyrimido [1,6-b] pyridazin-6-one

2-Chloro-5-(2,4,6-trifluorophenyl)-6*H*-pyrimido[1,6-b]pyridazin-6-one (0.2 g, 0.6 mmol), 2,4-difluorophenol (84 mg, 0.65 mmol) and potassium carbonate (0.266 g, 1.9 mmol) were added to 10 mL of N,N-dimethylformamide. The reaction solution was stirred at room temperature overnight. The end of the reaction was monitored by TLC. The reaction solution was quenched with 20 mL of water, extracted with ethyl acetate (20 mL x 3). The obtained extracts were combined, washed with water three time, partitioned. The obtained ethyl acetate solution was dried over anhydrous sodium sulfate, filtered and concentrated under reduced pressure to obtain a crude product. The crude product was purified by column chromatography to obtain a yellow solid, and the obtained residue was purified by column chromatography (eluent: petroleum ether/ethyl acetate = 2/1) to obtain a yellow solid (0.107 g, 0.263 mmol) with a yield of 40%. LC-MS: 406.0 [M+H] detection value; ¹H NMR (400 MHz, *Chloroform-d)* δ 8.56 (d, *J* = 0.7 Hz, 1H), 7.33-7.25 (m, 2H), 7.09-6.97 (m, 2H), 6.95 (d, *J=* 9.9 Hz, 1H), 6.87-6.80 (m, 2H).

### Embodiment 53 Synthesis of 5-(3,4-dichlorophenyl)-2-(2,4-difluorophenoxy)-6H-pyrimido[1,6-b]pyridazin-6-one (I-58)

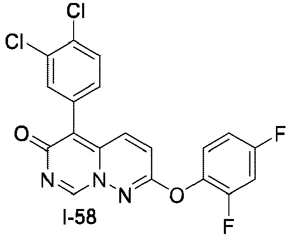

### Step A: Synthesis of 2-(6-chloropyridazin-3-yl)-2-(3,4-dichlorophenyl) acetonitrile:

To a 100 mL reaction round bottom flask, 1.0 g of 2-(3,4-dichlorophenyl)acetonitrile, 0.72 g of 3,6-dichloropyridazine, 20 mL of tetrahydrofuran were added sequentially, and 0.9 g of anhydrous potassium tert-butoxide was added to the reaction finally. The reaction solution was heated to reflux overnight, then cooled to 0 °C. The reaction solution was quenched with saturated ammonium chloride solution, added with IN dilute hydrochloric acid to adjust the pH to 5, and extracted with ethyl acetate three times finally. The obtained extracts were combined, washed with brine three times, dried over anhydrous sodium sulfate, filtered, and concentrated under reduced pressure to obtain a crude product. The obtained crude product was purified by column chromatography to obtain a brown solid (0.5 g, with a yield of 34.6%). LC-MS: 298.1 [M+H] detection value.

### Step B: Synthesis of 2-(6-chloropyridazin-3-yl)-2-(3,4-dichlorophenyl)acetamide

To a 100-mL round-bottom flask, 0.5 g of 2-(6-chloropyridazin-3-yl)-2-(3,4-dichlorophenyl)acetonitrile was added, and then 3 mL of concentrated sulfuric acid was added. The reaction solution was heated to 100°C and stirred for two hours. The reaction solution was cooled to room temperature, quenched with ice water, added with saturated sodium bicarbonate solution to adjust the pH to 6, and then extracted with ethyl acetate three times. The extracts were combined, washed with water once and with saturated brine once, partitioned, and dried over anhydrous sodium sulfate, filtered and concentrated to obtain a crude product (0.42 g, with a yield of 79.6%). The crude product was used directly in the next step. LC-MS: 316.1 [M+H] detection value.

### Step C: Synthesis of 2-chloro-5-(3,4-dichlorophenyl)-6H-pyrimido[1,6-b] pyridazin-6-one

To a 100 mL round bottom flask, 15 mL of toluene, 0.42 g of 2-(6-chloropyridazin-3-yl)-2-(3,4-dichlorophenyl)acetamide, 0.85 g of trimethyl orthoformate were added, and 30 mg of p-toluenesulfonic acid as catalyst was added finally. The reaction solution was heated to reflux for two hours, and cooled to room temperature. The end of the reaction was monitored by TLC. The reaction was quenched with 30 mL of water, extracted with ethyl acetate three times. The extracts were combined, washed with saturated brine three times, and partitioned. The obtained ethyl acetate phase was dried over anhydrous sodium sulfate, filtered and concentrated under reduced pressure to obtain a crude product. The obtained crude product was purified by column chromatography to obtain a yellow solid (0.2 g, 0.61 mmol) with a yield of 46.0%. LC-MS: 326.1 [M+H] detection value.

### Step D: Synthesis of 5-(3,4-dichlorophenyl)-2-(2,4-difluorophenoxy)-6H-pyrimido [1,6-b] pyridazin-6-one

80 mg of 2-chloro-5-(3,4-dichlorophenyl)-6*H*-pyrimido[1,6-b]pyridazin-6-one was dissolved in 10 mL of N,N-dimethylformamide, and anhydrous cesium carbonate (120 mg, 0.37 mmol) was added at room temperature, and then 2,4-difluorophenol (38 mg, 0.29 mmol) was added. The reaction solution was stirred at room temperature for 16 hours. The end of the reaction was monitored by TLC. The reaction was quenched with 30 mL of water, extracted with ethyl acetate three times. The extracts were combined, washed with water three times, and partitioned. The obtained ethyl acetate phase was dried over anhydrous sodium sulfate, filtered and concentrated under reduced pressure to obtain a crude product. The obtained crude product was purified by column chromatography to obtain yellow solid (60 mg, 0.183 mmol) with a yield of 58.2%. LCMS: 420.0 [M+H] detection value; ¹H NMR (400 MHz, DMSO-d₆) δ 8.70 (s, 1H), 7.75 (d, *J=* 4.0 Hz, 1H), 7.67-7.56 (m, 4H), 7.37 (dd, *J1* = 2.0 Hz, *J2* = 8.4 Hz, 1H), 7.27-7.19 (m, 2H).

### Embodiment 54 Synthesis of 5-(2,6-dichlorophenyl)-2-(2,4-difluorophenoxy)-3-(pyridin-4-yl)-6H-pyrimido[1,6-b]pyridazin-6-one (I-59)

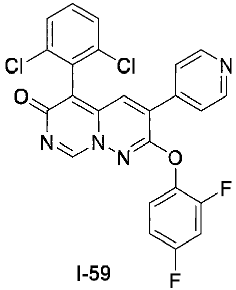

### Step A: Synthesis of 4-(pyridin-4-yl)-1,2-dihydropyridazine-3,6-dione

To a 100 mL round bottom flask, 4-bromo-1,2-dihydropyridazine-3,6-dione (1.0 g, 5.23 mmol), 4-pyridineboronic acid (0.68 g, 5.5 mmol), tetrakis(triphenylphosphine)palladium (0.6 g, 0.52 mmol), cesium carbonate (2.56 g, 7.85 mmol) were added sequentially, and then dioxane (50 mL), isopropanol (30 mL), and water (15 mL) as solvents were added. The reaction solution was replaced with argon gas three times, heated to reflux and stirred for 16 hours. The reaction solution was cooled to room temperature, concentrated under reduced pressure, and then extracted with dichloromethane (100 mL x 3). The extracts were combined, washed with saturated brine (150 mL x 1), dried over anhydrous sodium sulfate, and filtered and concentrated to obtain a brown crude product (0.8 g,4.22 mmol) with a yield of 80%. LCMS: 190[M+H] detection value.

### Step B: Synthesis of 3,6-dichloro-4-(pyridin-4-yl)pyridazine

To a 100 mL round bottom flask, 4-(pyridin-4-yl)-1,2-dihydropyridazine-3,6-dione (0.8 g, 4.22 mmol) was added, and then phosphorus oxychloride (10 mL) as solvent was added. The reaction solution was heated to reflux and stirred for 16 hours. The reaction was cooled to room temperature, poured into warm water, added with saturated sodium bicarbonate solution dropwise to adjust the pH to 8, and then extracted with dichloromethane (50 mL x 3). The extracts were combined, washed with water (50 mL x 3) and saturated brine (50 mL x 1), dried over anhydrous sodium sulfate, filtered, and concentrated under reduced pressure. The obtained crude product was purified by column chromatography to obtain a brown product (0.5 g, 4.22 mmol) with a yield of 52.6%. LCMS: 226[M+H] detection value.

### Step C: Synthesis of 2-(6-chloro-5-(pyridin-4-yl)pyridazin-3-yl)-2-(2,6-dichlorophenyl)acetonitrile

To a 100 mL round bottom flask, 3,6-dichloro-4-(pyridin-4-yl)pyridazine (0.41 g, 1.81 mmol), 2,6-dichlorophenylacetonitrile (0.38 g, 1.81 mmol) were added, and then tetrahydrofuran (10 mL) as solvent was added. The reaction solution was stirred at room temperature for 5 minutes, then potassium tert-butoxide (0.3 g, 2.72 mmol) was added one time. The reaction solution was stirred at room temperature for 16 hours. The reaction solution was added dropwise to 20 mL of saturated ammonium chloride solution, extracted with ethyl acetate (50 mL x 3). The extracts were combined, washed with water (50 mL x 1), saturated brine (50 mL x 1), dried over anhydrous sodium sulfate, filtered and concentrated. The obtained crude product was purified by column chromatography to obtain a brown solid (0.3 g, 0.8 mmol) with a yield of 44%. LCMS: 375[M+H] detection value.

### Step D: Synthesis of 2-(6-chloro-5-(pyridin-4-yl)pyridazin-3-yl)-2-(2,6-dichlorophenyl)acetamide

To a 100 mL round bottom flask, 2-(6-chloro-5-(pyridin-4-yl)pyridazin-3-yl)-2-(2,6-dichlorophenyl)acetonitrile (0.3 g, 0.8 mmol) was added, and then concentrated sulfuric acid (2 mL) was added. The reaction solution was heated to 100 °C and stirred for 2 hours. The reaction solution was cooled to room temperature, quenched with dropwise addition of water, added with saturated sodium bicarbonate solution dropwise to adjust the pH to 5, then extracted with ethyl acetate (50 mL x 3). The extracts were combined, washed with saturated brine (50 mL x 1), dried over anhydrous sodium sulfate, filtered and concentrated to obtain a brown crude product (0.27 g,0.68 mmol) with a yield of 85%. LCMS: 393[M+H] detection value.

### Step E: Synthesis of 2-chloro-5-(2,6-dichlorophenyl)-3-(pyridin-4-yl)-6H-pyrimido[1,6-b]pyridazin-6-one

To a 100 mL round bottom flask, 2-(6-chloro-5-(pyridin-4-yl)pyridazin-3-yl)-2-(2,6-dichlorophenyl)acetamide (0.27 g, 0.68 mmol), trimethyl orthoformate (0.45 g,4.1 mmol), p-toluenesulfonic acid (10 mg) were added sequentially, and toluene (10 mL) as solvent was added finally. The reaction solution was heated to 100 °C and stirred for 16 hours. The reaction solution was cooled to room temperature, concentrated under reduced pressure. The crude product was purified by column chromatography to obtain a brown solid (20 mg, 0.032 mmol) with a yield of 5%. LCMS: 403[M+H] detection value.

### Step F: Synthesis of 5-(2,6-dichlorophenyl)-2-(2,4-difluorophenoxy)-3-(pyridin-4-yl)-6H-pyrimido [1,6-b]pyridazin-6-one

To a 25 mL round bottom flask, 2-chloro-5-(2,6-dichlorophenyl)-3-(pyridin-4-yl)-**6*H*-pyrimido[1,6-b]pyridazin-6-one** (20 mg, 0.032 mmol), 2,4-difluorophenol (4 mg,0.032 mmol), cesium carbonate (16 mg, 0.048 mmol) were added, and then N,N-dimethylformamide (5 mL) as solvent was added finally. The reaction solution was stirred at room temperature for 16 hours. Water (30 mL) was added to the reaction solution, and then extracted with ethyl acetate (30 mL x 1). The extracts were combined, washed with water (30 mL x 3) and saturated brine (30 mL x 1), dried over anhydrous sodium sulfate, filtered and concentrated. The crude product was purified by column chromatography to obtain a brown solid (3.3 mg) with a yield of 20%. LCMS: 497 [M+H] detection value; ¹H NMR (400 MHz, CDCl₃) δ 8.78 (d, *J* = 5.6 Hz, 2H), 8.62 (s, 1H), 7.51-7.49 (m, 4H), 7.40-7.35 (m, 1H), 7.27-7.24 (m, 1H), 7.12-6.99 (m, 3H).

### Embodiment 55 Synthesis of 5-(2,6-dichlorophenyl)-2-(2,4-difluorophenoxy)-3-(4-isopropylpiperazin-1-yl)-6H-pyrimido[1,6-b]pyridazin-6-one (I-60)

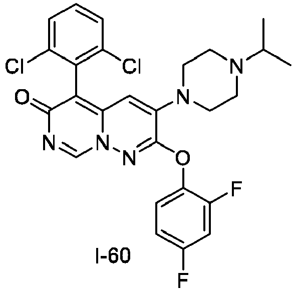

### Step A: Synthesis of 3,6-dichloro-4-(4-isopropylpiperazin-1-yl)pyridazine

To a 100 mL round bottom flask, 3,4,6-trichloropyridazine (1.2 g, 6.54 mmol), potassium carbonate (1.81 g, 13.08 mmol) were added, and then anhydrous N,N-dimethylformamide (20 mL) as solvent was added. The reaction solution was placed in an ice water bath and stirred for 5 minutes, and then 4-isopropylpiperidine (1.09 g, 8.5 mmol) was added. The reaction solution was stirred in an ice water bath, warmed to room temperature and stirred for 16 hours. The reaction solution was added with ethyl acetate (50 mL), washed with water (30 mL x 2) and saturated brine (30 mL x 1) sequentially, dried over anhydrous sodium sulfate, filtered and concentrated. The obtained crude product was purified by column chromatography to obtain a brown solid (1.74 g, 6.32 mmol) with a yield of 96%. LCMS: 275[M+H] detection value.

### Step B: Synthesis of 2-(6-chloro-5-(4-isopropylpiperazin-1-yl)pyridazin-3-yl)-2-(2,6-dichlorophenyl)acetonitrile

To a 100 mL round bottom flask, 2,6-dichlorophenylacetonitrile (1.17 g,6.28 mmol), anhydrous tetrahydrofuran (30 mL) as solvent were added sequentially, and then sodium hydride (60%, 1.05 g,26.17 mmol) was added. The reaction solution was stirred at room temperature for 10 minutes, and then 3,6-dichloro-4-(4-isopropylpiperazin-1-yl)pyridazine (1.44 g, 5.23 mmol) was added. The reaction solution was heated to reflux and stirred for 16 hours. The reaction solution was cooled to room temperature, added to ice saturated ammonium chloride solution dropwise, extracted with ethyl acetate (50 mL x 3). The extracts were combined, washed with water (50 mL x 2), saturated saline (50 mL x 1), dried over anhydrous sodium sulfate, filtered, and concentrated. The obtained crude product was purified by column chromatography to obtain brown solid (1.4 g, 3.29 mmol) with a yield of 63%. LCMS: 424 [M+H] detection value.

### Step C: Synthesis of 2-(6-chloro-5-(4-isopropylpiperazin-1-yl)pyridazin-3-yl)-2-(2,6-dichlorophenyl)acetamide

To a 100 mL round bottom flask, 2-(6-chloro-5-(4-isopropylpiperazin-1-yl) pyridazin-3-yl)-2-(2,6-dichlorophenyl)acetonitrile (0.8 g, 1.88 mmol), concentrated sulfuric acid (5 mL) were added. The reaction solution was heated to 50 °C and stirred for 2 hours. The reaction solution was cooled to room temperature, quenched with water, added with saturated sodium bicarbonate solution dropwise to adjust the pH to 5, then extracted with ethyl acetate (60 mL x 3). The extracts were combined, washed with water (50 mL x 2) and saturated brine (50 mL x 1), dried over anhydrous sodium sulfate, filtered and concentrated to obtain a brown solid (0.65 g, 1.47mmol) with a yield of 78%. LCMS: 442 [M+H] detection value. The obtained solid was used directly in the next step.

### Step D: Synthesis of 2-chloro-5-(2,6-dichlorophenyl)-3-(4-isopropylpiperazin-1-yl)-6H-pyrimido[1,6-b]pyridazin-6-one

To a 100 mL round bottom flask, 2-(6-chloro-5-(4-isopropylpiperazin-1-yl)pyridazin-3- yl)-2-(2,6-dichlorophenyl)acetamide (0.5 g,1.13 mmol), N,N-dimethylformamide dimethyl acetal (0.34 g,2.83 mmol) were added, and toluene (15 mL) as solvent was added finally. The reaction solution was heated to 50 °C and stirred for 16 hours, and then cooled to room temperature. The reaction solution was concentrated under reduced pressure, and the crude product was purified by column chromatography to obtain a brown solid (130 mg, 0.29 mmol) with a yield of 25%. LCMS: 452[M+H] detection value.

### Step E: Synthesis of 5-(2,6-dichlorophenyl)-2-(2,4-difluorophenoxy)-3-(4-isopropylpiperazin-1-yl)-6H-pyrimido[1,6-b]pyridazin-6-one

To a 100 mL round bottom flask, 2-chloro-5-(2,6-dichlorophenyl)-3-(4-isopropylpiperazin-1-yl)-6*H*-pyrimido[1,6-b]pyridazin-6-one (130 mg,0.29 mmol), 2,4-difluorophenol (45 mg,0.35 mmol), triethylamine (44 mg, 0.43 mmol), were added, and acetonitrile (10 mL) was added finally. The reaction solution was heated to 50 °C and stirred for 3 hours. The reaction solution was concentrated under reduced pressure, and the residue was dissolved in ethyl acetate (50 mL), washed with water (15 mL x 3) and saturated brine (15 mL x 1) sequentially, filtered and concentrated under reduced pressure. The crude product was purified by column chromatography to obtain a yellow solid (50 mg, 0.09 mmol) with a yield of 31%. LCMS: 546[M+H] detection value; ¹H NMR(400 MHz, CDCl₃) δ 8.42 (s, 1H), 7.48 (s, 1H), 7.46 (s, 1H), 7.24-7.26 (m, 2H), 7.10-6.99 (m, 2H), 5.90 (s, 1H), 3.31 (s, 4H), 2.67 (br, 5H), 1.07 (d, *J* = 6.0 Hz, 6H).

### Embodiment 56 Synthesis of 5-(2,6-dichlorophenyl)-2-(2,4-difluorophenoxy)-3-morpholinyl-6H-pyrimido[1,6-b]pyridazin-6-one (1-61)

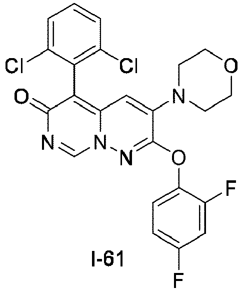

### Step A: Synthesis of 4-(3,6-dichloropyridazin-4-yl)morpholine

3,4,6-trichloropyridazine (1.5 g, 8.2 mmol), morpholine (1.06 g, 12.2 mmol) and potassium carbonate (2.26 g, 16.4 mmol) were added to N,N-dimethylformamide (15 mL). The reaction solution was stirred at room temperature for 16 hours. The reaction solution was diluted with water, extracted with ethyl acetate (80 mL x 3). The organic phases were combined, dried over anhydrous sodium sulfate and concentrated under reduced pressure to obtain a white solid (1.6 g, 6.8 mmol) with a yield of 84%. LCMS: 234.1[M+H] detection value.

### Step B: Synthesis of 2-(6-chloro-5-morpholinopyridazin-3-yl)-2-(2,6-dichlorophenyl)acetonitrile

2-(2,6-Dichlorophenyl)acetonitrile (1.24 g, 6.67 mmol) was added to 20 mL of tetrahydrofuran, and sodium hydrogen (474 mg, 11.85 mmol) was added at 0°C and reacted for 30 minutes, and then 2-(6-chloro-5-morpholinopyridazin-3-yl)-2-(2,6-dichlorophenyl) acetonitrile (1.2 g, 5.1 mmol) was added. The reaction solution was returned to room temperature and reacted for 24 hours. The reaction was quenched with water, extracted with ethyl acetate (80 mL x 3). The organic phases were combined, dried over anhydrous sodium sulfate, and was concentrated under reduced pressure to obtain a residue, which was purified by column chromatography to obtain a brown solid (1.8 g, 4.7 mmol) with a yield of 92%. LC-MS: 383.0 [M+H] detection value.

### Step C: Synthesis of 2-(6-chloro-5-morpholinopyridazin-3-yl)-2-(2,6-dichlorophenyl)acetamide

2-(6-Chloro-5-morpholinopyridazin-3-yl)-2-(2,6-dichlorophenyl)acetonitrile (1.8 g, 4.7 mmol) was added to 10 mL of concentrated sulfuric acid, and the reaction solution was heated to 100°C for 2 hours. The reaction solution was cooled to room temperature and poured into ice water, extracted with dichloromethane (80 mL x 3). The organic phases were combined, dried over anhydrous sodium sulfate, concentrated under reduced pressure to obtain a brown oil (1.7 g, 4.2 mmol) with a yield of 90%. LC-MS: 401.1 [M+H] detection value.

### Step D: Synthesis of 2-chloro-5-(2,6-dichlorophenyl)-3-morpholinyl-6H-pyrimido [1,6-b] pyridazin-6-one

2-(6-Chloro-5-morpholinopyridazin-3-yl)-2-(2,6-dichlorophenyl)acetamide (1.63 g, 4.06 mmol), trimethyl orthoformate (2.59 g, 24.4 mmol) and p-toluenesulfonic acid monohydrate (38 mg, 0.2 mmol) were added to 25 mL of toluene. The reaction solution was heated to 60°C for 16 hours. The reaction solution was concentrated under reduced pressure to obtain a residue, which was purified by column chromatography to obtain a brown solid (1.36 g, 3.30 mmol) with a yield of 81%. LC-MS: 411.1[M+H] detection value.

### Step E: Synthesis of 5-(2,6-dichlorophenyl)-2-(2,4-difluorophenoxy)-3-morpholinyl-6H-pyrimido[1,6-b]pyridazin-6-one

2-Chloro-5-(2,6-dichlorophenyl)-3-morpholinyl-6*H*-pyrimido[1,6-b]pyridazin-6-one (1.36 g, 3.30 mmol), 2,4-difluorophenol (516 mg, 3.97 mmol) and cesium carbonate (2.15 g, 6.62 mmol) were added to 15 mL of N,N-dimethylformamide. The reaction solution was stirred at room temperature for 16 hours. The reaction solution was diluted with water, extracted with ethyl acetate (100 mL x 3). The organic phases were combined, washed with brine, concentrated under reduced pressure to obtain a residue, which was purified by column chromatography to obtain a brown solid (800 mg, 1.58 mmol) with a yield of 48%. ¹H NMR (400 MHz, DMSO) δ 8.60 (s, 1H), 7.74-7.68 (m, 1H), 7.63-7.55 (m, 3H), 7.49 (dd, *J=* 8.8, 7.6 Hz, 1H), 7.30-7.22 (m, 1H), 5.85 (s, 1H), 3.72-3.67 (m, 4H), 3.28- 3.23 (m, 4H).

### Embodiment 57 Synthesis of 5-(2-chloro-6-fluorophenyl)-2-(2,4-difluorophenoxy)-6H-pyrimido[1,6-b]pyridazin-6-one (I-62)

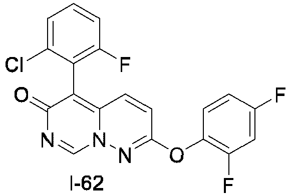

### Step A: Synthesis of 2-(2-chloro-6-fluorophenyl)-2-(6-chloropyridazin-3-yl) acetonitrile.

To a 100 mL reaction round bottom flask, 0.5 g of 2-chloro-6-fluorophenylacetonitrile, 0.57 g of 3,6-dichloropyridazine, 20 mL of tetrahydrofuran were added sequentially, and 0.45 g of potassium tert-butoxide anhydrous was added finally. The reaction solution was heated to reflux overnight, then cooled to 0 °C, quenched with saturated ammonium chloride solution, added with IN dilute hydrochloric acid to adjust the pH to 5, and extracted with ethyl acetate three times finally. The extracts were combined, washed with brine three times, dried over anhydrous sodium sulfate, filtered, and concentrated under reduced pressure to obtain a crude product. The obtained crude product was purified by column chromatography to obtain a brown solid (0.5 g, with a yield of 50%). LC-MS: 282.0 [M+H] detection value.

### Step B: Synthesis of 2-(2-chloro-6-fluorophenyl)-2-(6-chloropyridazin-3-yl) acetamide

To a 100-mL round-bottom flask, 0.5 g of 2-(2-chloro-6-fluorophenyl)-2-(6-chloropyridazin-3-yl)acetonitrile was added, and then 3 mL of concentrated sulfuric acid was added. The reaction solution was heated to 100 °C and stirred for 2 hours. The reaction solution was cooled to room temperature, quenched with ice water, and added with saturated sodium bicarbonate solution to adjust the pH to 6, and then extracted with ethyl acetate three times. The extracts were combined, washed with water once and with saturated brine once, partitioned, and dried over anhydrous sodium sulfate, filtered and concentrated to obtain 0.5 g of crude product. The crude product was used directly in the next step. LC-MS: 300.0 [M+H] detection value.

### Step C: Synthesis of 2-chloro-5-(2-chloro-6-fluorophenyl)-6H-pyrimido [1,6-b]pyridazin-6-one

To a 100 mL round bottom flask, 20 mL of toluene, 0.5 g of 2-(2-chloro-6-fluorophenyl)-2-(6-chloropyridazin-3-yl)acetamide, 0.95 g of trimethyl orthoformate were added, and 20 mg of p-toluenesulfonic acid as catalyst was added finally. The reaction solution was heated to reflux for two hours, and cooled to room temperature. The end of the reaction was monitored by TLC. The reaction was quenched with 30 mL of water, extracted with ethyl acetate three times. The extracts were combined, washed with saturated brine three times and partitioned. The obtained ethyl acetate phase was dried over anhydrous sodium sulfate, filtered and concentrated under reduced pressure to obtain a crude product. The obtained crude product was purified by column chromatography to obtain a yellow solid (0.32 g, 1.0 mmol) with a yield of 39.8%. LC-MS: 310.0 [M+H] detection value.

### Step D: Synthesis of 5-(2-chloro-6-fluorophenyl)-2-(2,4-difluorophenoxy)-6H-pyrimido [1,6-b] pyridazin-6-one

100 mg of 2-chloro-5-(2-chloro-6-fluorophenyl)-6*H*-pyrimido[1,6-b]pyridazin-6-one was dissolved in 10 mL of N,N-dimethylformamide, and anhydrous cesium carbonate (80 mg, 0.41 mmol) was added at room temperature, and then 2,4-difluorophenol (25 mg, 0.19 mmol) was added. The reaction was reacted at room temperature for 16 hours. The reaction was quenched with 25 mL of water, and then extracted with ethyl acetate (30 mL X 3). The obtained extracts were combined, washed with water three times and partitioned. The obtained ethyl acetate solution was dried over anhydrous sodium sulfate, filtered, and concentrated under reduced pressure to obtain a crude product. The obtained crude product was purified by column chromatography to obtain a yellow solid (20 mg, 0.05 mmol) with a yield of 30.7%. LCMS: 404.1[M+H] detection value; ¹H NMR (400 MHz, DMSO-d6) δ8.78 (s, 1H), 7.68-7.50 (m, 4H), 7.41-7.36 (m, 2H), 7.30-7.22 (m, 2H).

### Effect Embodiment 1 In vitro inhibition of the compounds on p38 kinase

The required 1 × reaction buffer for each kinase was prepared using 6.25× enzyme buffer. 10 mM of the storage solution of the test compound was taken and placed in a 96-well compound plate, and using DMSO for the compound at an initial concentration of 100×, which was then used as the first concentration and then carried out a 3-fold gradient dilution with DMSO for a total of 10 concentrations. After that, 1µL of gradient dilutions were added to 19µL of 1× reaction buffer to prepare 5x compound for later use. Then 2µL of 5× compound was transferred from the 96-well plate into a 384-well plate. To the compound-free control well was added 2 µL of the following liquid: 19µL of 1× reaction buffer with addition of 1µL of DMSO. To the Min control well was added 2µL of 250mM EDTA.

The kinase, substrate and ATP were formulated into 2.5× enzyme/substrate mixture and 2.5x ATP solution using 1× reaction buffer, respectively. 4µL of 2.5× enzyme/substrate mixture was added to a 384-well plate and incubated for 5 minutes at room temperature; 4µL of 2.5×ATP solution was added to 384-well plate, and the reaction was carried out at room temperature for 30 minutes.

1× of LANCE® Detection value Buffer was used to prepare a 2×Antibody for use, and the amount was 5µL per well, at the same time 10 mM EDTA was prepared for later use. After the enzyme reaction was carried out for 30 minutes, 5 µL 10 mM EDTA was added to the 384-well plate and the reaction was carried out at room temperature for 5 minutes. Afterwards, 5 µL LANCE® Ultra Europium-anti-phospho-eIF4E-binding protein 1 (Thr37/46) was added to the 384-well plate and the reaction was carried out at room temperature for one hour.

The 384-well plates were centrifuged at 2000 rpm for 2 minutes in a HERAEUS Multifuge X1R centrifuge; Data were measured on EnVisionTM, and the laser of 337nM wavelength was selected as excitation light to measure RFU_{665nM} and RFU_{620nM}, and RFU_{665nM}/RFU_{620nM} × 10000 was used as final data for analysis. Graphpad Prism 5.0 was used to perform curve fitting on the data and the corresponding IC₅₀ was calculated, which is shown in Table 1.

### Effect Embodiment 2:Inhibition of the compounds on inflammation in cells

Normal cultured 8th passage BV-2 cells were taken, and inoculated separately in 96-well plates at a certain cell density. The cell culture plates were cultured at 37°C and 5% CO₂ for 24 hours.

After inoculating the plate for 24 hours, the cell culture medium was removed, and then 100 µL of a 4-fold gradient dilution compound starting from 10,000 nM prepared in DMEM + 10% FBS, where High control and Low control contained 0.1% DMSO without compound. With 100µL per well, the cell culture plate was cultured at 37 °C with 5% CO₂ for one hour.

The compound-containing cultures were removed. 100 ng/mL of LPS was prepared with DMEM + 10% FBS culture medium, and then the compound was diluted in a 4-fold gradient from 10,000 nM with LPS-containing culture medium. In low control, the compound was replaced with 0.1% DMSO. In High control, 0.1% DMSO was prepared with culture medium without LPS. 100 µL per well, and the cell culture plates were cultured at 37 °C with 5% CO₂ for 4 hours.

After 4 hours of LPS stimulation, 110 µL of the culture solution was aspirated, and centrifuged at 2000 rpm for 2 minutes. A further 90µl of supernatant was aspirated and immediately put into -20 °C for freezing.

ELISA kits were used to detect the expression of inflammatory factors such as IL-1β, TNFα, etc. The OD450 signal values were measured by microplate reader. The in vitro inhibitory activity of the compounds was calculated according to the following formula: Inhibition rate: Inhibition rate (%) = (signal value of control - signal value of administration) / (high signal value of control - low signal value of control) × 100%. And according to the inhibition rate of each concentration, Graphpad Prism 5.0 was used to curve fit the fluorescence signal values at each concentration and calculate the corresponding IC₅₀, which is shown in Table 1.

**Table 1**

| Compound | Kinase in vitro ICso(nM) | | | BV-2 | |
|---|---|---|---|---|---|
| | p38α | p38β | p38γ | Rf IC₅₀(nM) | Max Inh. (%) |
| I-1 | >3333 | >3333 | >3333 | -- | -- |
| I-2 | 2686.0 | >3333 | >3333 | -- | -- |
| I-3 | >3333 | >3333 | >3333 | -- | -- |
| I-4 | 9.87 | 47.37 | >3333 | 6.473 | 100.00 |
| I-5 | 49.34 | 389.0 | >3333 | 94.33 | 44.04 |
| I-6 | 13.57 | 109.3 | >3333 | 16.15 | 44.66 |
| I-7 | 1676.0 | >3333 | >3333 | -- | -- |
| I-8 | >3333 | >3333 | >3333 | -- | -- |
| I-9 | 111.6 | 300.6 | >3333 | -- | -- |
| I-10 | 60.64 | 433.5 | >3333 | 75.63 | -60 |
| I-11 | 137.2 | 1220.0 | >3333 | -- | -- |
| I-12 | 32.82 | 301.7 | >3333 | 175.9 | 49.46 |
| I-13 | >3333 | >3333 | >3333 | -- | -- |
| I-14 | 1351.0 | >3333 | >3333 | -- | -- |
| I-15 | 41.26 | 262.6 | >3333 | 132.4 | 46.03 |
| I-16 | 25.88 | 109.0 | >3333 | 6.192 | 44.16 |
| I-17 | 34.76 | 315.6 | >3333 | 95.78 | -60 |
| I-18 | 14.78 | 97.06 | >3333 | 8.584 | 50.57 |
| I-19 | 22.41 | 390.7 | >3333 | 53.83 | 61.74 |
| I-20 | 17.62 | 95.98 | >3333 | 23.27 | -60 |
| I-21 | 27.68 | 226.0 | >3333 | 19.18 | 51.3 |
| I-22 | 31.31 | 149.9 | >3333 | 18.33 | 53.57 |
| I-23 | 46.90 | 243.1 | >3333 | 17.19 | -50 |
| I-24 | 1979 | >3333 | >3333 | -- | -- |
| I-25 | 150.9 | 1430.0 | >3333 | -- | -- |
| I-26 | >3333 | >3333 | >3333 | -- | -- |
| I-27 | 11.66 | 108.8 | >3333 | 18.83 | 41.92 |
| I-28 | 38.77 | >3333 | >3333 | -- | -- |
| I-29 | 34.10 | 405.9 | >3333 | 678.2 | -50 |
| I-33 | 23.99 | 470.0 | >3333 | -- | -- |
| I-34 | 49.84 | 388.5 | >3333 | -- | -- |
| I-37 | 503.5 | 2835 | >3333 | -- | -- |
| I-38 | 413.9 | 2161 | >3333 | -- | -- |
| I-39 | 35.56 | 602.0 | >3333 | 296.4 | 56.63 |
| I-40 | 22.98 | 110.9 | >3333 | 41.51 | 47.74 |
| I-41 | >3333 | >3333 | >3333 | -- | -- |
| I-42 | 227.7 | 685.9 | >3333 | -- | -- |
| I-43 | 10.69 | 95.13 | >3333 | 21.54 | 50.88 |
| I-44 | 31.01 | 635.40 | >3333 | -- | -- |
| I-45 | 35.40 | 643.0 | >3333 | -- | -- |
| I-46 | 19.69 | 402.7 | >3333 | -- | -- |
| I-49 | 14.06 | 212.6 | >3333 | -- | -- |
| I-50 | 34.23 | 1090.0 | >3333 | -- | -- |
| I-51 | 17.54 | 255.5 | >3333 | -- | -- |
| I-52 | 54.20 | 947.4 | >3333 | -- | -- |
| I-55 | 17.72 | 117.30 | >3333 | -- | -- |
| I-56 | 19.60 | 478.6 | >3333 | - | -- |
| I-57 | 22.68 | 613.7 | >3333 | -- | -- |
| I-58 | 137.2 | >3333 | >3333 | -- | -- |
| I-59 | 81.32 | 1220.0 | >3333 | -- | -- |
| I-60 | 14.77 | 111.0 | >3333 | 12.36 | 34.45 |
| I-61 | 11.73 | 32.68 | >3333 | -- | -- |
| I-62 | 12.07 | 100.70 | >3333 | 14.17 | 48.62 |
| 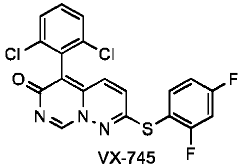 Positive compound | 14.76 | 128.50 | >3333 | 4.258 | 99.73 |

| | | | | | |
|---|---|---|---|---|---|
| Note: "--" in Table 1 indicates that this test has not been carried out. | | | | | |

From the comparison between the positive compound in the above table and compound 1-4 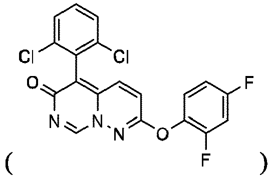 of the present application, it can be seen that compound 1-4 showed superior inhibitory activity against both p38α kinase and p38β kinase, compared to the positive compound. It can be seen that the inhibitory activity of this class of compounds against p38α kinase and p38β kinase has been significantly improved by replacing the heteroatom S with O.

In addition, in CN108349964A (Table 7 on page 74 of the specification), compound 12 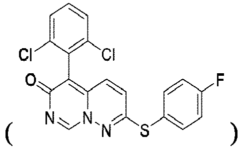 has an IC₅₀ grade of "++" (indicating 0.1-1.0 µM) for the inhibitory activity of p38 kinase, while IC₅₀ for p38α kinase of the compound 19 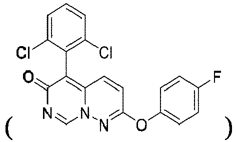 of the present application is only 22.41 nM. It is evident that the inhibitory activity of the compound of the present application against p38α kinase is significantly better than that of the comparative compound.

Thus, it is evident that the compounds of the present application have superior inhibitory activity against p38α kinase and/or p38β kinase than the positive compounds.

### Effect Embodiment 3 Pharmacokinetic properties of compounds in rats and mice

Ten male SD rats, weighing 180-220g (provided by Shanghai Xipuerbikai Laboratory Animal Co., Ltd, Certificate No. : 20130016008116). The rats were fasted for feed for 12-16 hours before the experiment but water was allowed.

Four rats were divided into two groups, which were administered intravenously with 3 mg/kg (which were dissolved in 0.9% saline: PEG400: NMP = 6:2:2 to obtain a clear solution with a concentration of 1 mg/mL, and administration volume: 3mL/kg) of compounds 1-4 (final product of Embodiment 4), I-19 (final product of Embodiment 19), I-43 (final product of Embodiment 43) and the positive compound VX-745 respectively. Blood samples were collected from the fundus vein of rats before and at 2, 5, 15, 30, 60, 90, 120, 240, 360, 480, 600 and 1440 minutes after administration, respectively.

Six rats were divided into two groups, which were administrated by gavage with 6 mg/kg (0.5% CMC-Na was added and grounded into a homogeneous suspension with a concentration of 0.75 mg/mL and administration volume: 8 mL/kg) of compounds 1-4, 1-19, 1-43 and positive compound VX-745 respectively. Blood samples were collected from the fundus vein of rats before and at 5, 15, 30, 60, 90, 120, 240, 360, 480, 600, 1440 minutes after administration respectively.

Forty-eight male ICR mice, weighing 18-22 g (provided by Shanghai Xipuerbikai Laboratory Animal Co., Ltd, Certificate No. : 20130016008116). The mice were for feed for 12-16 hours before the experiment but water was allowed.

Eighteen mice were divided into two groups, which were intravenously administrated with 3 mg/kg (which was dissolved in 0.9% saline: PEG400: NMP = 8:1:1 to obtain a clear solution with a concentration of 0.3 mg/mL, and administration volume: 10 mL/kg) of compounds 1-4, 1-19, 1-43, 1-46 (final product of Embodiment 46), 1-50 (final product of Embodiment 50), 1-62 (final product of Embodiment 62) and positive compound VX-745 respectively. Blood samples were collected from the fundus vein of mice before and at 2, 5, 15, 30, 60, 90, 120, 240, 480, 600, and 1440 minutes of the administration, respectively.

Thirty mice were divided into two groups, which were administrated by gavage with 10 mg/kg (0.5% CMC-Na was added and grounded into a homogeneous suspension with a concentration of 0.5 mg/mL, and administration volume: 20 mL/kg) of compounds I-4, I-19, 1-43, 1-46, 1-50, 1-62 and the positive compound VX-745 respectively. Blood samples were collected from the fundus vein of mice before and at 5, 15, 30, 60, 90, 120, 240, 480 and 1440 minutes of administration, respectively.

2-3 time points were taken for each mouse. The mice were sacrificed at 5, 15, 30, 60 and 1440 minutes after the administration (3 mice per time point), and the brains were taken, rinsed with saline, dried on filter paper and weighed.

Blood samples were centrifuged at 8000 rpm for 5 minutes, and supernatant plasma was taken. To 50 µL of plasma samples was added 300 µL of acetonitrile containing internal standard (propranolol, 25 ng/mL) to precipitate the protein. After vortexing for 10 minutes and centrifuging at 6000g and 4°C for 10 minutes, the supernatant was taken and placed in a 96-well plate for determination of drug concentration in plasma by UPLC/MS-MS.

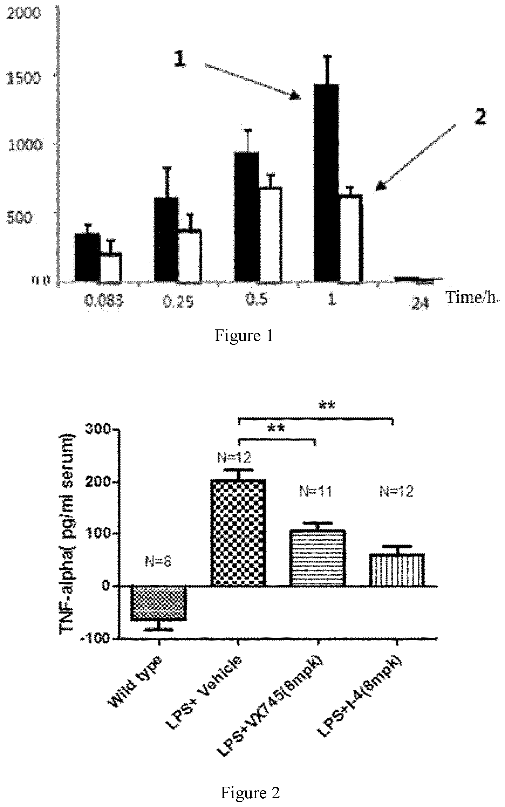

Brain tissues were weighed, homogenized in saline (1:4, w/v). 50 µL of homogenized samples were taken, added with 300 µL of acetonitrile containing internal standard (propranolol, 25 ng/mL) to precipitate protein. After vortexing for 10 minutes and centrifuging at 6000 g for 10 minutes, 80 µL of supernatant was taken and placed in a 96-well plate for determination of drug concentration in tissue by UPLC/MS-MS, as shown in Table 2. The histogram of the drug-time-concentration distribution (AUC(0-t)) in mice brain is shown in Figure 1.

| | Dose (po) | Cmax(po) | Tmax (po) | AUClast (po) | Thalf (iv) | MRT (po) | Clearance(iv) | Vss (iv) | BA |
|---|---|---|---|---|---|---|---|---|---|
| | mg/kg | ng/mL | h | (h)*(ng/mL) | h | h | L/kg*h | L/kg | % |
| Compound 1-4 (rat) | 6 | 993.7 | 4.5 | 8857.1 | 3.4 | 7.5 | 0.5 | 2.1 | 68.9 |
| VX-745 (rat) | 6 | 476.5 | 0.8 | 1644.5 | 5.2 | 4.3 | 1.5 | 4.5 | 41.2 |
| Compound 1-4 (mice) | 10 | 3005 | 1.5 | 8944 | 2.5 | 4.4 | 0.4 | 1.2 | 36.8 |
| VX-745 (mice) | 10 | 749.6 | 0.8 | 1844 | 3.9 | 3.2 | 1.1 | 2.3 | 20.2 |
| Compound I-19(rat) | 6 | 1091 | 3 | 7172 | 2.81 | 5.73 | / | / | 87.87 |
| Compound 1-19 (mice) | 10 | 1815 | 1.33 | 11457 | 3.73 | 5.55 | / | / | 158.7 |
| Compound 1-43 (rat) | 6 | 601 | 4 | 2994 | 3.7 | 4.7 | / | / | 123.3 |
| Compound 1-43 (mice) | 10 | 1282 | 2 | 6540 | 2.97 | 4.34 | / | / | 89.17 |
| Compound I-46(mi ce) | 10 | 891 | 2 | 4016 | 3.96 | 4.86 | / | / | 119.3 |
| Compound I-50(mi ce) | 10 | 927.64 | 0.83 | 3107 | 1.93 | 3.05 | / | / | 84.9 |
| Compound I-62(mice) | 10 | 1605 | 1 | 4522 | 3.32 | 3.18 | / | / | 103.4 |

| | | | | | | | | | |
|---|---|---|---|---|---|---|---|---|---|
| Note:"/" in Table 2 indicates that the item was not tested. | | | | | | | | | |

As shown in Table 2, when the administration dose was 6 mg/kg, the AUC of compound 1-4 in rats (5-1440 minutes) was 8857.1 (h)*(ng/mL), which was 5.4 times of that of the positive compound VX-45; when the administration dose was 10 mg/kg, the AUC of compound 1-4 in mice (5-1440 minutes) was 8944 (h)*(ng/mL), which was 4.8 times higher of that of the positive compound VX-45. It can be seen that the absorption of compound 1-4 in both rats and mice is significantly higher than that of the positive compound VX-45 at the same dose.

When the administration dose was 6 mg/kg, the clearance rate of compound 1-4 in rats was 0.5 L/kg*h, which was 1/3 of that of the positive compound VX-45; when the administration dose was 10 mg/kg, the clearance rate of compound 1-4 in mice was 0.4 L/kg*h, which was 4/11 of that of the positive compound VX-45. It can be seen that the clearance rate of compound 1-4 in both rats and mice is significantly lower than that of the positive compound VX-45 at the same dose.

As shown in Table 2, when the administration dose was 6 mg/kg, the absorption of compound 1-19 in rats was significantly higher than that of the positive compound VX-745 at the same dose; the absorption of compound 1-43 was better than that of the positive compound VX-745. When the administration dose was 10 mg/kg, the absorption of compound 1-19 in mice was significantly higher than that of the positive compound VX-745 at the same dose; the absorption of compounds 1-43, 1-46, 1-50 and 1-62 were all better than that of the positive compound VX-745.

As shown in Figure 1, the drug concentration of compound 1-4 in mice brain was significantly higher than that of the positive compound VX-45 at different time points, especially at the 1 h time point, the concentration of compound 1-4 in mouse brain was more than one time of that of the positive compound VX-45.

It can be seen that the bioavailability of this class of compounds is significantly improved by replacing the heteroatom S with O.

### Effect Embodiment 4 Pharmacodynamic study of compounds in a lipopolysaccharide (LPS)-induced inflammation model in mice

### Experimental materials:

Male SPF-grade C57BL/6J mice, 6-8 weeks old, provided by Shanghai Ling Chang Biotechnology Co., Ltd. Animals were housed in clean-grade plastic cages (light and dark cycle 12 / 12 hours, temperature 22 ± 2°C, humidity 50 ± 10%, pellet feed, free drinking water).

See Table 3 for reagent information.

**Table 3**

| Name | Manufacturer | Item No. | Batch No. | Storage Condition |
|---|---|---|---|---|
| Lipopolysaccharide (LPS) | SIGMA-ALDRICH | L2630 | 028M4021V | RT |
| CMC-Na | Aladdin | C104984-500g | L14310109 | RT |

### Experimental method:

**Table 4**

| No. | Group | Number of Animals | Dosage (mg/kg) | Treatment |
|---|---|---|---|---|
| 1 | Normal control | 6 | N/A | Tested compound, bid, Interval 4h |
| 2 | Vehicle | 12 | Blank solvent | |
| 3 | VX-745_8mpk | 11 | 8 | |
| 4 | I-4_8mpk | 12 | 8 | |

VX-745_8mpk group i.e. the reagent implemented in this group is the positive compound VX-745; I-4_8mpk group i.e. the reagent implemented in this group is the compound 1-4.

### Experimental steps:

1. Mice were weighed and administered according to an administration volume of 10 mL/kg, and oral administration by gavage was given for the first time. Each mouse was administrated at an interval of 3 minutes.
2. One hour after the first administration, LPS_5mpk was administered intraperitoneally at an administration volume of 10 mL/kg.
3. 4 hours after the first administration, a second oral administration by gavage was given according to the administration volume of 10 mL/kg.
4. 6 hours after LPS intraperitoneal injection, mice were sacrificed, serum and cerebral cortex and hippocampus were sampled. After weighing the wet weight and they were put into liquid nitrogen for quick-freezing and storage.

**Experimental results:** The expression of inflammatory factors such as TNF-α in serum were tested by ELISA kit, and the specific data are shown in Table 5 and Figure 2 below.

**Table 5**

| Group | Treatment | Animal NO: | TNF-α conc. Pg/mL |
|---|---|---|---|
| Group 1 | Normal control | 1-1 | -26.3 |
| | | 1-2 | -34.3 |
| | | 1-3 | -41.6 |
| | | 1-4 | -39.9 |
| | | 1-5 | -123.1 |
| | | 1-6 | -122.8 |
| Group 2 | Vehicle | 2-1 | 196.0 |
| | | 2-2 | 198.8 |
| | | 2-3 | 271.9 |
| | | 2-4 | 181.1 |
| | | 2-5 | 155.2 |
| | | 2-6 | 222.7 |
| | | 2-7 | 369.2 |
| | | 2-8 | 241.8 |
| | | 2-9 | 152.6 |
| | | 2-10 | 79.7 |
| | | 2-11 | 192.9 |
| | | 2-12 | 170.2 |
| Group 3 | VX-745_8mpk | 3-1 | 142.0 |
| | | 3-2 | 151.7 |
| | | 3-3 | 105.5 |
| | | 3-4 | 155.5 |
| | | 3-5 | 55.4 |
| | | 3-6 | 105.7 |
| | | 3-7 | 54.3 |
| | | 3-8 | 148.1 |
| | | 3-9 | 71.2 |
| | | 3-11 | 24.0 |
| | | 3-12 | 155.2 |
| Group 4 | I-4_8mpk | 4-1 | 11.9 |
| | | 4-2 | 127.8 |
| | | 4-3 | -39.4 |
| | | 4-4 | 107.7 |
| | | 4-5 | 92.4 |
| | | 4-6 | 17.4 |
| | | 4-7 | 97.1 |
| | | 4-8 | -37.7 |
| | | 4-9 | 86.6 |
| | | 4-10 | 58.6 |
| | | 4-11 | 89.4 |
| | | 4-12 | 116.9 |

As shown in Table 5, the LPS-induced inflammation model group in mice (G2) was able to significantly increase the levels of inflammatory factor TNF-α in plasma, after 6 hours of intraperitoneal injection of lipopolysaccharide (P<0.01) compared with the normal control group (G1). Compared with the mouse inflammation model group (G2), both the VX-745_8mpk (G3) and I-4_8mpk (G4) groups significantly reduced the levels of TNF-α (P<0.01) in plasma, and the I-4_8mpk group had a better anti-inflammatory effect than the VX-745_8mpk group.

It can be seen that the effect of this class of compounds is significantly improved by replacing the heteroatom S with O.

Although the above describes specific embodiments of the present disclosure, it should be understood by those skilled in the art that these are merely illustrative Embodiments and that a variety of changes or modifications can be made to these embodiments without departing from the principles and substance of the present disclosure. Therefore, the scope of protection of the present disclosure is limited by the appended claims.

## Claims

1. A nitrogen-containing heterocyclic compound represented by formula I, a pharmaceutically acceptable salt thereof, a hydrate thereof, a solvate thereof, a polymorph thereof, a metabolite thereof, a stereoisomer thereof, a tautomer thereof, or a prodrug thereof; 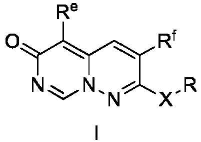
wherein, X is O, N or C₁₋₁₀ alkyleneoxy;
R is C₆₋₃₀ aryl, C₆₋₃₀ aryl substituted by R¹, C₁₋₃₀ heteroaryl, C₁₋₃₀ heteroaryl substituted by R², C₁₋₁₀ alkyl, C₁₋₁₀ alkyl substituted by R³, C₃₋₁₀ cycloalkyl, C₃₋₁₀ cycloalkyl substituted by R⁴, C₁₋₁₀ heterocycloalkyl, C₁₋₁₀ heterocycloalkyl substituted by R⁵, C₂₋₁₀ alkenyl, C₂₋₁₀ alkenyl substituted by R⁶, C₂₋₁₀ alkynyl, C₂₋₁₀ alkynyl substituted by R⁷, C₃₋₁₀ cycloalkenyl or C₃₋₁₀ cycloalkenyl substituted by R⁸;
R¹, R², R³, R⁴, R⁵, R⁶, R⁷ and R⁸ are independently one or more of halogen, C₁₋₁₀ alkyl, C₁₋₁₀ alkoxy, 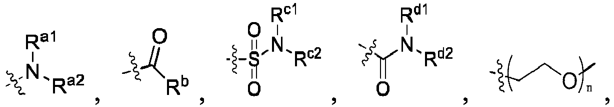 C₆₋₃₀ aryl, C₁₋₆ heterocycloalkyl, hydroxyl and cyano;
n is 1, 2 or 3;
R^{a1}, R^{a2}, R^{b}, R^{c1}, R^{c2}, R^{d1} and R^{d2} are independently H, C₁₋₆ alkyl, C₁₋₆ alkoxy, C₂₋₆ alkenyl, C₂₋₆ alkynyl, C₆₋₃₀ aryl or C₆₋₃₀ aryl substituted by R^{a-1};
R^{a-1} is one or more of C₁₋₃ alkyl, C₁₋₃ alkoxy, halogen, cyano, nitro, amino and hydroxyl;
R^{e} is C₆₋₃₀ aryl, C₆₋₃₀ aryl substituted by R^{e-1} or C₃₋₁₀ cycloalkenyl;
R^{e-1} is independently one or more of halogen, C₁₋₁₀ alkyl, C₁₋₁₀ alkoxy, 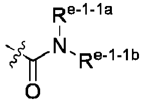 and 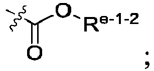
R^{e-1-1a}, R^{e-1-1b} and R^{e-1-2} are independently H or C₁₋₆ alkyl;
R^{f} is H, C₁₋₃₀ heteroaryl, C₁₋₃₀ heteroaryl substituted by R^{f-1}, C₁₋₁₀ heterocycloalkyl or C₁₋₁₀ heterocycloalkyl substituted by R^{f-2};
R^{f-2} is C₁₋₆ alkyl;
in the C₁₋₃₀ heteroaryl, the C₁₋₃₀ heteroaryl in the C₁₋₃₀ heteroaryl substituted by R², the C₁₋₁₀ heterocycloalkyl, the C₁₋₁₀ heterocycloalkyl in the C₁₋₁₀ heterocycloalkyl substituted by R⁴, the C₁₋₃₀ heteroaryl in the C₁₋₃₀ heteroaryl substituted by R^{f-1}, the C₁₋₁₀ heterocycloalkyl in the C₁₋₁₀ heterocycloalkyl substituted by R^{f-2}, and the C₁₋₆ heterocycloalkyl in "R¹, R², R³, R⁴, R⁵, R⁶, and R⁷", the number of heteroatom is independently 1-4, and the heteroatom is independently one or more of O, S and N.

2. The nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in claim 1, wherein, when X is C₁₋₁₀ alkyleneoxy, then the C₁₋₁₀ alkyleneoxy is C₁₋₆ alkyleneoxy, preferably C₁₋₃ alkyleneoxy, further can be 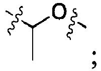 and/or, when R, R¹, R², R³, R⁴, R⁵, R⁶, R⁷, R⁸, R^{a1}, R^{a2}, R^{b}, R^{c1}, R^{c2}, R^{d1}, R^{d2} or R^{e} is C₆₋₃₀ aryl, then the C₆₋₃₀ aryl is C₆₋₁₈ aryl, and can be C₆₋₁₄ aryl;
and/or, when R is C₆₋₃₀ aryl substituted by R¹, then the C₆₋₃₀ aryl is C₆₋₁₈ aryl, and can be C₆₋₁₄ aryl, further preferably phenyl;
and/or, when R is a C₆₋₃₀ aryl substituted by R¹, then the number of R¹ is 1, 2 or 3, can be 2 or 3, and can also be 2; when the number of R¹ is more than one, R¹ can be the same or different, preferably the same;
and/or, when R is C₆₋₃₀ aryl substituted by R¹, then the substitution site of R¹ is located at one or more of the ortho position C, meta position C or para position C relative to the C connected to X, and can also be at least one located at the ortho position C relative to the C connected to X, further can be located at the ortho position C relative to the C connected to X, "the ortho position C relative to the C connected to X and the ortho position C relative to the C connected to X", " the ortho position C relative to the C connected to X and the para position C relative to the C connected to X", or "the ortho position C relative to the C connected to X, the ortho position C relative to the C connected to X and the para position C relative to the C connected to X ", and further can be located at "the ortho position C relative to the C connected to X and the para position C relative to the C connected to X";
and/or, when R is C₁₋₃₀ heteroaryl or C₁₋₃₀ heteroaryl substituted by R², then the heteroatom in the C₁₋₃₀ heteroaryl is N, and the number thereof is 1;
and/or, when R is C₁₋₃₀ heteroaryl or C₁₋₃₀ heteroaryl substituted by R², then the C₁₋₃₀ heteroaryl can be C₁₋₁₈ heteroaryl, and can also be C₁₋₁₄ heteroaryl, further can be pyridyl;
and/or, when R is C₁₋₃₀ heteroaryl substituted by R², then the number of the R² can be 1; and/or, when R is C₁₋₃₀ heteroaryl substituted by R², then the substitution site of R² can be located at the meta position C relative to the C connected to X;
and/or, when R, R¹, R², R³, R⁴, R⁵, R⁶, R⁷, R⁸ or R^{e-1} is C₁₋₁₀ alkyl, then the C₁₋₁₀ alkyl is C₁₋₆ alkyl, and can be C₁₋₃ alkyl, further can be methyl or ethyl;
and/or, when R is C₁₋₁₀ alkyl substituted by R³, then the number of the R³ is 2 or 3; when the number of R³ is more than one, then the R³ can be the same or different, preferably the same;
and/or, when R is C₃₋₁₀ cycloalkyl or C₃₋₁₀ cycloalkyl substituted by R⁴, then the C₃₋₁₀ cycloalkyl is C₃₋₁₀ saturated monocycloalkyl or C₅₋₁₀ bridged ring saturated alkyl, preferably C₃₋₆ monocyclo saturated alkyl or C₅₋₈ saturated bridged ring alkyl;
and/or, when R is C₃₋₁₀ cycloalkyl substituted by R⁴, then the number of the R⁴ is 1 or 2; when the number of R⁴ is more than one, then the R⁴ can be the same or different, preferably the same;
and/or, when R is C₁₋₁₀ heterocycloalkyl or C₁₋₁₀ heterocycloalkyl substituted by R⁵, then the heteroatom in the C₁₋₁₀ heterocycloalkyl is O, and the number thereof is 1;
and/or, when R is C₁₋₁₀ heterocycloalkyl or C₁₋₁₀ heterocycloalkyl substituted by R⁵, then the C₁₋₁₀ heterocycloalkyl is C₂₋₆ heterocycloalkyl, further can be heterocyclopentane or heterocyclohexane;
and/or, when R is C₂₋₁₀ alkenyl or C₂₋₁₀ alkenyl substituted by R⁶, then the C₂₋₁₀ alkenyl is C₂₋₆ alkenyl, and can be C₂₋₃ alkenyl, further can be 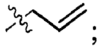 and/or, when R is a C₃₋₁₀ cycloalkenyl or C₃₋₁₀ cycloalkenyl substituted by R⁸, then the C₃₋₁₀ cycloalkenyl can be C₃₋₆ cycloalkenyl, further can be cyclopentenyl or cyclohexenyl;
and/or, when R is C₃₋₁₀ cycloalkenyl substituted by R⁸, then the number of the R⁸ can be 1;
and/or, when R is C₃₋₁₀ cycloalkenyl substituted by R⁸, then the substitution site of the R⁸ can be located at the ortho position C relative to the C connected to X;
and/or, when R¹, R², R³, R⁴, R⁵, R⁶, R⁷, R⁸, R^{a-1} or R^{e-1} is halogen, then the halogen is F, Cl, Br or I, and can be F or Cl;
and/or, when R¹, R², R³, R⁴, R⁵, R⁶, R⁷, R⁸, or R^{e-1} is C₁₋₁₀ alkoxy, then the C₁₋₁₀ alkoxy is C₁₋₃ alkoxy, further can be methoxy, ethoxy, n-propoxy or isopropoxy benzene;
and/or, when R^{e} is C₆₋₃₀ aryl substituted by R^{e-1}, then the C₆₋₃₀ aryl is C₆₋₁₈ aryl, and can be C₆₋₁₄ aryl, further can be phenyl;
and/or, when R^{e} is C₆₋₃₀ aryl substituted by R^{e-1}, then the number of the R^{e-1} is 1, 2 or 3;
and/or, when R^{e} is C6-30 aryl substituted by R^{e-1}, then the substitution site of the R^{e-1} is located at one or more of the ortho position C, meta position C or para position C relative to C connected to the connection site, and can at least one of them located at the ortho position C relative to C connected to the connection site, and further can "located at the ortho position C relative to C connected to the connection site and the ortho position C relative to C connected to the connection site", "located at the ortho position C relative to C connected to the connection site and the meta position C relative to C connected to the connection site", or "located at the ortho position C relative to C connected to the connection sit, the meta position C relative to C connected to the connection site and the para position C relative to C connected to the connection sit", and further can "located at the ortho position C relative to C connected to the connection site and the ortho position C relative to C connected to the connection site ";
and/or, when R^{e} is C₃₋₁₀ cycloalkenyl, then the C₃₋₁₀ cycloalkenyl is C₃₋₆ cycloalkenyl, further can be cyclohexenyl;
and/or, when R^{e-1-1a}, R^{e-1-1b} or R^{e-1-2} is C₁₋₆ alkyl, then the C₁₋₆ alkyl is C₁₋₃ alkyl, further can be methyl or ethyl;
and/or, when R^{f} is C₁₋₃₀ heteroaryl, then the heteroatom in the C₁₋₃₀ heteroaryl is N and the number thereof is 1;
and/or, when R^{f} is C₁₋₃₀ heteroaryl, then the C₁₋₃₀ heteroaryl is C₁₋₁₄ heteroaryl, further can be pyridyl;
and/or, when R^{f} is C₁₋₁₀ heterocycloalkyl or C₁₋₁₀ heterocycloalkyl substituted by R^{f-2}, then the heteroatom in the C₁₋₁₀ heterocycloalkyl is N and/or O, and the number thereof is 2, when the number of heteroatom is 2, then the heteroatom can be the same or different;
and/or, when R^{f} is C₁₋₁₀ heterocycloalkyl, then the C₁₋₁₀ heterocycloalkyl is C₂₋₆ heterocycloalkyl, further can be heterocyclohexane, and further can be 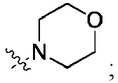 and/or, when R^{f} is C₁₋₁₀ heterocycloalkyl substituted by R^{f-2}, then the substitution position of R^{f-2} is on the heteroatom, and the number thereof is 1;
and/or, when R^{f-2} is C₁₋₆ alkyl, then the C₁₋₆ alkyl is C₁₋₃ alkyl, and further can be isopropyl;
and/or, the cycloalkyl is saturated monocyclic cycloalkyl or saturated bridged cycloalkyl;
and/or, the heterocycloalkyl is saturated monocyclic heterocycloalkyl.

3. The nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in claim 2, wherein, when R is C₆₋₃₀ aryl substituted by R¹, then the C₆₋₃₀ aryl substituted by R¹ is 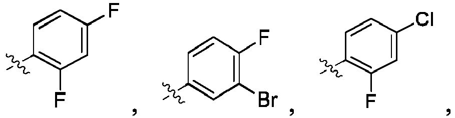 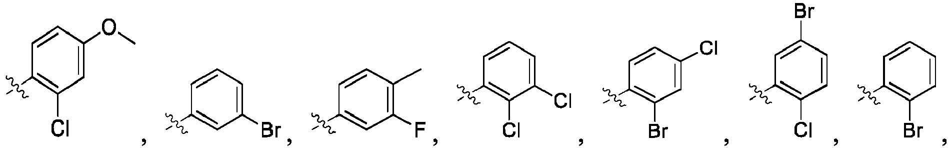 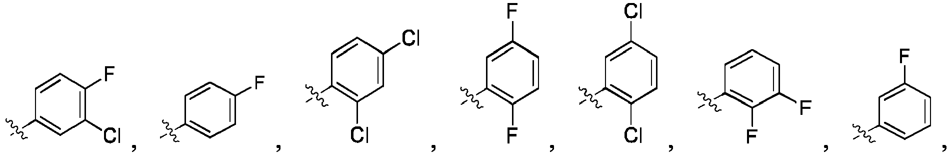 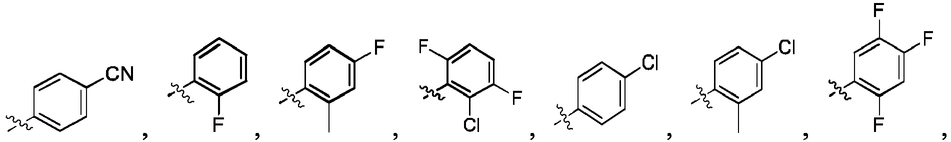 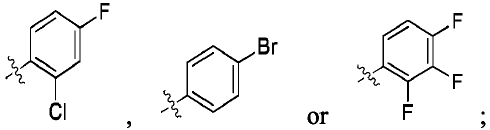 preferably 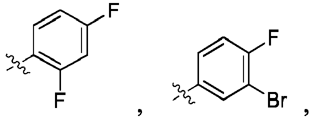 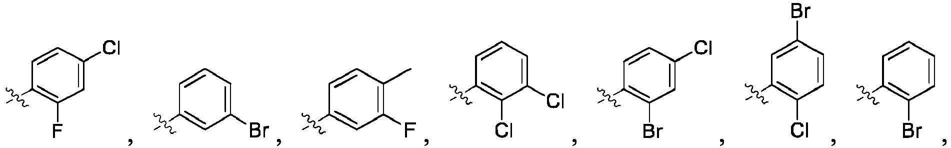 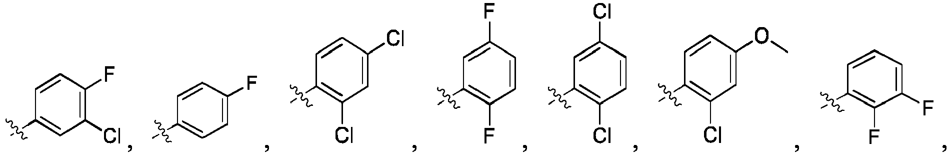 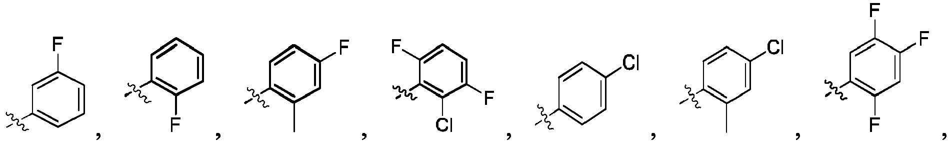 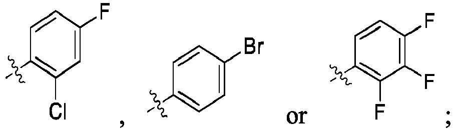 more preferably 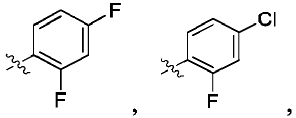 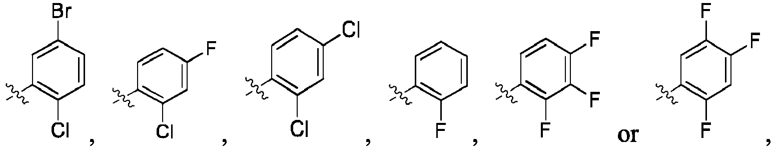 most preferably 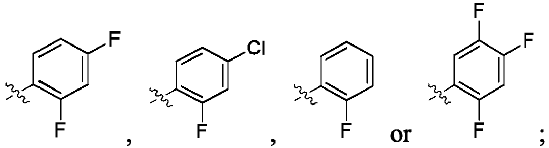 and/or, R is C₁₋₁₀ alkyl substituted by R³, then the C₁₋₁₀ alkyl substituted by R³ is 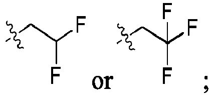 and/or, R is C₃₋₁₀ cycloalkyl substituted by R⁴, then the C₃₋₁₀ cycloalkyl substituted by R⁴ 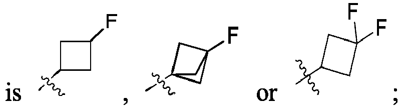 and/or, R is the C₃₋₁₀ cycloalkenyl substituted by R⁸, then the C₃₋₁₀ cycloalkenyl substituted by R⁸ is 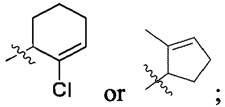 and/or, when R^{e} is C₆₋₃₀ aryl substituted by R^{e-1}, then the C₆₋₃₀ aryl substituted by R^{e-1} is 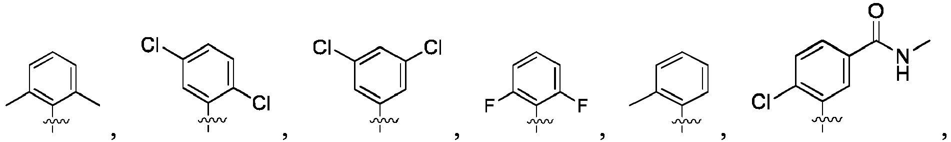 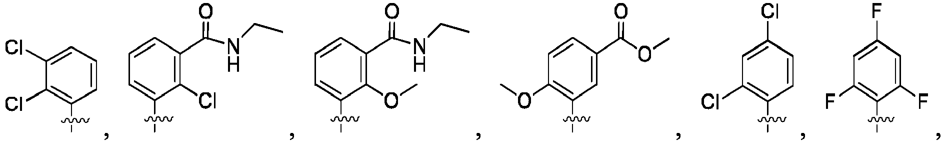 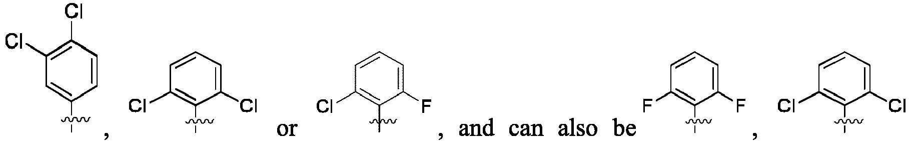 or 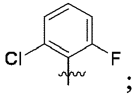 and/or, when R^{f} is C₁₋₁₀ heterocycloalkyl substituted by R^{f-2}, then the C₁₋₁₀ heterocycloalkyl substituted by R^{f-2} is

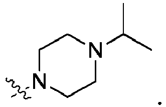

4. The nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in claim 1, wherein, in the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof,
X is O, N or C₁₋₁₀ alkyleneoxy;
R is C₆₋₃₀ aryl, C₆₋₃₀ aryl substituted by R¹, C₁₋₃₀ heteroaryl, C₁₋₃₀ heteroaryl substituted by R², C₁₋₁₀ alkyl, C₁₋₁₀ alkyl substituted by R³, C₃₋₁₀ cycloalkyl, C₃₋₁₀ cycloalkyl substituted by R⁴, C₁₋₁₀ heterocycloalkyl, C₂₋₁₀ alkenyl, C₃₋₁₀ cycloalkenyl or C₃₋₁₀ cycloalkenyl substituted by R⁸;
R¹ is one or more of halogen, C₁₋₁₀ alkyl, C₁₋₁₀ alkoxy and cyano; and,
R², R³, R⁴, R⁵, R⁶, R⁷ and R⁸ are independently halogen.

5. The nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in at least one of claims 1-4, wherein, X is O or N, preferably O;
and/or, R is a C₆₋₃₀ aryl, C₆₋₃₀ aryl substituted by R¹, C₃₋₁₀ cycloalkenyl or C₃₋₁₀ cycloalkenyl substituted by R⁸, preferably C₆₋₃₀ aryl substituted by R¹;
and/or, R¹ is one or more of halogen, C₁₋₁₀ alkyl and C₁₋₁₀ alkoxy, preferably halogen;
and/or, R^{e} is C₆₋₃₀ aryl substituted by R^{e-1} or C₃₋₁₀ cycloalkenyl, preferably C₆₋₃₀ aryl substituted by R^{e-1};
and/or, R^{e-1} is independently one or more of halogen, C₁₋₁₀ alkoxy and 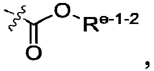 preferably halogen;
and/or, R^{f} is H, C₁₋₁₀ heterocycloalkyl or C₁₋₁₀ heterocycloalkyl substituted by R^{f-2}, preferably H or C₁₋₁₀ heterocycloalkyl.

6. The nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in at least one of claims 1-5, wherein, in the pharmaceutically acceptable salt thereof, the hydrate thereof, the solvate thereof, the polymorph thereof, the metabolite thereof, the stereoisomer thereof, the tautomer thereof, or the prodrug thereof, the X, the R, the R¹, the R^{e}, the R^{e-1} or the R^{f} are defined as shown below:
X is O; R is C₆₋₃₀ aryl substituted by R¹, C₃₋₁₀ cycloalkenyl or C₃₋₁₀ cycloalkenyl substituted by R⁸; R¹ is one or more of halogen, C₁₋₁₀ alkyl and C₁₋₁₀ alkoxy; R^{e} is C₆₋₃₀ aryl substituted by R^{e-1} or C₃₋₁₀ cycloalkenyl; R^{e-1} is one or more of halogen, C₁₋₁₀ alkoxy and 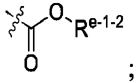 and, R^{f} is H, C₁₋₁₀ heterocycloalkyl or C₁₋₁₀ heterocycloalkyl substituted by R^{f-2};
or,
X is O; R is C₆₋₃₀ aryl substituted by R¹; R¹ is one or more of halogen, C₁₋₁₀ alkyl and C₁₋₁₀ alkoxy; R^{e} is C₆₋₃₀ aryl substituted by R^{e-1} or C₃₋₁₀ cycloalkenyl; R^{e-1} is one or more of halogen, C₁₋₁₀ alkoxy and 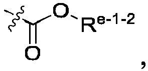 and at least one of the R^{e-1} is located at the ortho position C relative to C connected to the connection site; and, R^{f} is H, C₁₋₁₀ heterocycloalkyl or C₁₋₁₀ heterocycloalkyl substituted by R^{f-2};
or,
X is O; R is a C₆₋₃₀ aryl substituted by R₁; R¹ is F, and at least one of the R¹ substitution sites is located at the ortho position C relative to the C connected to X; R^{e} is C₆₋₃₀ aryl substituted by R^{e-1}; R^{e-1} is halogen, and at least one of the of R^{e-1} substitution site is located at the ortho position C relative to C connected to the connection site; and, R^{f} is H or C₁₋₁₀ heterocycloalkyl.

7. The nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in at least one of claims 1-6, wherein, in the pharmaceutically acceptable salt thereof, the hydrate thereof, the solvate thereof, the polymorph thereof, the metabolite thereof, the stereoisomer thereof, the tautomer thereof, or the prodrug thereof;
X-R is 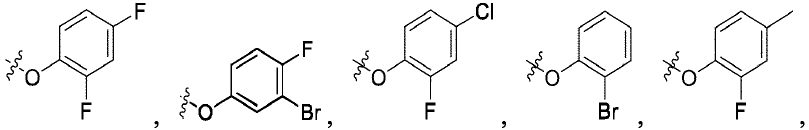 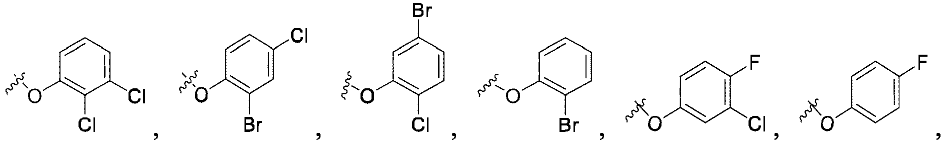 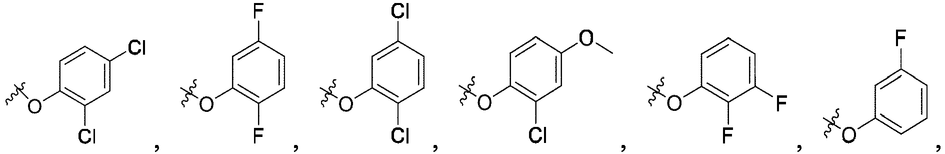 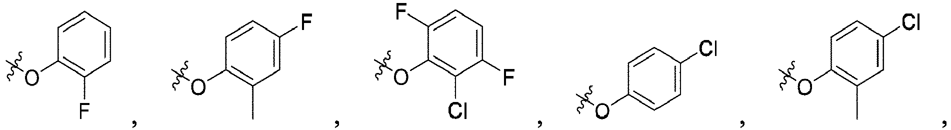 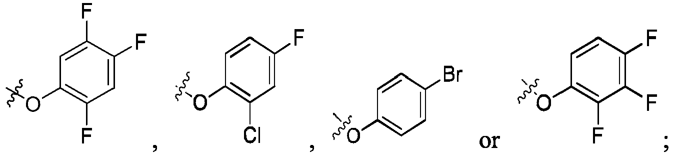 R^{e} is 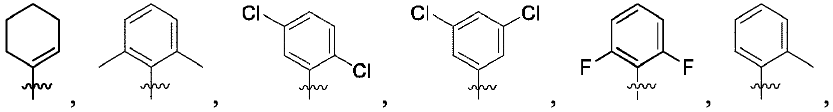 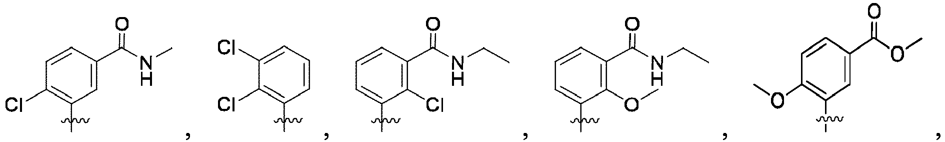 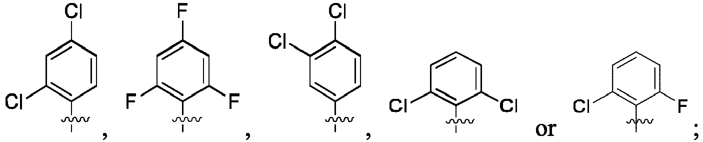 R^{f} is H,

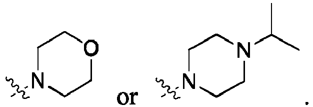

8. The nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in at least one of claims 1-7, wherein, in the pharmaceutically acceptable salt thereof, the hydrate thereof, the solvate thereof, the polymorph thereof, the metabolite thereof, the stereoisomer thereof, the tautomer thereof, or the prodrug thereof;
X-R is 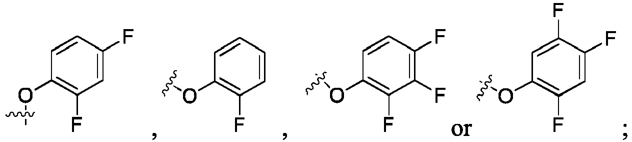 R^{e} is 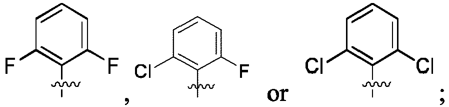 R^{f} is H or

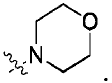

9. The nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in claim 1, wherein, is nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof is represented by formula IA; 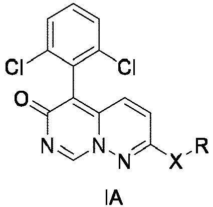
wherein, X is O or N;
R is C₆₋₃₀ aryl, C₆₋₃₀ aryl substituted by R¹, C₁₋₃₀ heteroaryl, C₁₋₃₀ heteroaryl substituted by R², C₁₋₁₀ alkyl, C₁₋₁₀ alkyl substituted by R³, C₃₋₁₀ cycloalkyl, C₃₋₁₀ cycloalkyl substituted by R⁴, C₁₋₁₀ heterocycloalkyl, C₁₋₁₀ heterocycloalkyl substituted by R⁵, C₂₋₁₀ alkenyl, C₂₋₁₀ alkenyl substituted by R⁶, C₂₋₁₀ alkynyl, C₂₋₁₀ alkynyl substituted by R⁷;
R¹, R², R³, R⁴, R⁵, R⁶ and R⁷ are independently selected from one or more of halogen, C₁₋₁₀ alkyl, C₁₋₁₀ alkoxy, 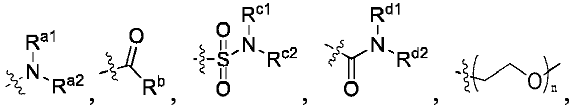 C6-30 aryl, C₁₋₆ heterocycloalkyl, hydroxyl and cyano;
n is 1, 2 or 3;
R^{a1}, R^{a2}, R^{b}, R^{c1}, R^{c2}, R^{d1} and R^{d2} are independently selected from H, C₁₋₆ alkyl, C₁₋₆ alkoxy, C₂₋₆ alkenyl, C₂₋₆ alkynyl, C₆₋₃₀ aryl or C₆₋₃₀ aryl substituted by R^{a-1};
R^{a-1} is selected from one or more of C₁₋₃ alkyl, C₁₋₃ alkoxy, halogen, cyano, nitro, amino and hydroxyl;
in the C₁₋₃₀ heteroaryl, the C₁₋₃₀ heteroaryl substituted by R², the C₁₋₁₀ heterocycloalkyl, the C₁₋₁₀ heterocycloalkyl in the C₁₋₁₀ heterocycloalkyl substituted by R⁴, and, the C₁₋₆ heterocycloalkyl in " R¹, R², R³, R⁴, R⁵, R⁶, and R⁷ ", the number of heteroatom is independently 1-4, and the heteroatom is independently one or more of O, S and N.

10. The nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in claim 1, wherein, the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof is anyone of the following compound; 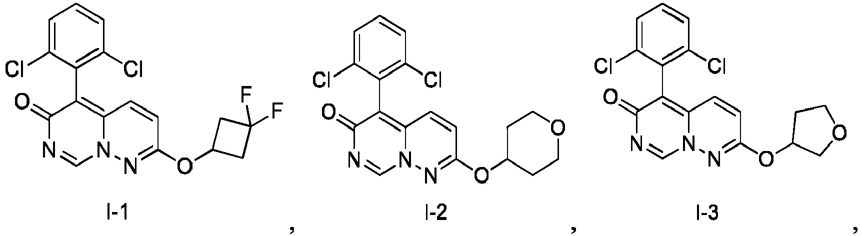 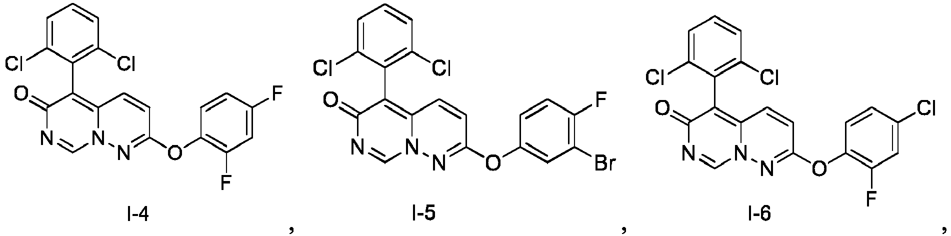 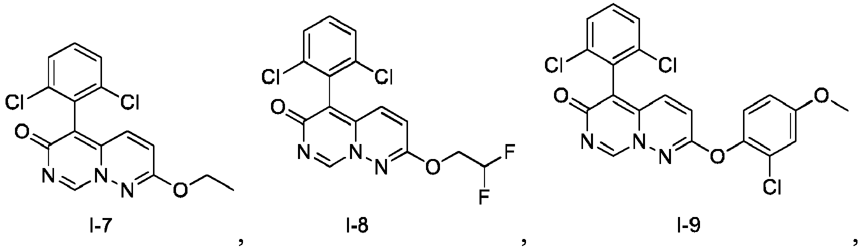 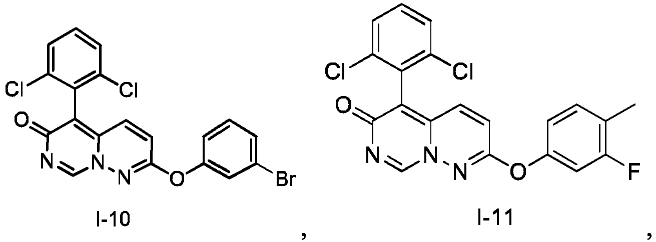 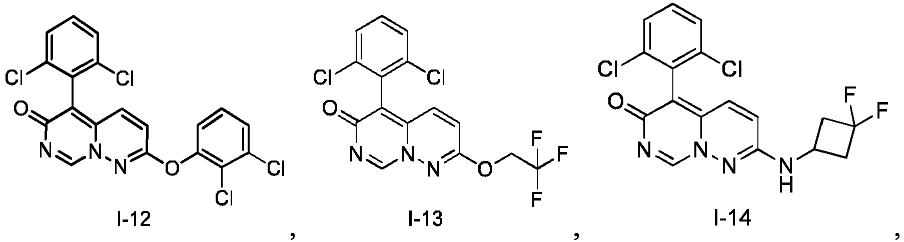 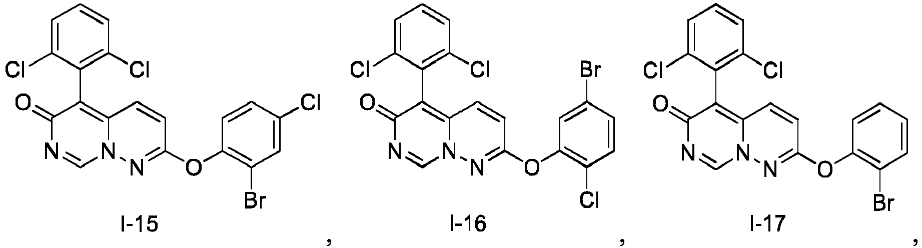 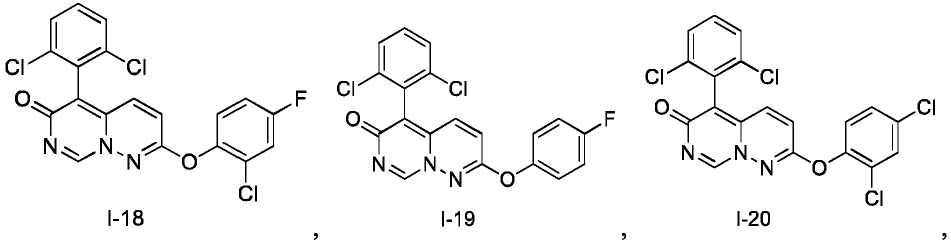 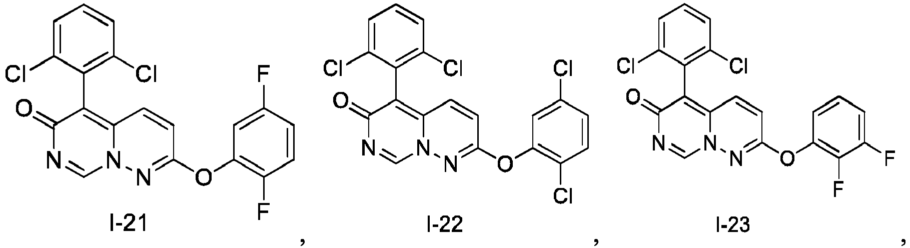 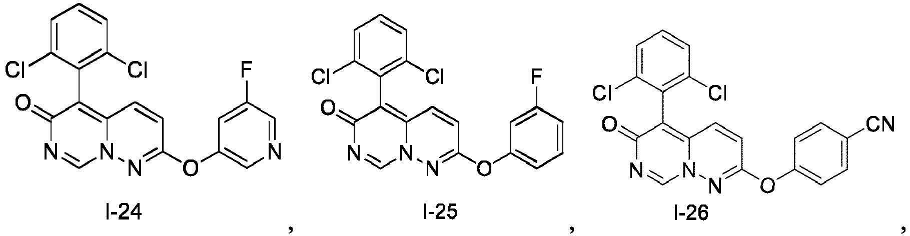 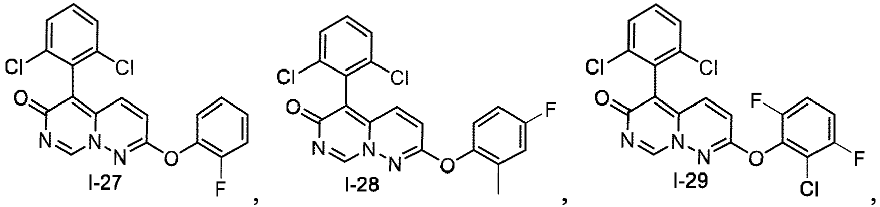 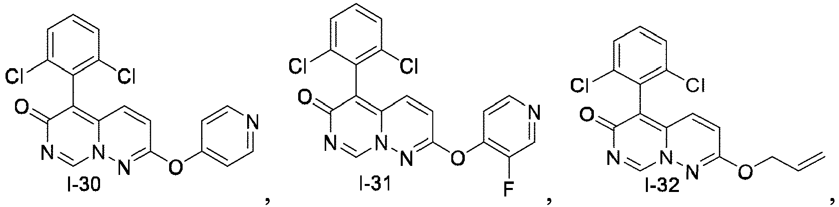 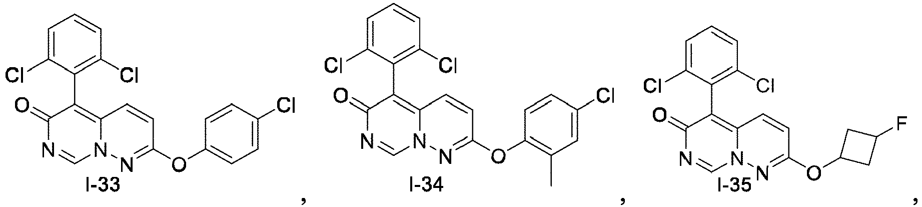 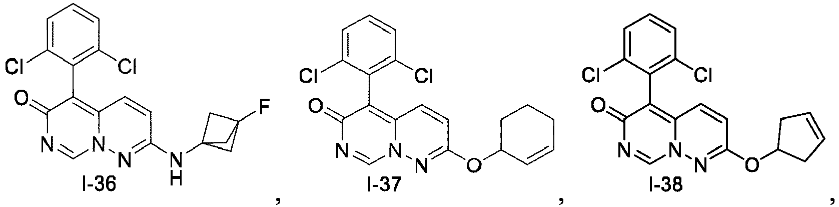 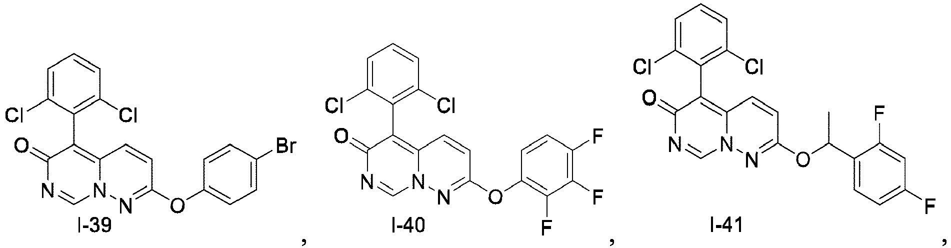 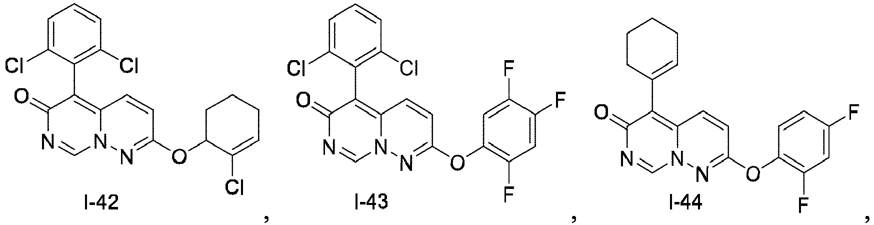 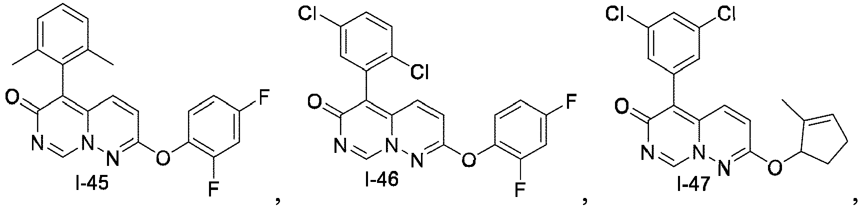 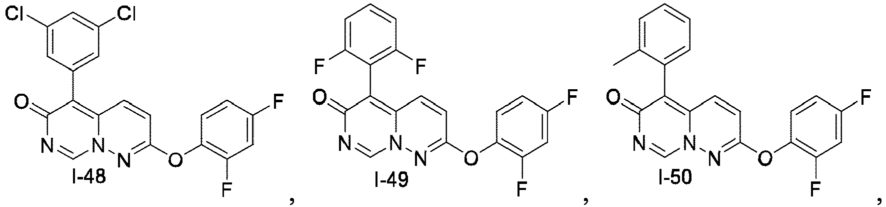 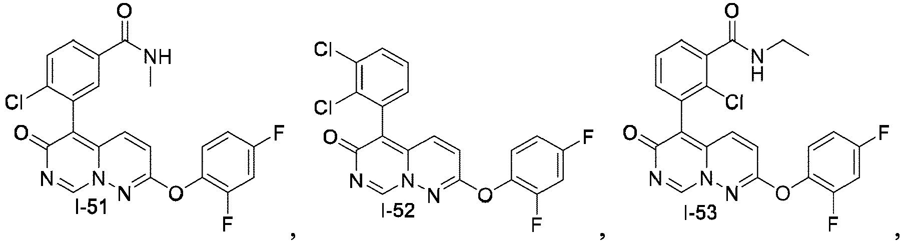 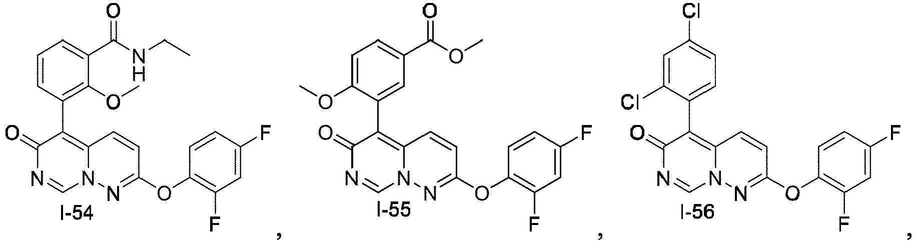 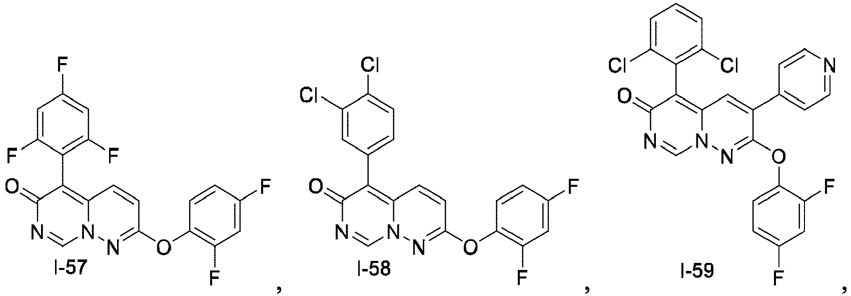 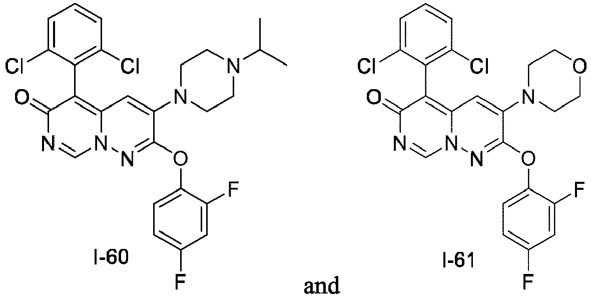 and

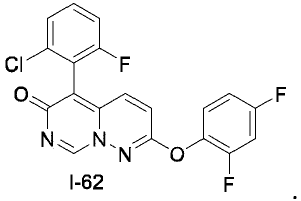

11. A method for preparing the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in at least one of claims 1-10, comprising the following steps: carrying out a substitution reaction of compound II and compound III in the presence of a basic reagent in an organic solvent as shown below to obtain the nitrogen-containing heterocyclic compound represented by formula I, 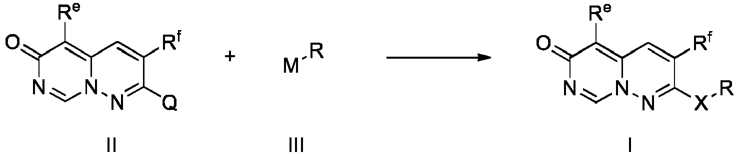 wherein, X, R, R^{e} and R^{f} are all defined as at least one of claims 1-10, Q is halogen, and M is a hydroxyl or amino.

12. A compound represented by formula II: 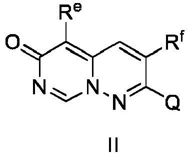 wherein X, R, R^{e} and R^{f} are all defined as at least one of claims 1-10, and Q and M are defined as claim 11.

13. The compound represented by formula II as defined in claim 12, wherein, the compound II is any of the following compound;

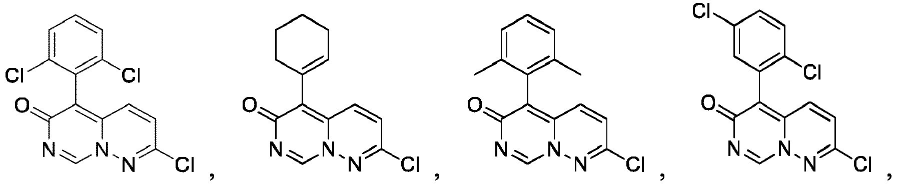

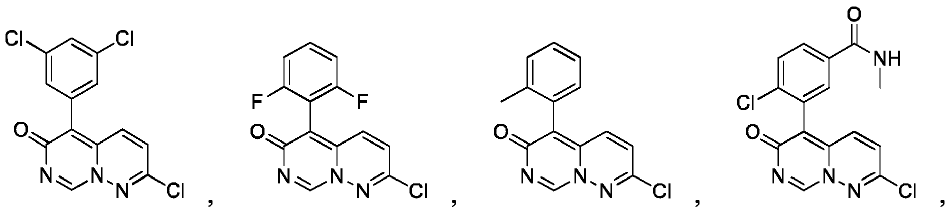

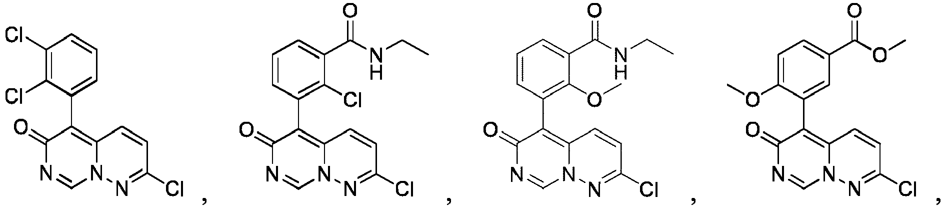

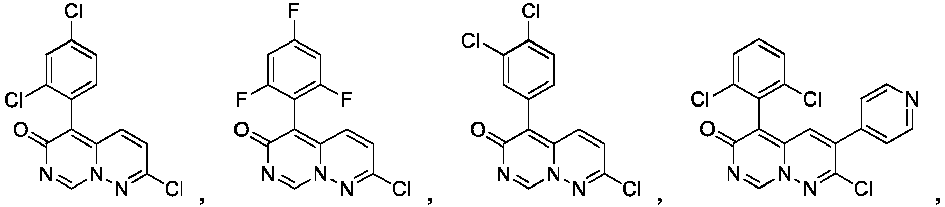

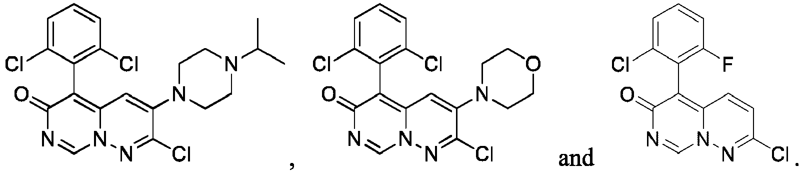

14. A pharmaceutical composition, comprising the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in at least one of claims 1-10, and pharmaceutically acceptable carrier.

15. A use of the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in at least one of claims 1-10, or the pharmaceutical composition as defined in claim 14, in manufacturing a medicament;
or, in manufacturing p38 protein kinase inhibitor.

16. The use as defined in claim 15, the medicament is used to treat or prevent disease, the disease is one or more of inflammatory disease, autoimmune disease, destructive bone disease, neurodegenerative disease, stroke, prostaglandin endoperoxide synthase-2 (pain) related disease, cardiac disease, proliferative disease, allergy, and cancer;
and/or, the p38 protein kinase can be p38α protein kinase and/or p38β protein kinase.

17. A method for treating a disease, comprising administering to a subject in need thereof an effective amount of the nitrogen-containing heterocyclic compound represented by formula I, the pharmaceutically acceptable salt thereof, the hydrate thereof, the solvate thereof, the polymorph thereof, the metabolite thereof, the stereoisomer thereof, the tautomer thereof, or the prodrug thereof as defined in at least one of claims 1-10, or the pharmaceutical composition as defined in claim 14; wherein the disease is related disease mediated by p38 protein kinase;
or, the disease is one or more of inflammatory disease, autoimmune disease, destructive bone disease, neurodegenerative disease, stroke, prostaglandin endoperoxide synthase-2 (pain) related disease, cardiac disease, proliferative disease, allergy, and cancer.
